# EUROPÄISCHE PATENTSCHRIFT

(11) **EP 1 652 920 B1**
(45) Veröffentlichungstag und Bekanntmachung des Hinweises auf die Patenterteilung: **04.08.2010**
(21) Anmeldenummer: 06002567.3
(22) Anmeldetag: 01.10.2001
(51) Int. Cl.: C12N 15/10

(54) **Verfahren zur Herstellung von Protein-Bibliotheken und zur Selektion von Proteinen daraus**
Methods of producing protein libraries and selection of proteins from them
Méthodes pour construire des banques de protéines et pour isoler des protéines de ces banques

(43) Veröffentlichungstag der Anmeldung: 03.05.2006
(62) Teilanmeldung aus: 01123596.7
(73) Patentinhaber: Deutsches Krebsforschungszentrum Stiftung des öffentlichen Rechts, 69120 Heidelberg (DE)
(72) Erfinder: Breitling, Frank, 69115 Heidelberg (DE); Moldenhauer, Gerhard, 69120 Heidelberg (DE); Poustka, Annemarie, 69120 Heidelberg (DE); Kühlwein, Thorsten, 68519 Viernheim (DE); Lüttgau, Sandra, 69469 Weinheim (DE)
(74) Vertreter: Schüssler, Andrea

(56) Entgegenhaltungen:
- WO-A-00/76310
- US-A- 5 202 238
- US-A- 6 091 001
- US-B1- 6 284 536

## Beschreibung

Die vorliegende Erfindung betrifft ein in den Ansprüchen näher charakterisiertes Verfahren zur Humanisierung einer Hybridomzelle. Somit betrifft die vorliegende Erfindung ein Verfahren zur Humanisierung einer Hybridomzelle, **dadurch gekennzeichnet, dass**
(a)eine eine oder mehrere humane konstante IgG-, IgM-, IgA-, IgD- oder IgE-Domänen codierende DNA-Sequenz in die Hybridomzelllinie transfiziert wird;
(b) die DNA-Sequenz der humanen konstanten IgG-, IgM-, IgA-, IgD- oder IgE-Domänen von DNA-Sequenzen flankiert wird, die zu den chromosomalen Genbereichen homolog sind, die die konstanten Domänen des aktiven, die schwere Kette des Antikörpers codierenden Genlocus der genannten Hybridomzelle flankieren;
(c) eine oder wenige Zellen expandiert werden, die auf Grund einer homologen Rekombination eine IgG-, IgM-, IgA-, IgD- oder IgE-schwere Kette mit einem humanisierten konstanten Anteil exprimieren;
(d) eine humane konstante Kappa- oder Lambda-Domäne codierende DNA-Sequenz in die Hybridomzelllinie transfiziert wird, wobei diese DNA-Sequenz von DNA-Sequenzen flankiert wird, die zu den chromosomalen Genbereichen homolog sind, die die konstante Kappa- oder Lambda-Domäne des aktiven Kappa- oder Lambda-Genlocus der Hybridomzelle flankieren;
(e) anschliessend eine oder wenige Zellen expandiert werden, die auf Grund einer homologen Rekombination eine Kappa- oder Lambda-Kette mit einem humanisierten konstanten Anteil exprimieren,
wobei die exprimierten Antikörper auf Grund der kovalenten Kopplung der membranständigen Spleissvariante der schweren Antikörperkette an die Oberfläche der sie jeweils exprimierenden Zellen gebunden sind, was den Nachweis und die Selektion derjenigen Zellen erlaubt, die humanisierte konstante Antikörperdomänen präsentieren.

Bisher wurden schon zahlreiche Versuche unternommen, um Antikörper mit gewünschter Spezifität, in hoher Ausbeute und guter Humanverträglichkeit, insbesondere als Therapeutika erhalten zu können, wobei die einzelnen Verfahren nachstehend kurz beschrieben werden.

**Hybridom-Antikörper.** In den siebziger Jahren wurde von Köhler und Milstein eine Methode zur Antikörper-Gewinnung entwickelt, die Hybridom-Technik (Köhler und Milstein, 1975, Continuous cultures of fused cells secreting antibody of predefined specificity. Nature, 256, 495--497). Sie erfordert zuerst die Immunisierung eines Versuchstiers (meist Maus oder Ratte). Danach werden Milz oder Lymphknoten entnommen und die darin in reicher Zahl enthaltenen B-Lymphozyten (Vorstufen der Antikörper produzierenden Zellen) gewonnen. Jeder B-Lymphozyt produziert aufgrund "seines" individuellen Gen-Rearrangements Antikörper mit nur einer einzigen Bindungsspezifität. Die Abkömmlinge dieses B-Lymphozyten, die B-Lymphozyten-Klone, produzieren ausschliesslich Antikörper dieser Bindungsspezifität.

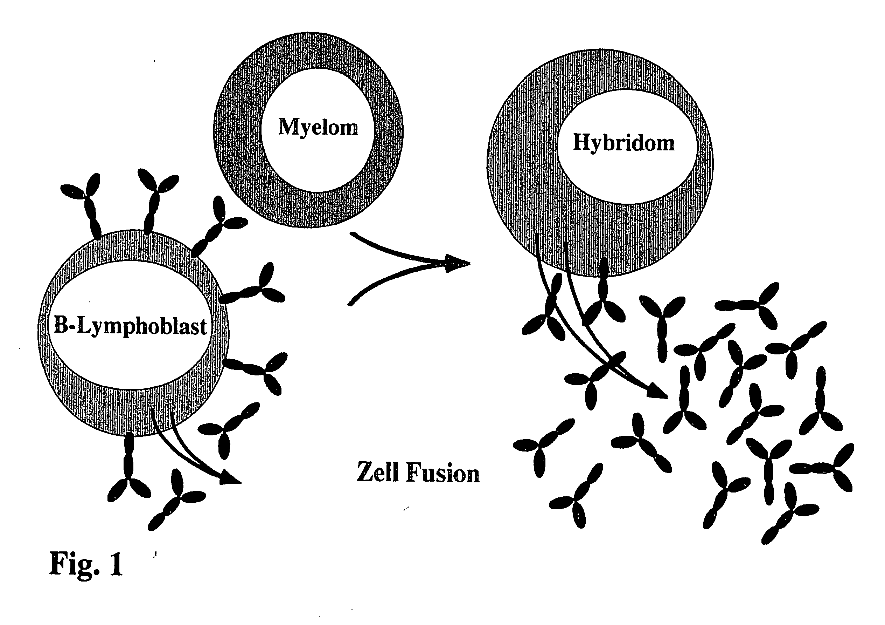

Einige der aus der Milz eines immunisierten Tieres gewonnenen Zellen produzieren Antikörper der gewünschten Spezifität. Um diese *in vitro* herstellen zu können, muss man die B-Lymphozyten in Zellkultur vermehren. Dies wird erreicht, indem man sie mit Myelom-Zellen, Abkömmlingen eines Plasmazelltumors, fusioniert. Die resultierenden Hybridom-Zellen haben Eigenschaften beider Fusionspartner: Einerseits haben sie die Unsterblichkeit der Krebszellen, andererseits produzieren sie den jeweiligen Antikörper des B-Lymphozyten-Partners. Die Abkömmlinge einer einzelnen Hybridom-Zelle (d. h. ihr Klon) produzieren Antikörper mit dieser definierten Spezifität. Man nennt diese deshalb auch monoklonale Antikörper. Die Technik der Hybridom-Herstellung ist schematisch in Figur 1 gezeigt. Ein Vorteil der monoklonalen Antikörper gegenüber den polyklonalen Antikörpern liegt darin, dass sie durch die nun unsterblichen Zellen in prinzipiell unbegrenzter Menge produziert werden können.

**Nachteile**: Bei der bisherigen Hybridom-Technik werden Mäuse immunisiert und anschliessend die Maus-B-Lymphocyten mit einer Myelom-Zelllinie fusioniert. Die so gebildeten Hybridom-Zellen werden danach als Einzelzellklone getrennt propagiert und der Überstand der einzelnen Klone auf Antikörper gewünschter Spezifität hin untersucht. Die identifizierten Einzelklone müssen anschliessend sofort in einer zweiten Selektionsrunde subkloniert werden, da sie in dieser Zeitphase genetisch instabil sind. Dieses Verfahren ist sehr zeitaufwändig, so dass im Endeffekt maximal einige tausend Hybridom-Klone auf die gewünschte Spezifität hin getestet werden können. Der Aufbau und die Durchsuchung einer Hybridom-Bibliothek ist mit dieser Technik nur sehr begrenzt möglich. Damit ist die Automatisierung dieses Verfahrens nur schwer möglich. Diese konventionelle Technik erlaubt nicht die Herstellung von humanen Antikörpern.

Mausstämme, die humane Hybridom-Antikörper produzieren. Ein Sonderfall sind humane Hybridom-Antikörper, die aus transgenen Mäusen gewonnen werden, deren eigener Immunoglobulin-Genlocus durch Teile des humanen Immunoglobulin-Genlocus ersetzt wurde (Jakobovits, 1995, Production of fully human antibodies by transgenic mice. Curr Opin Biotechnol 6, 561-566; Lonberg und Huszar, 1995, Human antibodies from transgenic mice. Int Rev Immunol. 13, 65-93; Kucherlapati et al. US-Patent 6114598: Generation of xenogeneic antibodies). Die humanen Antiköper-Gene rearrangieren, durchlaufen den *class switch* und werden somatisch hypermutiert. Diese transgenen Mäuse produzieren also humane Antikörper in Mäusezellen, die (i.Ggs. zu humanen Hybridom-Zellen) zu stabilen Mäuse-Hybridomen führen.

**Nachteile**: Mit dieser Technik können zwar humane Antikörper hergestellt werden, diese die Technik ist aber genauso aufwändig und kompliziert wie die vorstehend diskutierte Hybridomtechnik. Über die tatsächliche Qualität der generierten transgenen Mäusestämme ist bisher wenig bekannt. Dazu gehören Fragen wie: Stört das Zusammenspiel von vermenschlichten Antikörpern mit anderen Maussignalen? Wie gut ist die Immunantwort der Mäuse? Wie viele Antikörper-Gene funktionieren / sind in dem Mausgenom? usw. Es ist deswegen derzeit noch nicht klar, ob diese "humanisierten Mäuse" die in sie gesetzten Hoffnungen erfüllen können.

**Humanisierte Hybridom-Antikörper**. Eine Vielzahl von potenziell therapeutisch interessanten Hybridom-Antikörpern aus der Maus ist bereits vorhanden. Ein Problem bei ihrer therapeutischen Verwendung ist jedoch ihre Herkunft aus der Maus, denn Proteine aus einer fremden Spezies werden vom humanen Immunsystem als fremd erkannt. Das gilt auch für Antikörper aus der Maus. Es kommt zur Bildung der sogenannten "HAMA"-Immunantwort) (Humane anti-Maus-Antikörper). Diese innerhalb weniger Tage vom humanen Immunsystem gebildeten Antikörper neutralisieren in der Regel den therapeutisch eingesetzten Maus-Antikörper und machen ihn damit unwirksam. Auch eine wiederholte Therapie ist dadurch nur sehr eingeschränkt möglich (Courtenay-Luck et al., 1986, Development of primary and Secondary Immune Responses to Mouse Monoclonal Antibodies Used in the Diagnosis and Therapy of Malignant Neoplasms. Cancer Res. 46, 6489-6493; Lamers et al., 1995, Inhibition of bispecific monoclonal antibody (bsAb) targeted cytolysis by human anti mouse antibodies in ovarian carcinoma patients treated with bsAb targeted activated T lymphocytes. Int J Cancer 60, 450-457).

Die grosse Mehrzahl der HAMA-Antikörper sind gegen den konstanten Teil der Antikörper gerichtet, weshalb zur Herstellung von Antikörper-Chimären übergegangen wurde. Diese enthalten eine variable Maus-Antikörper-Domäne, gefolgt von den konstanten Antikörper-Domänen aus dem Menschen. Dazu wird zunächst ein humanes Antikörper-Gen in einen Klonierungsvektor gesetzt (Wright et al., 1992, Genetically engineered Antibodies: Progress and Prospects. Critical Rev Immunol. 12, 125 168). Die einzelnen Antikörper-Domänen bilden kompakte Faltungseinheiten, die durch einen Peptidstrang untereinander verbunden sind. Beim Austausch ganzer Antikörper-Domänen ist somit die Chance einer Störung der Antikörper-Funktion am geringsten. Mit der Erfindung der PCR ist es problemlos möglich, chimäre cDNAs herzustellen, da auf die Base genaue Klonierungen mit dieser Technik wesentlich vereinfacht werden. Die resultierenden chimären Antikörper binden immer noch spezifisch an das Antigen, die HAMA-Antwort ist allerdings deutlich herabgesetzt.

Wichtiger als die HAMA-Reaktion ist aber noch ein weiterer Punkt: Da nun die konstanten Domänen aus dem Menschen stammen, aktivieren diese chimären Antikörper auch einige Helferfunktionen des humanen Immunsystems, wie die Antikörper abhängige zelluläre Zytotoxizität (ADCC) oder die Komplementaktivierung, deutlich besser. Dies ist ein weiterer Grund, warum sich einige solcher humanisierten Antikörper bereits in klinischen Anwendungen befinden (McLaughlin et al., 1998, Clinical status and optimal use of Rituximab for B-cell lymphomas. Oncology 12, 1763-1777).

**Nachteile**: Auch die Humanisierung von bereits existierenden Hybridom-Antikörpern ist sehr mühsam und zeitaufwändig. Auch die Stabilität der so hergestellten Hybridome ist oft ein Problem: Die Zellen mutieren oder sie sekretieren wenig Antikörper ins Medium.

**Humanisierung durch homologe Rekombination**. In dem US-Patent Nr. 5202238 wird eine Methode beschrieben, mit der monoklonale Maus-Antikörper humanisiert werden können. Im Zentrum der Methode steht die sog. "homologe Rekombination". Dabei werden humane Sequenzen, die von geeigneten genomischen Maussequenzen flankiert sind, ortsgenau in den aktiven Antikörper-Lokus rekombiniert. Der grosse Vorteil dieser Methode ist, dass dabei die auf gute Antikörper-Produktion optimierten Signale des Antikörper-Lokus weitgehend erhalten bleiben (Yarnold und Fell, 1994, Chimerization of antitumor antibodies via homologous recombination conversion vectors. Cancer Research 54, 506-512; Fell et al., 1989, Homologous recombination in hybridoma cells: heavy chain chimeric antibody produced by gene targeting. PNAS 86, 8507-8511).

Nachteile: Ähnlich wie bei der Generierung von Hybridomas wird bei diesem Verfahren sehr viel Arbeit benötigt, bis ein einziges humanisiertes Hybridom isoliert werden kann. Tausende von Klonen müssen dafür jeweils getrennt kultiviert und analysiert werden. Ein weiterer Nachteil resultiert aus dem verwendeten Selektionsmarker: Dieser führt offensichtlich zu einer hohen Zahl von Revertanten (der einrekombinierte Mensch-Antikörper-Lokus wird in der Rückreaktion wieder ausgeschnitten) und/oder zu einer Beeinträchtigung des Expressionsniveaus (Baker et al., 1994, J. Immunological Methods 168, 25-32). Zusätzlich dazu verhindern die eingesetzten Resistenzgene die Oberflächenpräsentation der Antikörper, wenn sie in das Intron zwischen dem CH3-Exon und dem M1-Exon eines IgG eingefügt werden.

**Rekombinante Antikörper**. In den letzten Jahren wurde mit der Konstruktion von *rekombinanten Antikörpern* ein auf gentechnologischen Methoden beruhender Weg zur Herstellung von Antikörperfragmenten eröffnet (Breitling und Dübel, 1997, "Rekombinante Antikörper", Spektrum-Verlag ISBN 3-8274-0029-5). Dabei werden die Antikörper nicht mehr in einem Versuchstier (oder im humanen Organismus) erzeugt, sondern *in vitro* in Bakterien oder Zellkultur. Im Mittelpunkt steht dabei der Antigen-bindende Teil des Antikörpers. Meist verzichtet man zugunsten einer höheren Ausbeute auf den Rest des Antikörper-Moleküls. Diese Fragmente können natürlich nicht mehr alle Funktionen eines natürlich hergestellten Antikörpers vermitteln. Dafür können sie aber vergleichsweise einfach mit Enzymen oder anderen Antikörpern fusioniert werden. Diese rekombinanten Antikörper bekommen dabei vollkommen neue Eigenschaften. Der Begriff "rekombinante Antikörper" hat sich für ein gentechnologisch *in vitro* hergestelltes Antikörperfragment, das ansonsten ausschliesslich über die Antigen-Spezifität definiert ist, eingebürgert.

**Nachteile:** Rekombinanten Antikörpern fehlt der konstante Antikörperteil und damit die für viele Therapieansätze wesentlichen Effektor-Funktionen. Deswegen werden neu aufgefundene "Antikörper-Suchköpfe" für viele Anwendungen auf eukaryontische Expressionsvektoren "aufgepfropft", d.h. es wird die vorstehend beschriebene arbeitsaufwändige Methode benützt. Neben der damit verbundenen experimentellen Arbeit kann es zu schlechter Expression kommen, da im bakteriellen System andere Codons bevorzugt werden als in eukaryontischen Systemen. Ein weiterer Nachteil speist sich aus den Eigenschaften der meist verwendeten "single chain"-Antikörper (scFv): Diese sind meist relativ instabil und aggregieren leicht. Darüber hinaus werden viele variable Domänen offensichtlich von E.coli-Proteasen attackiert. Damit sind auch die publizierten Komplexitäten der scFv-Antikörper Bibliotheken (bis 10¹¹) mit Vorsicht zu betrachten. Zusätzlich zu den gerade beschriebenen Problemen befinden sich in diesen Bibliotheken offensichtlich auch noch sehr viele Klonierungsartefakte. Dies wiederum bedeutet, dass nach spätestens 4 Selektionsrunden die selektionierten Klone fast nur noch Artefakte repräsentieren. Ein weiterer Nachteil ist die Tatsache, dass man in der Regel mehr als eine Selektionsrunde benötigt, bis der gewünschte Antikörper erhalten wird. Dies liegt daran, dass pro Phage nur ca. 0,1 scFv-Antikörper auf der Phagenoberfläche präsentiert werden. Es ist sehr wahrscheinlich, dass dieser Wert stark schwankt, je nachdem welcher Antikörper präsentiert wird. Ein weiterer Nachteil ist, dass die Identität der einzelnen Klone (d.h., produziert der selektionierte Klon überhaupt einen Antikörper?) relativ aufwändig überprüft werden muss.

**Präsentation von Antikörpern auf der Oberfläche von Hybridomzellen**. Diese Technik beruht auf der vorstehend beschriebenen Hybridom-Technik. Im Unterschied dazu wird jedoch dabei eine stabile Myelomzelllinie verwendet (und hergestellt), die ein Antikörper-Bindeprotein (z.B. Protein G) in grossen Mengen auf der Zelloberfläche verankert (Breitling et al., 1999, Selektion von monoklonalen Antikörpern. DE 199 00 635 A1. PCT Anmeldung unter der Nummer PCT DE00/00079). Dadurch wird ein grosser Teil der Arbeit vermieden, der durch das Klonieren und Subklonieren der monoklonalen Hybridome entsteht. Stattdessen können die gewünschten Antikörper-Spezifitäten im FACS-Sorter oder mit Magnetobeads aus einem Pool von Hybridomen isoliert werden, da die produzierten Antikörper durch die Bindung an das beschriebene Antikörper-Bindeprotein auf der Zelloberfläche verankert werden.

**Nachteile**: Diese Technik vermeidet nur einen Teil der aufwändigen Arbeit zur Selektion einzelner Antikörper-Spezifitäten. Immer noch müssen Mäuse immunisiert und anschliessend die Maus-B-Lymphocyten mit einer Myelom-Zelllinie fusioniert werden. Dies geschieht mit relativ schlechter Effizienz: In der Regel werden pro Fusion und Maus nur 100-500 verschiedene Hybridome generiert. Die so gebildeten Hybridom-Zellen weisen zudem eine unerwünscht hohe Variabilität in der Zahl der präsentierten Antikörper auf, was die Selektion im FACS-Sorter stark beeinträchtigt. Darüber hinaus gibt es wegen der nicht-kovalenten Verankerung der Antikörper auf der Oberfläche einen Cross-talk unterschiedlicher Antikörper-Spezifitäten. Damit präsentieren die unterschiedlichen Hybridomzellen nicht nur jeweils "ihren" Antikörper auf der Oberfläche, sondern auch weitere Antikörper-Spezifitäten, die von anderen Hybridomzellen ins Medium abgegeben wurden. Auch dieses Verfahren ist zeitaufwändig, so dass im Endeffekt maximal einige zehntausend unterschiedliche Hybridome generiert werden können (und anschliessend auf die gewünschte Spezifität hin getestet werden können). Damit ist der Aufbau und die Durchsuchung einer Hybridom-Bibliothek mit dieser Technik nur begrenzt möglich. Dies gilt auch für die Automatisierung dieses Verfahrens. Diese Technik ermöglicht auch nicht die Herstellung von humanen Antikörpern.

**Kassetten-Austausch mittels spezifischer Rekombination**. Es gibt mittlerweile eine Reihe von Verfahren, die eine DNA-Lokus-spezifische Rekombination innerhalb einer eukaryontischen Zelle ermöglichen (siehe z.B. Sauer, US-Patent 4959317: Site-specific recombination of DNA in eukaryotic cells; Leboulch et al., US-Patent 5928914: "Methods and compositions for transforming cells; Feng et al., 1999, Site-specific chromosomal integration in mammalian cells: highly efficient CRE recombinase-mediated cassette exchange. J-Mol-Biol. 292, 779-785). Alle diese Verfahren verwenden eine Rekombinase (z.B. Flp, Cre, Int etc.), die spezifische DNA Sequenzen erkennt und mit anderen DNA-Sequenzen rekombiniert. Kennzeichen dieser Verfahren sind die oftmals recht hohen Effizienzen der spezifischen Rekombinationsereignisse, die *in vitro*, aber auch *in vivo* mit diesen Verfahren erreichbar sind. Angewendet werden diese Verfahren z.B. als Klonierungshilfe (Austausch von DNA Kassetten in vitro), aber auch in vivo zur Rekombination in lebenden Bakterien, in lebenden eukaryontischen Zellen, oder gar in transgenen Mäusen.

**Nachteile**: Der Kassettenaustausch von Antikörpergenen in eukaryontischen Zellen in Kombination mit der Oberflächenexpression und anschliessenden Selektion monoklonaler Antikörper ist bisher nicht beschrieben worden.

Das der vorliegenden Erfindung zugrunde liegende technische Problem war somit die Bereitstellung eines Verfahrens zur Erzeugung einer Protein-Bibliothek, vorzugsweise einer Antikörper-Bibliothek, bzw. zur Selektion von Proteinen, vorzugsweise Antikörpern, mit gewünschten Spezifitäten, das die Nachteile der bisherigen, vorstehend geschilderten Verfahren nicht aufweist. Dieses Verfahren sollte auch z.B. die Erzeugung einer Bibliothek unterschiedlicher T-Zellrezeptoren umfassen. Insbesondere soll dieses Verfahren die Vorteile der Technik der rekombinanten Antikörper und der Hybridom-Antikörper miteinander kombinieren:
■ Die einfache Selektion spezifischer Reaktivitäten aus einer sehr grossen Zahl unterschiedlicher Antikörper (allgemeiner: Proteine), möglichst in Verbindung mit einer hohen Signalstärke während des Selektionsvorgangs (z.B. indem jede Zelle viele gleichartige Antikörper präsentiert);
■ Die vergleichsweise einfache Änderung der exprimierten Gene, wie z.B. die Fusion der leichten Antikörperkette mit einem Einzelkettenantikörper (bispezifische Antikörper) oder die Fusion mit einem weiteren Proteinanteil (siehe auch: Bispecific Antibodies von Michael W. Fanger. Springer Verlag, 1995, ISBN 3-540-58885-X);
■ Die Produktion grosser Mengen der selektionierten Antikörper (Proteine) in guter Qualität für diagnostische oder therapeutische Zwecke, insbesondere durch eine ins Kulturmedium abgegebene Variante und
■ dadurch die einfache Verifizierung und Charakterisierung der selektionierten Zelllinie oder Sub-Bibliothek.

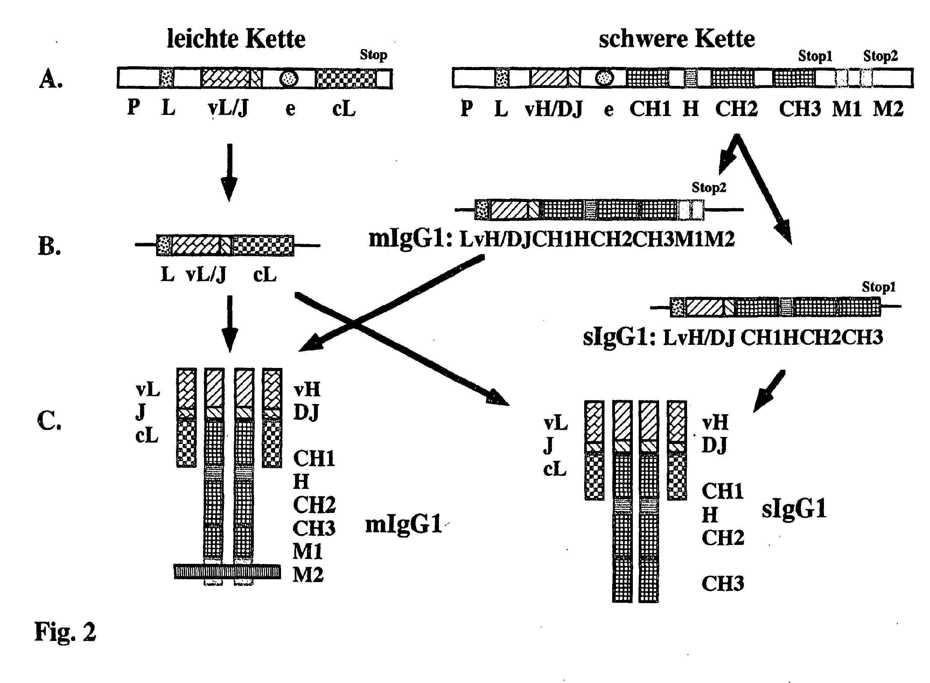

Die Lösung dieses technischen Problems erfolgte durch die Bereitstellung der in den Ansprüchen gekennzeichneten Ausführungsformen. Mit der vorliegenden Erfindung kann die Selektion von Zellen, die spezifische Proteine (z.B. monoklonale Antikörper) produzieren, beschleunigt werden. Die Zahl der Zellen, die auf eine vorgegebene Spezifität hin untersucht werden kann, kann mit dem erfindungsgemäßen Verfahren um mehrere Grössenordnungen erhöht werden. Die Selektion von Proteinen (Antikörpern) geschieht dabei in einer einzigen Selektionsrunde. Dies wird durch die grosse Zahl der auf der Zelloberfläche präsentierten Proteine (Antikörper) ermöglicht und zusätzlich dazu durch die vergleichsweise homogene Anzahl der von den jeweiligen Zellen präsentierten Proteine (Antikörper). Mittels der nach dem erfindungsgemäßen Verfahren hergestellten Antikörper-Bibliothek kann die Suche nach unterschiedlichen Antikörper-Spezifitäten leicht durchgeführt werden. Ausserdem erlaubt diese die Automatisierung der Suche nach monoklonal exprimierten Antikörpern, womit die riesige Bandbreite der Anwendung monoklonaler Antikörper besser genutzt werden kann. Damit kann wiederum das offensichtlich grosse Potenzial humaner oder humanisierter Antikörper in der Therapie von Krankheiten besser genutzt werden. Die vorstehend genannte vereinfachte Selektion monoklonaler Hybridom-Antikörper ergibt Zellklone, die monoklonale Antikörper in grossen Mengen und verglichen mit den rekombinanten Antikörpern auch in überlegener Qualität produzieren. Ausserdem kann die Selektion der Zellen ohne eingebaute Resistenzmarker durchgeführt werden, was vorteilhaft ist, da diese offensichtlich in vielen Fällen die Expression des veränderten Genproduktes und gleichzeitig die Stabilität der erhaltenen Zelllinie beeinträchtigen. So verhindert z.B. die Integration des Resistenzmarkers zwischen das CH3-Exon (ggfs. CH4-Exon) und das M1-Exon, dass eine Membran-gebundene Spleissvariante die Antikörper auf der Oberfläche der sie präsentierenden Zelle verankern kann, wodurch eine besonders einfache Selektion auf Grund der Oberflächenexpression Antigen-spezifischer Antikörper verhindert wird (Figur 2). Darüberhinaus ermöglicht eine Ausführungsform der vorliegenden Erfindung die einfache Humanisierung von Maus-Hybridomen und damit z.B. auch die Etablierung einer Palette von monoklonalen Antikörpern mit derselben Antigen-Spezifität aber unterschiedlichen Fc-Anteilen. Durch diesen Aspekt kann die grosse Vorarbeit auf dem Gebiet der monoklonalen Mausantikörper für die vergleichsweise einfache Herstellung von Humantherapeutika genutzt werden.

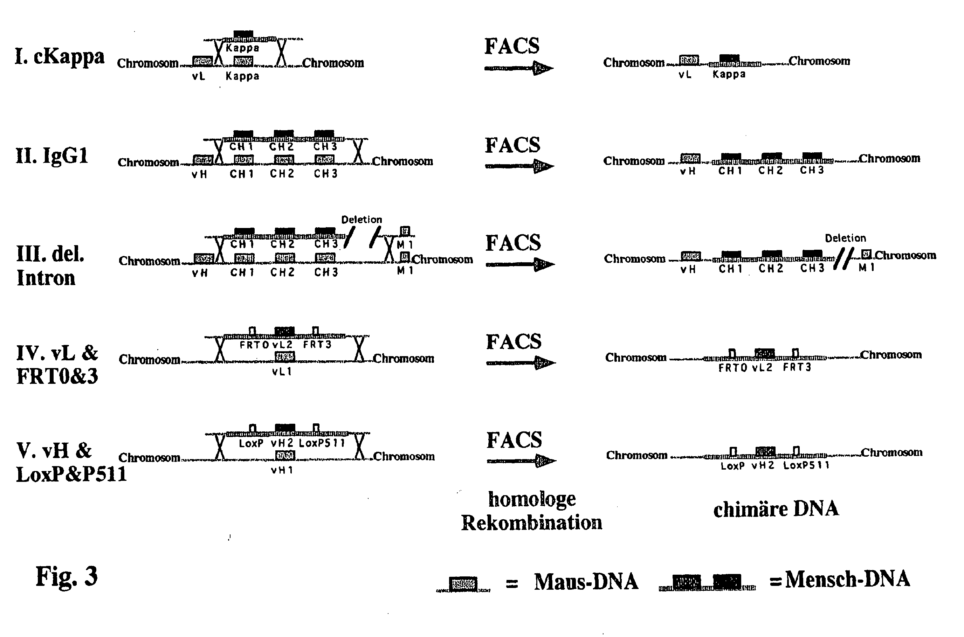

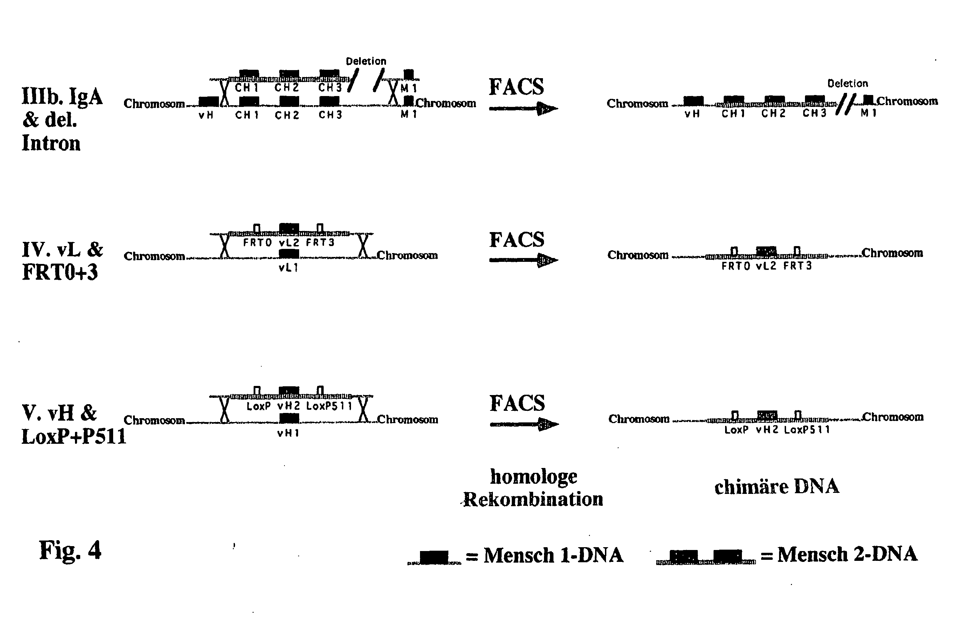

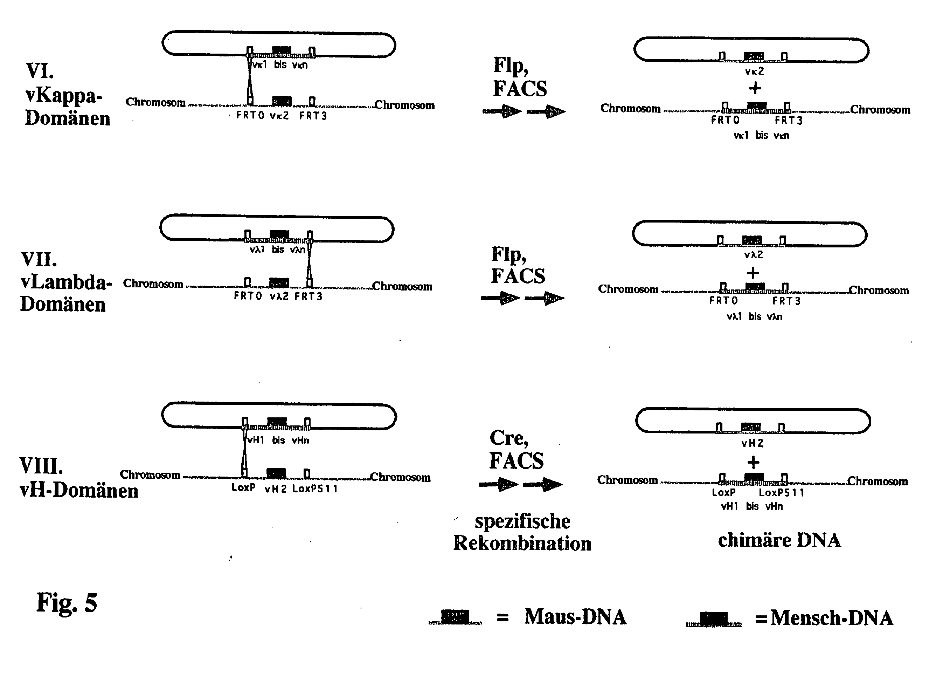

Das erfindungsgemäße Verfahren basiert darauf, dass eine grosse und verglichen von Zelle zu Zelle sehr uniforme Anzahl von Antikörpern kovalent an die Oberfläche einer eukaryontischen Zelle gebunden werden und damit von Zelle zu Zelle vergleichbare Signale erwartet werden können. Damit können spezifische Antikörper zusammen mit der präsentierenden Zelle aus einer Vielzahl von Zellen vergleichsweise einfach selektioniert werden. Durch diese Selektion wird ein monoklonaler Antikörper erhalten. Die Schar von Antikörperpräsentierenden Zellen wird durch den Umbau einer eukaryontischen Zelle erhalten. Dies erfolgt insbesondere durch mehrere nacheinander durchgeführte homologe (Figur 3, 4, 7 und 8) und spezifische Rekombinationsereignisse (Figur 5, 6 und 9). Insbesondere eignen sich dafür Maus-Hybridomzellen (Figur 3). Alternativ kann auch z.B. eine humane Myelom-Zelllinie als Ausgangspunkt für die sequenziellen homologen Rekombinationen verwendet werden (Figur 4). Dies hat den zusätzlichen Vorteil, dass die verglichen mit humanen Zellen leicht unterschiedliche Glykosilierung der von Mauszellen produzierten Antikörper vermieden wird. Es können aber auch andere stabile Zelllinien verwendet werden, insbesondere solche Zelllinien, die ein bestimmtes Genprodukt in grosser Menge prodzieren, wie z.B. T-Zelllymphome. Durch das erfindungsgemäße Verfahren wird eine hohe Antikörper-Vielfalt generiert (Figur 5, 6, 9). Ein weiteres Element dieser Erfindung ermöglicht die Selektion individueller Zellen aus der vorher generierten Vielfalt . Dies wird durch die Oberflächenpräsentation der Antikörper ermöglicht (Figur 2, 9, 10). Im Unterschied zu der bisher verwendeten Technik geschieht dies jedoch (a) vorzugsweise durch kovalente Kopplung der Antikörper auf der Zelloberfläche und (b) mittels eukaryontischer Zellen, vorzugsweise Säugerzellen, wobei die vorstehend beschriebenen Nachteile einiger der bisher verwendeten Techniken vermieden werden.

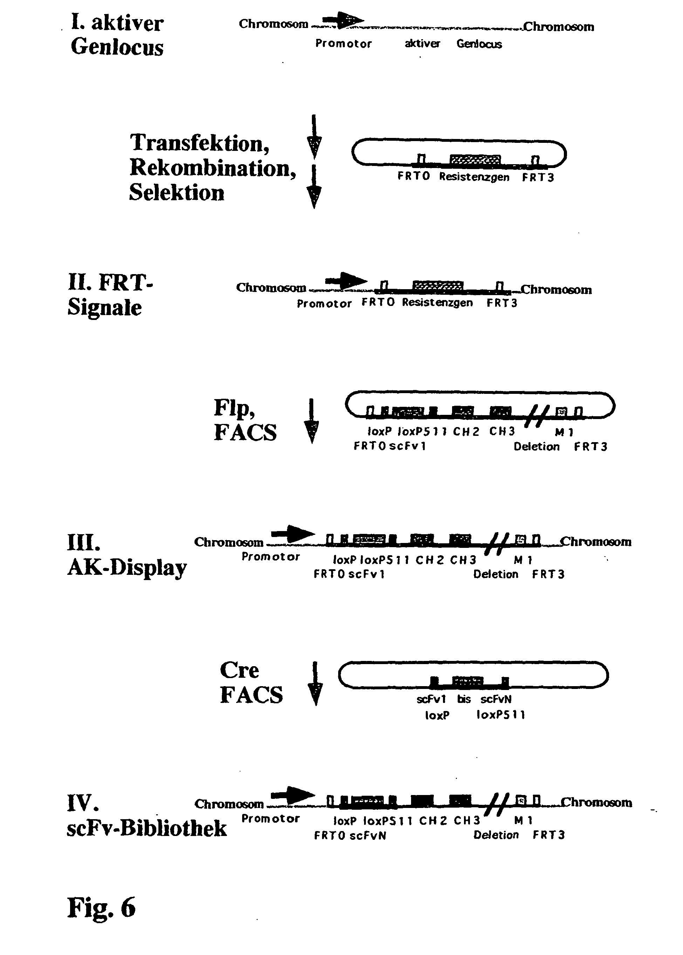

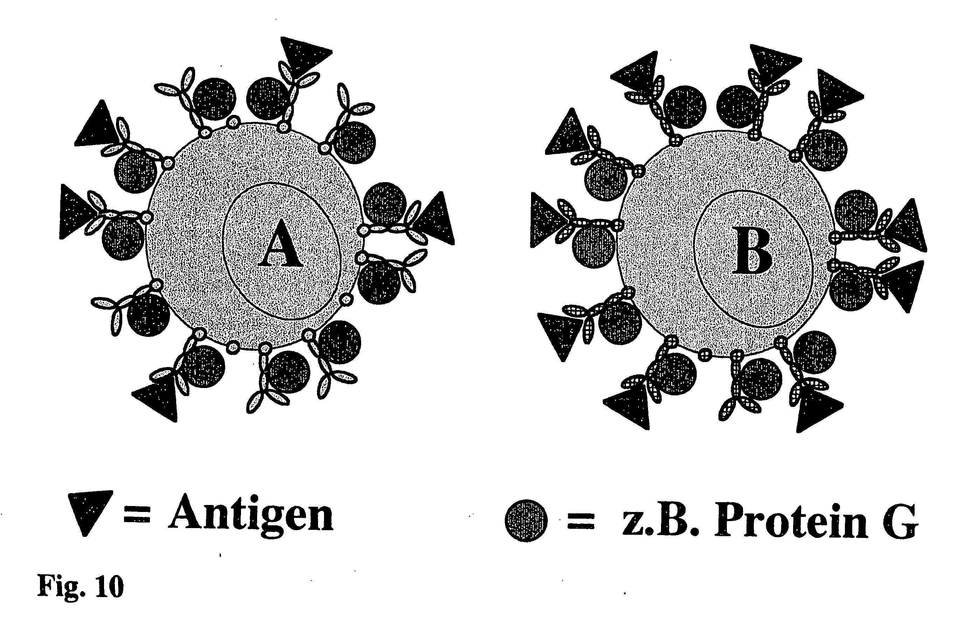

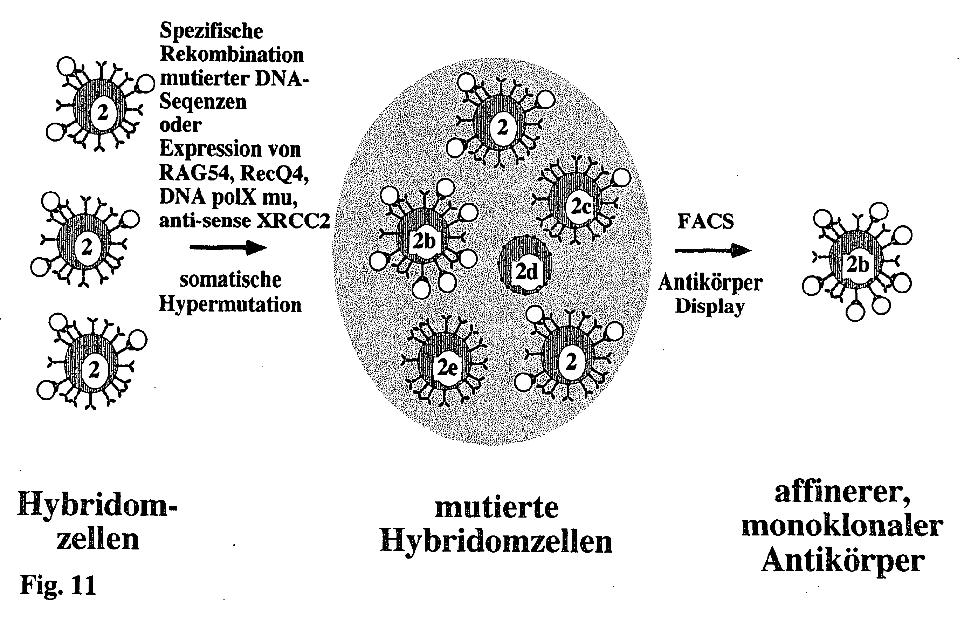

In einer besonderen Ausführungsform schliesst sich an das erfindungsgemäße Verfahren eine somatische Hypermutation der präsentierten Antikörper (und Gene) an, insbesondere um Antikörper zu selektionieren, die ein Antigen mit höherer Affinität binden können. Dazu können z.B. im Kontext der Antikörper-Genloci Expressionsvektoren für RAD54, RecQ4 und für die DNA-Polymerase polX mu verwendet werden (Figur 11), insbesondere in Kombination mit einem Vektor, der anti-sense RNA oder siRNA (Martines et al., (2002), Cell 110, 563ff; Elbashir et al., (2001), Nature 411, 494-498)gegen XRCC2, XRCC3 oder RAD51B exprimiert. Alternativ werden z.B.:
■ durch eine Fehler-anfällige-PCR eine Vielzahl von ungerichteten Mutationen, insbesondere in bereits vorselektionierte variable Antikörpergene eingeführt;
■ eine Vielzahl dieser mutierten variablen Antikörpergene mittels spezifischer Rekombinationssignale in den Antikörperlokus einrekombiniert (Figur 5, 6); und
■ anschliessend die Zellen z.B. im FACS selektioniert, die einen Antikörper höherer Affinität auf der Oberfläche präsentieren (Figur 10).

Die ungerichteten Mutationen können dabei z.B. nach dem von Stemmer (1994, Nature 370, 389-391) entwickelten Verfahren miteinander kombinierte werden. Es ist aber auch möglich in einer vorselektionierten Antikörper-produzierenden Zelle mit Hilfe der spezifischen Rekombinationssignale einfach die variable Domäne einer Antikörperkette gegen eine Vielzahl anderer variabler Domänen auszutauschen (Figur 5, 6, 9) und anschliessend nach affineren Varianten zu suchen. Somit zeigt das erfindungsgemäße Verfahren mehrere Wege auf, die eine somatische Hypermutation gefolgt von einer Selektion affinerer Antikörper in vitro ermöglichen (Figur 10, 11).

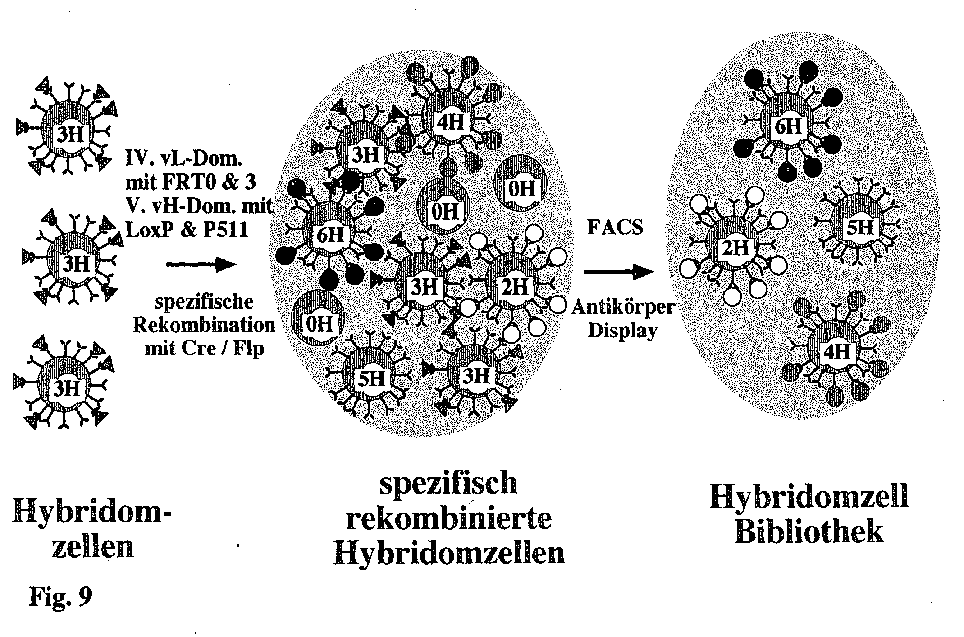

Zusammengefasst weist das erfindungsgemäße Verfahren folgende Vorteile auf: Es erlaubt die Herstellung einer hochkomplexen Bibliothek z.B. als Quelle monoklonaler Antikörper (Figur 6, 9) sowie die einfache Selektion, insbesondere hochaffiner humaner monoklonaler Antikörper (Figur 10, 11). Gleichzeitig können die Erfolgsaussichten einer solchen Selektion vergrössert werden. Durch die grosse und vergleichsweise uniforme Zahl präsentierter Antikörper gleicher Spezifität kann die Signalstärke bei der Suche nach spezifischen monoklonalen Antikörpern deutlich erhöht werden. Die mit dem erfindungsgemäßen Verfahren in hoher Ausbeute gewinnbaren humanen monoklonalen Antikörper mit hoher Affinität können z.B. als Tumordiagnostika und -therapeutika eingesetzt werden. Darüber hinausgehend können anstelle von Antikörpern auch andere Proteine, oder einzelne Proteindomänen, insbesondere Exons auf der Oberfläche jeweils "ihrer" Zelle präsentiert werden, wodurch eine besonders einfache Suche nach Bindungspartnern für diese präsentierten Proteinfragmente ermöglicht wird.

### Kurze Beschreibung der Figuren

### Figur 1

Schematische Darstellung der Herstellung von Hybridomzellen. B-Lymphoblasten werden meist unter Zugabe eines Gemisches von Polyethylenglykoll (PEG) mit DMSO mit einer Myelomzelllinie (z.B. Ag8.653) fusioniert. Anschliessend werden durch limitierte Verdünnung einzelne Zellklone propagiert und nach ca. 14 Tagen deren Zellkulturüberstand auf die gesuchte spezifische Antikörperreaktivität hin untersucht. Die resultierende Hybridomzelle erwirbt Eigenschaften beider parentalen Zellen: Die Myelomzelle steuert die Unsterblichkeit einer Tumorzelllinie sowie einige Steuersignale zur effizienten Produktion von Antikörpern bei, während der B-Lymphoblast eine spezifische Antikörperreaktivität genomisch rekombinierter Antikörpergenfragmente mitbringt. Die resultierende Hybridomzelle ist zunächst genetisch instabil, so dass in mindestens einer weiteren Selektionsrunde eine genetisch stabilere Variante etabliert werden muss.

### Figur 2

A. Genomische Sequenzen eines genomisch rekombinierten IgGl-Antikörpergens. Dargestellt ist die genomische Struktur der leichten Kette mit Promotor (P), Enhancer (e), Leader-Exon (L), der vL-Domäne (vL) mit genomisch rekombiniertem J-Segment (J) und dem Exon für die konstante Domäne der leichten Kette (cL). Daneben ist die genomische Struktur der schweren Kette mit Promotor (P), Enhancer (e), Leader-Exon (L), der vH-Domäne (vH) mit genomisch rekombiniertem D- und J-Segment (DJ) und den Exons für die konstanten CH1-, Hinge (H), CH2- und CH3-Domänen der schweren Kette dargestellt. Am 3'-Ende des IgG1-Gens befinden sich zwei Exons, die einen Membrananker codieren (M1 und M2).
B. Gespleisste mRNAs eines IgG1-Antikörpers. Dargestellt ist die gespleisste mRNA der leichten Kette und von zwei differenziell gespleissten mRNAs der schweren Kette, die eine sekretorische Variante (sIgG1), bzw. eine Membran-gebundene Variante (mIgGl) eines IgG1 codieren.
C. IgG1-Antikörperprotein. Dargestellt ist die leichte Antikörperkette mit vL- und CL-Domäne. Das Leaderpeptid ist abgespalten. Die schwere Antikörperkette existiert in 2 Varianten,
   _ mit vH, CH1, H, CH2, CH3-Domäne (sekretierte Form; sIgGl) bzw.
   _mit vH, CH1, H, CH2, CH3, M1 und M2-Domäne (Membranständige Form; mIgG1).

### Figur 3

Homologe Rekombination. Mittels homologer Rekombination können die DNA-Sequenzen definierter Genloci verändert werden. Diese Technik steht derzeit v.a. im Zentrum der Herstellung veränderter ES-Zelllinien, bzw. transgener Mäuse. Dabei gibt der Experimentator zunächst eine definierte erste DNA-Sequenz vor, die an beiden Seiten von weiteren DNA-Sequenzen flankiert wird. Diese weiteren DNA-Sequenzen sollten i.d.R. >700 Bp lange homologe Bereiche enthalten, die ihre Entsprechung auf den Chromosomen finden: Den dadurch definierbaren Genloci. Innerhalb dieser weiteren DNA-Sequenzen müssen Rekombinationsereignisse stattfinden (dargestellt durch gekreuzte Linien), damit die zwischen den weiteren DNA-Sequenzen liegenden chromosomalen Bereiche zwischen der chimären und der chromosomalen DNA ausgetauscht werden. Nach der Transfektion geeigneter chimärer, insbesondere linearisierter DNA (dargestellt oberhalb der chromosomalen DNA) findet dieses sehr seltene Ereignis in ca. 1 aus 10⁷ Zellen statt. In der Figur dargestellt sind Genloci der im Sinne dieser Erfindung dafür geeigneten Maus-Hybridomzelllinie HEA125 (siehe auch Figur 2). Wird auf Grund einer homologen Rekombination der auf der Oberfläche präsentierte Antikörper verändert, so kann die entsprechende Zelle mittels FACS isoliert werden. Je nachdem, wie die genannten weiteren DNA-Sequenzen gewählt werden, entscheidet der Experimentator, ob zusätzlich zur Einführung der genannten ersten DNA-Sequenz nach der homologen Rekombination mehr oder weniger grosse Bereiche deletiert werden. In der Figur dargestellt sind:
I.Der Austausch des Exons für die konstante Maus-Kappa-Domäne gegen ein Exon, das eine konstante Mensch-Kappa-Domäne codiert,
II.Der Austausch der CH1, CH2 und CH3 Exons für die konstanten Maus-IgG1-Domänen gegen die entsprechenden Exons der konstanten Mensch-IgG1-Domänen (das Hinge-Exon ist nicht dargestellt),
III.Wie (II.), wobei zusätzlich DNA-Sequenzen des Introns zwischen CH3-Dömane und M1-Domäne des codierten IgG1 deletiert werden,
IV.Der Austausch des Exons für die aktive variablevL1-Domäne gegen ein Exon, das eine z.B. im FACS selektionierbare vL2-Domäne codiert, wobei gleichzeitig zwei spezifische Rekombinationssignale eingeführt werden (FRT0 und FRT3), die von der Rekombinase Flp erkannt werden und
V.Der Austausch des Exons für die aktive variablevH1-Domäne gegen ein Exon, das eine z.B. im FACS selektionierbare vH2-Domäne codiert, wobei gleichzeitig zwei spezifische Rekombinationssignale eingeführt werden (loxP1 und loxP2), die von der Rekombinase Cre erkannt werden.

Werden die in der Figur dargestellten homologen Rekombinationsereignisse I, III, IV und V nacheinander durchgeführt und die entsprechenden Zelllinien isoliert, so entsteht eine Hybridom-Zelllinie, die einen definierten monoklonalen Antikörper in vergleichsweise grosser Zahl auf ihrer Oberfläche präsentiert. Die variablen Domänen dieses monoklonalen Antikörpers können auf Grund der eingeführten spezifischen Rekombinationssignale vergleichsweise einfach gegen die entsprechenden Domänen anderer Antikörper oder von Scharen von Antikörpern ausgetauscht werden.

### Figur 4

Umbau einer menschlichen Zelllinie durch homologe Rekombination. Im Unterschied zu den in Figur 3 dargestellten homologen Rekombinationsereignissen ist in Figur 4 die Veränderung einer menschlichen Zelllinie (z.B. IM-9 oder U266) durch verschiedene homologe Rekombinationsereignisse dargestellt. In der Figur dargestellt sind:
III. (b.) Der Austausch der CH1, CH2 und CH3 Exons z.B. für die konstanten Mensch-IgA-Domänen gegen die entsprechenden Exons der konstanten Mensch-IgG1-Domänen, wobei zusätzlich DNA-Sequenzen des Introns zwischen CH3-Dömane und M1-Domäne des codierten IgG1 / IgA deletiert werden,
IV. Der Austausch des Exons für die aktive variablevL1-Domäne gegen ein Exon, das eine z.B. im FACS selektionierbare vL2-Domäne codiert, wobei gleichzeitig zwei spezifische Rekombinationssignale eingeführt werden (FRT0 und FRT3), die von der Rekombinase Flp erkannt werden (wie in Figur 3) und
V. Der Austausch des Exons für die aktive variablevHl-Domäne gegen ein Exon, das eine z.B. im FACS selektionierbare vH2-Domäne codiert, wobei gleichzeitig zwei spezifische Rekombinationssignale eingeführt werden (loxP1 und loxP2), die von der Rekombinase Cre erkannt werden (wie in Figur 3).

### Figur 5

Spezifische Rekombination. Mittels spezifischer Rekombination können chromosomale DNA-Sequenzen, bzw. definierte Genloci mit vergleichsweise hoher Effizienz verändert werden. Dabei rekombinieren DNAs unter dem Einfluss einer Rekombinase wie Cre oder Flp an spezifischen Rekombinationssignalen miteinander, wodurch die von diesen Signalen flankierten DNA-Sequenzen mit ausgetauscht werden. Bei dieser Art von Kassettenaustausch wird die auszutauschende DNA jeweils von zwei unterschiedlichen Rekombinationssignalen flankiert, die nicht miteinander reagieren. In der Figur dargestellt ist der Kassettenaustausch von DNA-Sequenzen mittels Cre oder Flp. Nach der Transfektion geeigneter chimärer, insbesondere zirkulärer DNA (dargestellt oberhalb der chromosomalen DNA), integriert die zirkuläre DNA zunächst durch Rekombination an einem der beiden spezifischen Rekombinationssignale ins Chromosom (dargestellt durch gekreuzte Linien). Anschliessend wird eine wiederum zirkuläre DNA auf Grund der Aktion der Rekombinase ausgeschnitten (nicht dargestellt). Dies geschieht zufällig, entweder an denselben Rekombinationssignalen wie bei der Integration, oder bei den beiden anderen Rekombinationssignalen. Dadurch stellt sich ein Gleichgewicht aus ursprünglichen und ausgetauschten DNA-Sequenzen ein. Wird daraufhin ein veränderter Antikörper auf der Oberfläche präsentiert (siehe auch Figur 2), so kann die entsprechende Zelle mittels FACS isoliert werden. In der Figur dargestellt sind:
VI. Der Austausch des Exons für eine definierte vKappa - Domäne gegen ein Exon aus einer Schar unterschiedlicher Exons, die jeweils andere-vKappa-Domänen codieren,
VII. Der Austausch des Exons für eine definierte vLambda - Domäne gegen ein Exon aus einer Schar unterschiedlicher Exons, die jeweils andere-vLambda-Domänen codieren und
VIII. Der Austausch des Exons für eine definierte vH - Domäne gegen ein Exon aus einer Schar unterschiedlicher Exons, die jeweils andere-vH-Domänen codieren.

Werden die in der Figur dargestellten spezifischen Rekombinationsereignisse VI und VIII, bzw. VII und VIII nacheinander durchgeführt und die entsprechende Schar von Zellen angereichert, so entsteht eine Hybridom-Antikörper-Bibliothek, deren einzelne Vertreter (Zellen) jeweils einen definierten monoklonalen Antikörper in vergleichsweise grosser Zahl auf ihrer Oberfläche präsentieren. Daraus isolierte Hybridomzellen produzieren monoklonale, insbesondere humane Antikörper in guter Ausbeute und Qualität.

### Figur 6

Einführung spezifischer Rekombinationssignale in aktive Genloci. In der Figur dargestellt ist:
I. Die Transfektion einer Zelllinie mit Vektor-DNA, die ein Resistenzgen (z.B. G418-Resistenz) codiert, das von 2 verschiedenen FRT-Stellen flankiert ist und die Selektion (z.B. mit G418) einer Zelllinie, die das Resistenzgen zusammen mit den FRT-Stellen chromosomal in einen aktiven Genlocus integriert trägt.
II. Der Kassettenaustausch des Resistenzgens mittels spezifischer Rekombination durch Flp gegen DNA-Sequenzen, die die Hinge (nicht dargestellt), CH2, CH3, M1 und ggfs. M2 Exons eines IgG-Antikörpers fusioniert an ein erstes Gen eines ersten scFv-Antikörpers codieren (siehe auch Figur 2). Das Gen des ersten scFv-Antikörpers ist von zwei unterschiedlichen loxP-Stellen flankiert. Die Selektion dieses Rekombinationsereignisses erfolgt z.B. im FACS durch die Oberflächenpräsentation des scFv-Antikörper-Fusionsproteins auf der Zelloberfläche.
III. Der Austausch des ersten Gens eines ersten scFv-Antikörpers gegen ein weiteres Gen aus einer Schar von weiteren Genen weiterer scFv-Antikörper mit Hilfe von Cre.
IV. Werden die in der Figur dargestellten Rekombinationsereignisse I, II und III nacheinander durchgeführt und die entsprechende Schar von Zellen angereichert, so entsteht eine scFv-Antikörper-Bibliothek, deren einzelne Vertreter (Zellen) jeweils einen definierten monoklonalen scFv-Antikörper in vergleichsweise grosser Zahl auf ihrer Oberfläche präsentieren. Daraus isolierte Zellen produzieren monoklonale, insbesondere humane scFv-CH2-CH3-Antikörper in guter Ausbeute und Qualität.

Ggfs. kann die in der Figur 6, II. dargestellte zirkuläre DNA-Sequenz mit einem ersten scFv-Antikörpergen weitere scFv-Antikörpergene enthalten, die jeweils von einer loxP und einer loxP511-Stelle flankiert sind (z.B. loxP scFv1 loxP511 loxP scFv2 loxP511 ... loxP scFvN loxP511). Damit ergibt eine einrekombinierte DNA-Sequenz durch die Aktivität von Cre innerhalb dieser Zelle viele verschiedene Antikörperpräsentierende Zellen. Durch die Kombination einer Vielzahl von vH- und von vL-Exons kann diese genannte Vielzahl potenziert werden.

### Figur 7

Humanisierung einer Maus-Hybridom-Zelllinie (2) durch homologe Rekombination. Dargestellt ist der Phänotyp verschiedener veränderter Hybridomzellen (2Kh, 2h, 2H), nachdem die in Figur 3 unter (I.) und (II.) oder (III.) beschriebenen homologen Rekombinationsereignisse durchgeführt wurden. Die sehr seltenen homologen Rekombinationsereignisse finden in ca. 1 aus 10⁷ Zellen statt. Die veränderten Zellen müssen anschliessend z.B. im FACS selektioniert werden. Im Zentrum der Figur ist beispielhaft eine homolog rekombinierte Hybridomzelle (2H) dagestellt, bei der zunächst das in Figur 3 unter (I.) und anschliessend das unter (III.) beschriebene homologe Rekombinationsereignis stattgefunden hat. In der Figur dargestellt ist:
I. Der Austausch des Exons für die konstante Maus-Kappa-Domäne gegen ein Exon, das eine konstante Mensch-Kappa-Domäne codiert führt zur Oberflächen-Präsentation einer konstanten Mensch-Kappa-Domäne, während die Antigen-Spezifität des präsentierten Antikörpers gleich bleibt (2kh),
II. Der Austausch der CH1, CH2 und CH3 Exons für die konstanten Maus-IgG1-Domänen gegen die entsprechenden Exons der konstanten Mensch-IgG1-Domänen führt zur Oberflächen-Präsentation konstanter Mensch-IgG1-Domänen, während die Antigen-Spezifität des präsentierten Antikörpers gleich bleibt (2h) und
III. Wie (II.), wobei zusätzlich DNA-Sequenzen des Introns zwischen CH3-Dömane und M1-Domäne des codierten IgG1 (siehe Figur 2) deletiert werden, so dass deutlich mehr Antikörper auf der Oberfläche der Hybridomzelle präsentiert werden (2H).

Werden die in der Figur dargestellten homologen Rekombinationsereignisse I und II oder I und III nacheinander durchgeführt und die entsprechenden Zelllinien isoliert, so entsteht eine Hybridom-Zelllinie, die einen humanisierten monoklonalen Antikörper mit der ursprünglichen Antigen-Spezifität produziert (2H).

### Figur 8

Einführung spezifischer Rekombinationssignale bei gleichzeitiger Veränderung der Antikörper-Spezifität einer Hybridom-Zelllinie (2H) durch homologe Rekombination.

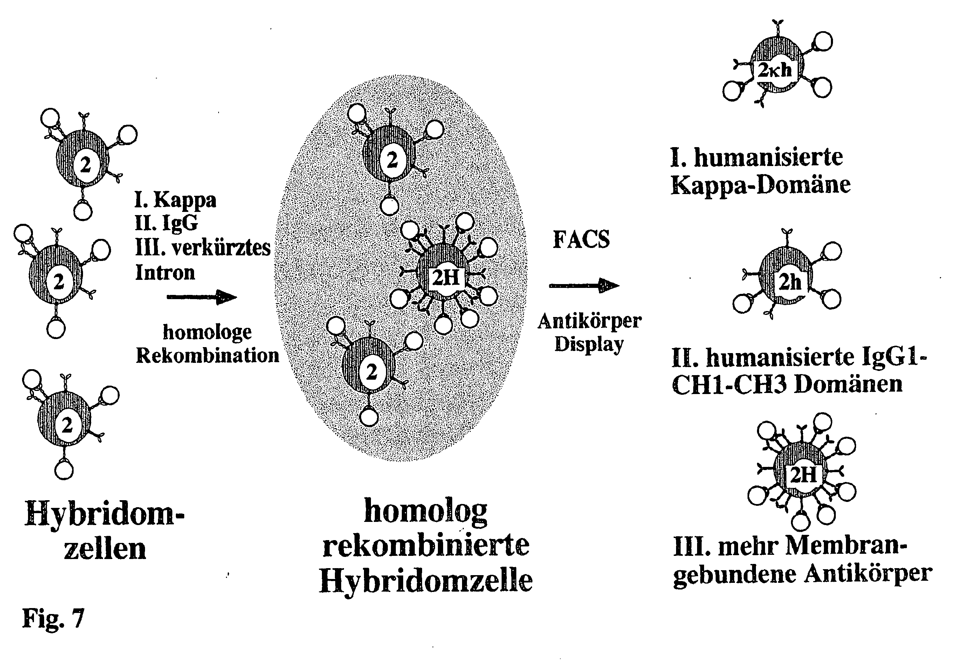

Dargestellt ist der Phänotyp der veränderten Hybridomzellen (3aH, 3H), nachdem die in Figur 3 unter (IV.) und (V.) beschriebenen homologen Rekombinationsereignisse durchgeführt wurden. Die veränderten Zellen werden anschliessend z.B. im FACS selektioniert. Die Ausgangszelllinie (2H) ist in Figur 7 beschrieben worden. Im Zentrum der Figur ist beispielhaft eine homolog rekombinierte Hybridomzelle dagestellt (3H), bei der zunächst das in Figur 3 unter (IV.) und anschliessend das unter (V.) beschriebene homologe Rekombinationsereignis stattgefunden hat. In der Figur dargestellt sind:
IV. Der Austausch des Exons für die aktive variablevL1-Domäne gegen ein Exon, das eine z.B. im FACS selektionierbare vL2-Domäne codiert, führt zur Oberflächen-Präsentation einer veränderten vL-Domäne, wodurch sich die Antigen-Spezifität des präsentierten Antikörpers (3aH) ändert, während gleichzeitig 2 FRT-Stellen eingeführt werden und
V. Der anschliessende Austausch des Exons für die aktive variablevHl-Domäne gegen ein Exon, das eine z.B. im FACS selektionierbare vH2-Domäne codiert, führt zur Oberflächen-Präsentation einer veränderten vH-Domäne, wodurch sich die Antigen-Spezifität des präsentierten Antikörpers (3H) ändert, während gleichzeitig 2 loxP-Stellen eingeführt werden.
   Werden die (auch in der Figur 3) dargestellten homologen Rekombinationsereignisse IV und V nacheinander durchgeführt und die entsprechenden Zellen isoliert, so entsteht eine veränderte Hybridom-Zelllinie, die einen veränderten humanisierten monoklonalen Antikörper auf ihrer Oberfläche präsentiert. Die Antigen-Spezifität dieses Antikörpers hängt von den einrekombinierten variablen Domänen ab.

### Figur 9

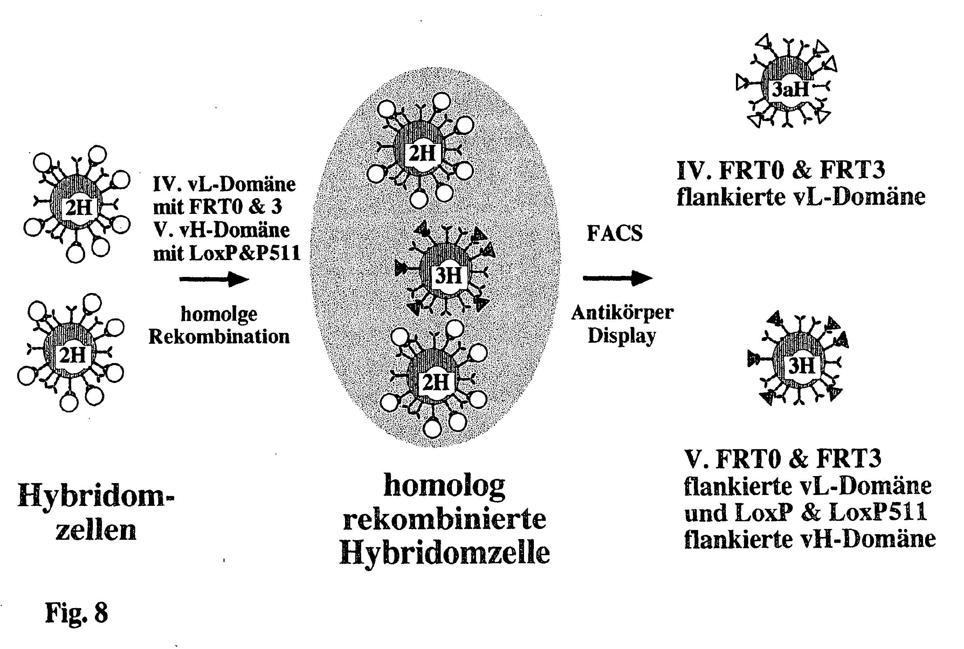

Herstellung einer Hybridom-Antikörper-Bibliothek (2H, 3H, 4H, 5H, 6H) durch spezifische Rekombination. Dargestellt ist der Phänotyp der veränderten Hybridomzellen (2H, 3H, 4H, 5H, 6H), nachdem zunächst die in Figur 3 unter (I.), (III.) (IV.) und (V.) beschriebenen homologen und anschliessend die in Figur 5 unter (VI. & VIII.) bzw. unter (VII. & VIII.) beschriebenen spezifischen Rekombinationsereignisse durchgeführt wurden. Die Ausgangszelllinie (3H) ist in Figur 8 beschrieben worden. Die in Figur 9 dargestellte Hybridomzelllinie (3H) wurde jeweils mit einer Schar unterschiedlicher DNA-Sequenzen transfiziert und anschliessend eine Schar von Phänotypen nach spezifischen Rekombinationsereignissen im FACS selektioniert. In der Figur dargestellt sind:
IV. Der Austausch des Exons für die aktive variablevL3-Domäne gegen eine Schar von Exons, führt zur Oberflächen-Präsentation einer Schar unterschiedlich veränderter vL-Domänen und
V. Der anschliessende Austausch des Exons für die aktive variablevH3-Domäne gegen eine Schar von Exons, führt zur Oberflächen-Präsentation einer Schar unterschiedlich veränderter vH-Domänen wodurch sich die Antigen-Spezifität der präsentierten Antikörper (2H, 3H, 4H, 5H, 6H) ändert.

Werden die in der Figur dargestellten spezifischen Rekombinationsereignisse IV und V mit einer Schar von Zellen bzw. einer Schar unterschiedlicher DNA-Sequenzen nacheinander durchgeführt und die entsprechenden Scharen unterschiedlicher Zellen angereichert, so entsteht eine Hybridom-Antikörper-Bibliothek, deren einzelne Vertreter jeweils unterschiedliche humanisierte monoklonale Antikörper auf ihrer Oberfläche präsentieren. Abgereichert werden bei diesem Verfahren die Ausgangszellen (3H) und fehlerhaft bzw. unproduktiv rekombinierte Zellen (0H).

### Figur 10

"On-line" Affinitätsvergleich präsentierter Antikörper. Die Affinität eines Antikörpers an sein Antigen ist ein Mass für das Verhältnis Antikörper-gebundener Antigene vs freier Antikörper und freier Antigene in Lösung (allgemein: Rezeptor vs Ligand). Dies ermöglicht zeitgleich mit der Selektion mittels FACS den direkten Affinitätsvergleich unterschiedlicher Oberflächen-präsentierter Antikörper oder unterschiedlicher präsentierter Proteine (bzw. der entsprechenden Hybridomzellen) durch Fluoreszenz-markierte Antigene (rote Dreiecke). Um die Zahl der jeweils pro Zelle präsentierten Antikörper zu normalisieren, können diese z.B. mit FITC-markiertem Protein G gegengefärbt werden (grüne Kreise). Im dargestellten Beispiel bindet der von der Hybridomzelle B präsentierte Antikörper deutlich affiner an das Antigen, verglichen mit dem von der Zelle A präsentierten Antikörper. Das Mass für die Affinität ist in diesem Fall der Quotient von roter zu grüner Fluoreszenz der im FACS sortierten Zellen.

### Figur 11

Somatische Hypermutation. Zunächst werden, wie in Figur 9 beschrieben, durch spezifische Rekombination die Exons der aktiven variablen vL2-Domäne gegen eine Schar von Exons ausgetauscht, die jeweils eine unterschiedlich mutierte vL2-Domäne codieren (siehe auch Figur 5 und 6). Dabei entstehen unproduktive Mutationen (2d), schwächer affine Antikörper (2c und 2e) und in seltenen Fällen höher affine Antikörper (2b). Diese können, wie in Figur 10 beschrieben im FACS sortiert werden. Alternativ führt die Expression von RAD54, RecQ4 und gleichzeitig von polX mu innerhalb der Hybridomzelllinie (2) zur Einführung ungerichteter Mutationen innerhalb von ca. 1,5 kb downstream der jeweiligen Promotoren für die aktive leichte und schwere Antikörperkette. Vorzugsweise wird dabei gleichzeitig anti-sense RNA oder siRNA gegen XRCC2, XRCC3 oder RAD51B exprimiert. Voraussetzung für diesen alternativen Weg zur Einführung somatischer Hypermutationen ist der Kontext des aktiven Antikörpergenlocus.

### Figur 12

Chimäre Maus-Mensch-DNA zur Humanisierung der Hybridomzelllinie HEA125.
A. Humanisierung der konstanten Kappa-Domäne mit Hilfe des DNA-Vektors pBS MhKappaM. Dargestellt ist der Vektor pBS MhKappaM mit den chimären DNA-Sequenzen zur Humanisierung der konstanten Kappa-Domäne der Hybridomzelllinie HEA125. Klonierungsvektor war pBSIISK+ von Stratagene. Restriktionsstellen sind unterstrichen. Sequenzierungsprimer sind blau dargestellt. Die cyan und unterstrichen dargestellten PCR-Primer HK1 und HK2 dienten zur Vervielfältigung des humanen Exons, das die konstante Kappa Kette codiert. Template DNA war dabei menschliche genomische DNA. Die codierende Sequenz des konstanten humanen Kappa-Domänen-Exons ist grün und kleingeschrieben dargestellt. Die rot und kleingeschrieben dargestellten PCR-Primer MK1 und MK2 bzw. MK3 und MK4 dienten zur Vervielfältigung der flankierenden homologen Bereiche des Maus-Genoms von HEA125. Template DNA war dabei genomische DNA von HEA125. Die Grenze zwischen Maus und humanen Sequenzen wird durch die Restriktionsstellen NotI bzw. BstBl markiert. Vor der Elektroporation in HEA125-Zellen wurde die Vektor-DNA mit dem Restriktionsenzym BglI linearisiert. Die cyan unterstrichen dargestellten PCR-Primer HK3 und HK4 dienten (in Kombination mit der Oberflächenpräsentation humanisierter Antikörper) zur Sequenzierung und Verifizierung der durch homologe Rekombination veränderten Subklone der Hybridomzelle HEA125.
B. Humanisierung der konstanten IgG1-CH1, CH2 und CH3-Domänen mit Hilfe des DNA-Vektors pBS MhIgG1M. Dargestellt ist der Vektor pBS MhIgG1M mit den chimären DNA-Sequenzen zur Humanisierung der konstanten IgG1-CH1, CH2 und CH3-Domänen der Hybridomzelllinie HEA125. Klonierungsvektor war pBSIISK+ von Stratagene. Restriktionsstellen sind unterstrichen. Die cyan unterstrichen dargestellten PCR-Primer HG1 und HG2 dienten zur Vervielfältigung der humanen Exons, die die konstanten IgG1-Domänen codieren. Template DNA war dabei menschliche genomische DNA. Die rot und kleingeschrieben dargestellten PCR-Primer MG1 und MG2 bzw. MG3 und MG4 dienten zur Vervielfältigung der flankierenden homologen Bereiche des Maus-Genoms von HEA125. Template DNA war dabei genomische DNA von HEA125. Die Grenze zwischen Maus und humanen Sequenzen wird durch zwei HindIII-Restriktionsstellen markiert. Codierende humane Sequenzen sowie die Maus-M1- und Maus-M2-Exons sind mit grünen Kleinbuchstaben dargestellt. Vor der Elektroporation in HEA125-Zellen wurde die Vektor-DNA mit dem Restriktionsenzym SspI linearisiert. Die cyan unterstrichen dargestellten PCR-Primer HG3 und HG4 dienten (in Kombination mit der Oberflächenpräsentation humanisierter Antikörper) zur Sequenzierung und Verifizierung der durch homologe Rekombination veränderten Subklone der Hybridomzelle HEA125.
   Blau kursiv unterstrichen sind 5 verschiedene Primer dargestellt (Primer delta1 bis delta5), mit deren Hilfe zwischen ca. 350 Bp und ca. 900 Bp-lange DNA-Sequenzen zwischen der nächstgelegenen HindIII-Stelle und den jeweiligen Primern deletiert werden können. Dazu wird eine PCR mit dem Primer MG4 und den jeweiligen Primern delta1 bis delta5 durchgeführt. Diese besitzen einen zusätzlichen nicht dargestellten HindIII-Überhang. Damit können die 5 PCR-Banden in HindIII (Teilverdau) und EagI geschnittenen Vektor pBS MhIgG1M einligiert werden. Die dadurch erhaltenen Deletionen im Intron zwischen CH3 und M1-Domäne führen zu einer verstärkten Oberflächenexpression. Der Vektor pBS MhIgG1Mdelta350 ist identisch mit dem Vektor pBS MhIgG1M, es fehlen darin jedoch die Sequenzen zwischen den Primern MG3 (inclusive der MG3-Primer-Sequenzen) und delta1 (exclusive der delta1-Primer-Sequenzen).

### Figur 13

Chimäre Maus-G418-Resistenz-DNA zur Einführung spezifischer Rekombinationssignale in den vH- und vKappa Genlocus der Hybridomzelllinie HEA125.
A. Einführung von FRT-Stellen in den vKappa-Genlocus der Hybridomzelllinie HEA125 mit Hilfe des DNA-Vektors pBS MKappaG418M. Dargestellt ist der Vektor pBS MKappaG418M mit den chimären DNA-Sequenzen zur Einführung von FRT-Stellen in den aktiven vKappa Genlocus der Hybridomzelllinie HEA125. Klonierungsvektor war pBSIISK+ von Stratagene. Restriktionsstellen sind unterstrichen. Das Resistenzgen PGKneo wurde mit den PCR-Primern Neo1 und Neo2 vervielfältigt (rot kleingeschrieben unterstrichen dargestellt) und anschliessend mit AatII einkloniert. Als Quelle bzw. Template für das Resistenzgen PGKneo diente dabei der Vektor ploxPfrtPGKneofrtloxP (Dr. Erich Greiner, dkfz, Abteilung Molekularbiologie der Zelle I). Die codierende Sequenz des Neophosphoryltransferase II Gens und des Maus-vKappa-Leader-Exons ist grün und kleingeschrieben dargestellt. Das Neophosphoryltransferase II Gen steht unter der Kontrolle des Promotors für die Phosphoglycerin Kinase (PGK; cyan kursiv dargestellt). Die rot und kleingeschrieben dargestellten PCR-Primer MVK1 und MVK2 bzw. MVK3 und MVK4 dienten zur Vervielfältigung der die vKappa-Domäne flankierenden homologen Bereiche des Maus-Genoms von HEA125. Template DNA war dabei genomische DNA von HEA125. Die genannten Primer brachten in einer nachgeschalteten PCR zusätzlich die Sequenzen der jeweils flankierenden FRT-Stelle und eine halbe AatII-Stelle mit. Die Grenze zwischen Maus und PGKneo-Sequenzen wird durch die kursiv blau dargestellten FRT0- bzw. FRT3-Stellen markiert. Vor der Elektroporation in HEA125-Zellen wurde die Vektor-DNA linearisiert. Die cyan und unterstrichen dargestellten PCR-Primer KG418-3 und KG418-4 (sowie Primer ausserhalb der dargestellten Regionen) dienten zur Sequenzierung und Verifizierung der durch homologe Rekombination veränderten Subklone der Hybridomzelle HEA125.
B. Einführung von loxP-Stellen in den vH-Genlocus der Hybridomzelllinie HEA125 mit Hilfe des DNA-Vektors pBS MvHG418M. Dargestellt ist der Vektor pBS MvHG418M mit den chimären DNA-Sequenzen zur Einführung von loxP-Stellen in den aktiven vH-Genlocus der Hybridomzelllinie HEA125. Klonierungsvektor war pBSIISK+ von Stratagene. Restriktionsstellen sind unterstrichen. Das Resistenzgen PGKneo wurde mit AatII einkloniert, die Quelle der DNA war dieselbe wie in (A.) dargestellt. Die codierende Sequenz des Neophosphoryltransferase II Gens ist grün und kleingeschrieben dargestellt. Der PGK-Promotor ist cyan kursiv dargestellt. Dem Vektor pBS MvHG418MdeltaPGK fehlen die cyan kursiv dargestellten PGK-Promotor-Sequenzen, ansonsten ist dieser Vektor identisch mit pBS MvHG418M. Um diesen Vektor herzustellen, wurde das Neophosphoryltransferase II Gen mit den PCR-Primern Neo2 und Neo3 (rot kleingeschrieben unterstrichen dargestellt) vervielfältigt. Dabei brachten die Primer am 5'-Ende jeweils überhängende AatII-Stellen mit. Template war dabei der Vektor ploxPfrtPGKneofrtloxP. Die rot und kleingeschrieben dargestellten PCR-Primer MvH1 und MvH2 bzw. MvH3 und MvH4 dienten zur Vervielfältigung der die vH-Domäne flankierenden homologen Bereiche des Maus-Genoms von HEA125. Template DNA war dabei genomische DNA von HEA125. Die genannten Primer brachten in einer nachgeschalteten PCR zusätzlich die Sequenzen der jeweils flankierenden loxP-Stelle und eine halbe AatII-Stelle mit. Die Grenze zwischen Mausgenomischen und PGKneo-Sequenzen wird durch die kursiv blau dargestellten loxP- bzw. loxP511-Stellen markiert. Vor der Elektroporation in HEA125-Zellen wurde die Vektor-DNA linearisiert. Die cyan und unterstrichen dargestellten PCR-Primer vHG418-3 und vHG418-4 (sowie Primer ausserhalb der dargestellten Regionen) dienten zur Sequenzierung und Verifizierung der durch homologe Rekombination veränderten Subklone der Hybridomzelle HEA125.

### Figur 14

Primer für die Vervielfältigung der Vielfalt genomisch rekombinierter humaner Antikörpergene. Dargestellt sind Primer, mit denen die genomische DNA der entsprechenden variablen Gene vervielfältigt werden kann. Als GegenstrangPrimer dienen die dazugehörigen J-Segment-Primer. Die humanen Gensequenzen wurden anhand des Buches Immunoglobulin Facts Book (Lefranc und Lefranc, 2001, Academic Press, ISBN 0-12-441351-X) identifiziert. Die DNA-Sequenzen der darin aufgeführten variablen Antikörper-Gene wurden anhand der Acession Numbers aus der öffentlich zugänglichen Datenbank Genbank importiert und anschliessend vH-Gen-spezifische Primer entworfen. Diese hybridisieren jeweils ca. 182 Bp vom 5'-Ende des ATG-Startcodons des Leader-Exons entfernt. Mit vKappa und vLambda-spezifischen PCR-Primern wurde analog verfahren. Diese hybridisieren innerhalb des Introns zwischen Leader-Exon und vL-Exon. Die 5'-Enden dieser Primer hybridisieren jeweils ca. 130 Bp vom 5'-Ende des vL-Exons entfernt. Die 5'-Enden der J_{H-}Segment-spezifischen Gegenstrangprimer hybridisieren ca. 83 Bp vom 3'-Ende der J_{H}-Segmente in 3'-Richtung gerechnet. Die 5'-Enden der Jₖₐₚₚₐ- und J_{Lambda}-Segment-spezifischen Gegenstrangprimer hybridisieren ca. 89 Bp vom 3'-Ende der J_{L-}Segmente in 3'-Richtung gerechnet. Mit den aufgeführten PCR-Primern und genomischer DNA aus humanen peripheren Lymphocyten als Template wurden bei 65°C (+/-5°C) folgende PCRs durchgeführt:
■ 4 J_{Lambda}-Segmente x 24 vLambda-Gene = 96 vLambda-spezifische PCRs;
■ 5 J_{Kappa}-Segmente x 34 vKappa-Gene = 170 vKappa-spezifische PCRs; und
■ 6 J_{H}-Segmente x 44 vH-Gene = 264 vH-spezifische PCRs,
   A. humane vLambda-Primer und J_{Lambda}-Primer
   B. humane vKappa-Primer und J_{Kappa}-Primer
   C. humane vH-Primer und J_{H}-Primer

### Figur 15

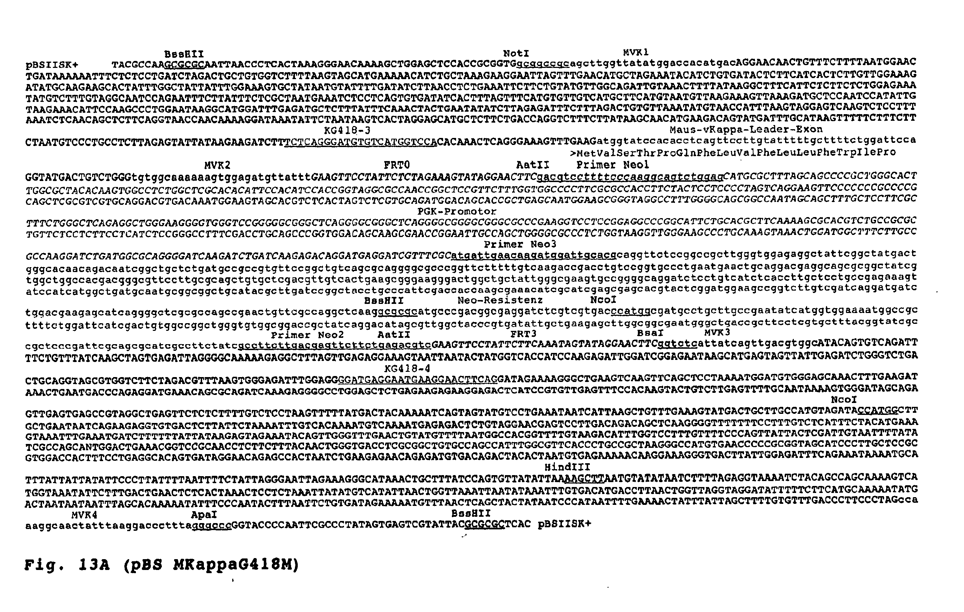

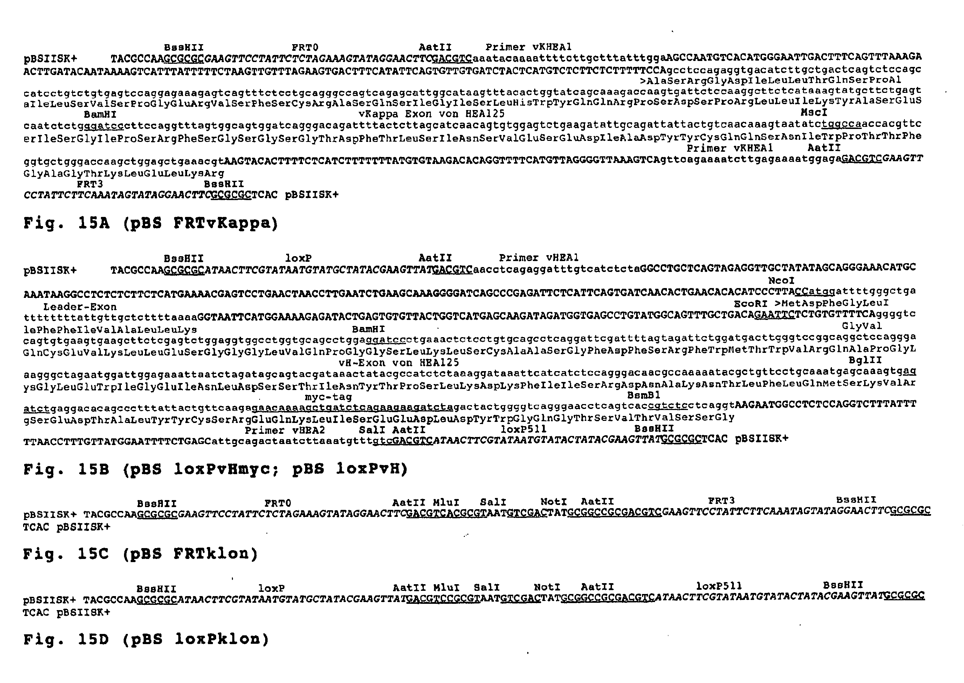

Vektoren für die Einführung variabler Exons in den vH- und vKappa Genlocus der Hybridomzelllinie HEA125mittels spezifischer Rekombination.
A. Dargestellt ist der Vektor pBS FRTvKappa, durch den mit Hilfe von Flp die ursprüngliche vKappa-Domäne von HEA125 in den vKappa-Genlocus der Hybridomzelllinie HEA125 wieder einrekombiniert werden kann. Restriktionsstellen sind unterstrichen. Die codierenden Sequenzen des vKappa-Exons sind grün und kleingeschrieben dargestellt. Die rot und kleingeschrieben dargestellten PCR-Primer vKHEA1 und vKHEA2 dienten zur Vervielfältigung der genomischen DNA des aktiven vKappa-Gens von HEA125. Template DNA war dabei genomische DNA von HEA125. Die genannten Primer brachten in einer nachgeschalteten PCR zusätzlich die Sequenzen der jeweils flankierenden FRT-Stelle und eine BssHII-Stelle mit. Mit Hilfe von BssHII wurde das PCR-Fragment in pBSIISK+ kloniert. Die Grenze der genomischen vKappa-Sequenzen wird durch die kursiv blau dargestellten FRT0- bzw. FRT3-Stellen markiert. Die in Figur 13A cyan und unterstrichen dargestellten PCR-Primer KG418-3 und KG418-4 dienten zur Sequenzierung und Verifizierung der durch spezifische Rekombination veränderten Subklone der Hybridomzelle HEA125.
   Die Sequenz des aktiven HEA125-vKappa-Genlocus ergibt sich aus den Figuren 15A (aktives genomisch rekombiniertes vKappa-Exon) und 13A (5'- und 3'-flankierende Bereiche), wobei in den Figuren zwei zusätzliche im HEA125-Genom nicht vorhandene FRT-Stellen dargestellt sind.
B. Dargestellt ist der Vektor pBS loxPvHmyc, durch den mit Hilfe von Cre die ursprüngliche vH-Domäne von HEA125 (+ myc-tag) in den vH-Genlocus der Hybridomzelllinie HEA125 wieder einrekombiniert werden kann. Restriktionsstellen sind unterstrichen. Die codierenden Sequenzen des Leader-Exons und des vH-Exons sind grün und kleingeschrieben dargestellt. Die rot und kleingeschrieben dargestellten PCR-Primer vHEA1 und vHEA2 dienten zur Vervielfältigung der genomischen DNA des aktiven vH-Gens von HEA125. Template DNA war dabei genomische DNA von HEA125. Die genannten Primer brachten in einer nachgeschalteten PCR zusätzlich die Sequenzen der jeweils flankierenden loxP-Stelle und eine BssHII-Stelle mit. Mit Hilfe von BssHII wurde das PCR-Fragment in pBSIISK+ kloniert. Die Grenze der genomischen vH-Sequenzen wird durch die kursiv blau dargestellten loxP- bzw. loxP511-Stellen markiert. Zusätzlich zu den natürlich in HEA125 vorkommenden Sequenzen wurde im Bereich des CDR3 ein magenta unterstrichen dargestelltes myc-tag eingeführt. Dazu wurde die dargestellte DNA-Sequenz synthetisiert und mit Hilfe der flankierenden Restriktionsstellen BsmB1 und BglII einkloniert. Abgesehen von diesem myc-tag ist der Vektor pBS loxPvH identisch mit dem Vektor pBS loxPvHmyc.

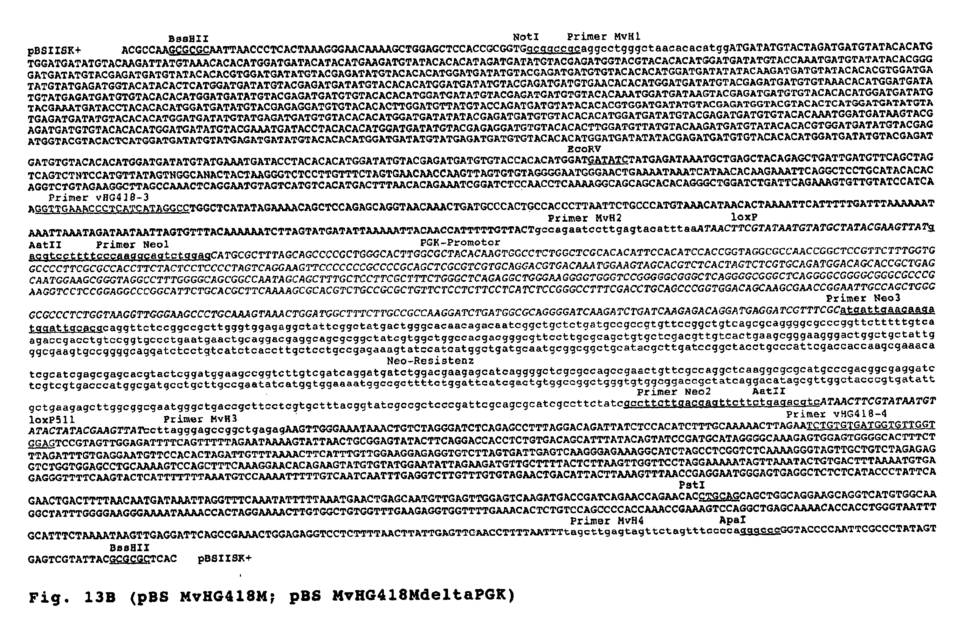

   Die in Figur 13B cyan und unterstrichen dargestellten PCR-Primer vHG418-3 und vHG418-4 dienten zur Sequenzierung und Verifizierung der durch spezifische Rekombination veränderten Subklone der Hybridomzelle HEA125.
   Die Sequenz des aktiven HEA125-vH-Genlocus ergibt sich aus den Figuren 15B (aktives genomisch rekombiniertes vH-Exon) und 13B (5'- und 3'-flankierende Bereiche), wobei in den Figuren zwei zusätzliche im HEA125-Genom nicht vorhandene loxP-Stellen dargestellt sind. Zusätzlich ist in Figur 15B im CDR3 der vH-Domäne ein myc-tag dargestellt, das ebenfalls in dem ursprünglichen HEA125-Genom nicht vorhanden ist.
C. Dargestellt ist der Klonierungsvektor pBS FRTklon.
D. Dargestellt ist der Klonierungsvektor pBS loxPklon.

### Figur 16

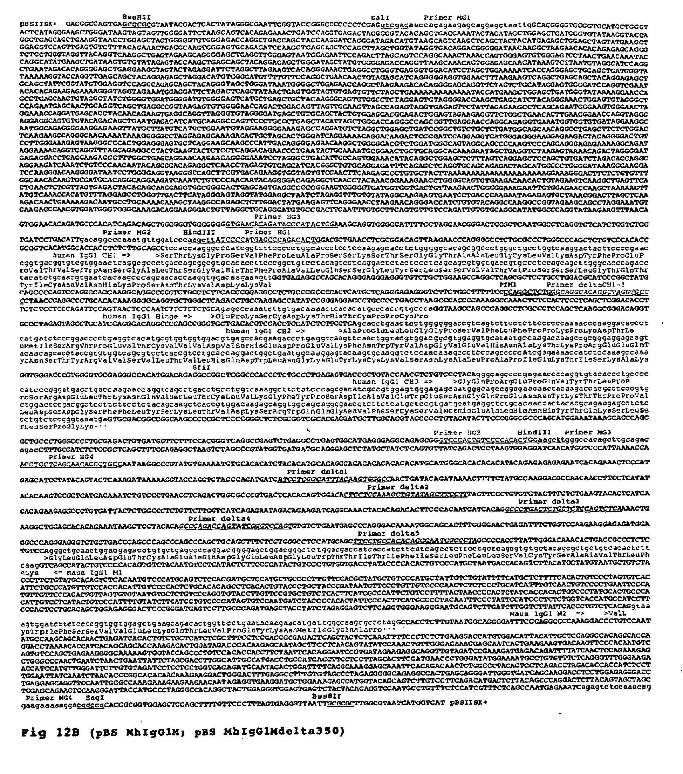

Vektor für die Einführung spezifischer Rekombinationssignale in vorselektionierte Genloci. Dargestellt ist der Vektor pBS loxP-IgG1, durch den mit Hilfe von Flp die konstanten Domänen einschliesslich der Membran-Domänen M1 und M2 eines IgG1 z.B. in eine vorselektionierte FRT-Kassette einrekombiniert werden können. Zusätzlich bringt dieser Vektor eine loxP-Kassette mit, in die mit Hilfe von Cre z.B. variable Domänen einrekombiniert werden können. Klonierungsvektor war zunächst der mit AatII geschnittene Vektor pBS FRTvKappa (Figur 15A). In diesen wurden synthetische Oligonucleotidsequenzen gesetzt, die die loxP und die loxP511 Stelle mit einer zusätzlichen BamH1-Stelle codierten. Der resultierende Vektor wurde mit BamH1 geschnitten. Die cyan und unterstrichen dargestellten PCR-Primer hIgG1-1 und hIgG1-2 dienten zur Vervielfältigung eines chimären genomischen IgG1-Gens. Diese Primer brachten in einer nachgeschalteten PCR-Reaktion jeweils eine BamH1-Stelle mit. Template DNA war dabei der in Figur 12B beschriebene Vektor pBS MhIgG1M (alternativ pBS MhIgG1Mdelta350). Nach dem Einligieren dieser gleichfalls BamH1 verdauten PCR-Bande entstand der Vektor pBS loxP-IgG1, bzw. alternativ der Vektor pBS loxP-IgGldelta350. Restriktionsstellen sind unterstrichen. Die Grenze zwischen Maus und humanen Sequenzen wird durch eine HindIII-Restriktionsstelle markiert. Codierende humane Sequenzen sowie die Maus-M1- und Maus-M2-Exons sind mit grünen Kleinbuchstaben dargestellt. Der Splice-Akzeptor am 5'-Ende des Hinge-Exons ist rot unterstrichen dargestellt.

Ausgehend von dem Vektor pBS loxP-IgG1 wurde der Vektor pBS loxP-IgG1deltaCH1 erhalten. Beide Vektoren sind identisch, nur fehlen bei dem Vektor pBS loxP-IgG1deltaCH1 die DNA-Sequenzen zwischen der PfM1- und der BamH1-Stelle. Dazu wurde der Vektor pBS loxP-IgG1 mit BamH1 geschnitten und ein mit Hilfe der PCR-Primer deltaCH1-lund hIgG1-2 erhaltenes Fragment einkloniert. Als DNA-Template diente dabei der Vektor pBS loxP-IgG1.

### Figur 17

Bispezifische und bifunktionelle von Hybridomzellen präsentierte Antikörper.
(A) Bispezifischer Antikörper. Dargestellt ist der Vektor pBS FRT KappaHEAscFv215, durch den mit Hilfe von Flp ein chimäres Gen in den aktiven vKappa-Genlocus der Hybridomzelllinie HEA125-mhloxPmycFRTG418 einrekombiniert werden kann (Beispiel 12). Unter der Kontrolle des Kappa-Promotors (mit endogenem Leader-Exon) und des im anschliessenden Intron (vor dem endogenen cKappa-Exon) liegenden Kappa-Enhancers codiert dieses chimäre Gen:
   ■ die vKappa-Domäne von HEA125 (grüne Kleinbuchstaben);
   ■ fusioniert an die cKappa-Maus-Domäne von HEA125 (blaue Grossbuchstaben);
   ■ fusioniert an eine Linker-Sequenz (kursiv Magenta);
   ■ fusioniert an die vH-Domäne des scFv-Antikörpers 215 (cyane Kleinbuchstaben);
   ■ fusioniert an einen GlySer-Linker (kursiv Magenta);
   ■ fusioniert an die vKappa-Domäne des scFv-Antikörpers 215 (grüne Kleinbuschstaben); und
   ■ schliesslich fusioniert an ein myc-tag und ein His-tag (jeweils kursiv Magenta).
   Restriktionsstellen sind unterstrichen. Die in Klammern gesetzte Restriktionsstelle PvuII / MscI enthält auf Grund der Klonierung entstandene residuale Sequenzen. Die rot und kleingeschrieben dargestellten PCR-Primer vHEAcDNA1 und vHEAcDNA2 dienten zur Vervielfältigung der cDNA des aktiven Kappa-Gens von HEA125. Template DNA war dabei cDNA von HEA125. Der Primer vHEAcDNA2 brachte in einer nachgeschalteten PCR zusätzlich die Sequenzen des dargestellten flankierenden Linkers bis einschliesslich der PvuII-Stelle mit. Dieses PCR-Fragment wurde in den MscI-geschnittenen Klonierungsvektor pBS FRTvKappa ligiert (siehe Figur 15A). Anschliessend wurde der resultierende Vektor pBS FRT KappaHEAPvuII mit PvuII geschnitten. Die rot und kleingeschrieben dargestellten PCR-Primer scFv1 und scFv2 dienten zur Vervielfältigung der codierenden DNA des scFv-Antikörpers. Template DNA war dabei das Plasmid pOPE101-215. Nach dem Einklonieren dieses PCR-Fragments entstand der Vektor pBS FRT KappaHEAscFv215.
B. Bifunktioneller Antikörper. Dargestellt ist der Vektor pBS FRT KappaHEAbla, durch den mit Hilfe von Flp ein bifunktionelles chimäres Gen in den aktiven vKappa-Genlocus der Hybridomzelllinie HEA125-mhloxPmycFRTG418 einrekombiniert werden kann (Beispiel 13). Unter der Kontrolle des Kappa-Promotors (mit endogenem Leader-Exon) und des im anschliessenden Intron (vor dem endogenen cKappa-Exon) liegenden Kappa-Enhancers codiert dieses chimäre Gen:
   ■ die vKappa-Domäne von HEA125 (grüne Kleinbuchstaben);
   ■ fusioniert an die cKappa-Maus-Domäne von HEA125 (blaue Grossbuchstaben);
   ■ fusioniert an eine Linker-Sequenz (kursiv Magenta)und
   ■ fusioniert an die codierende Sequenz der beta-Lactamase (grüne Kleinbuschstaben).

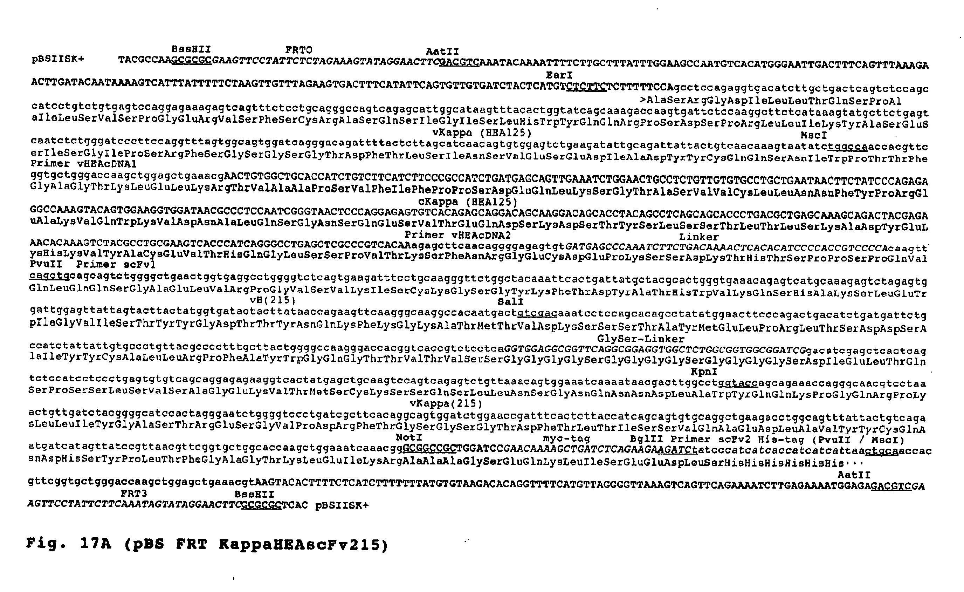

Restriktionsstellen sind unterstrichen. Die in Klammern gesetzten Restriktionsstellen PvuII bzw. PvuII / MscI enthalten auf Grund der Klonierung entstandene residuale Sequenzen. Ausgangspunkt war der PvuII-geschnittene in Figur 17A beschriebene Vektor pBS FRT KappaHEAPvuII. Die rot und kleingeschrieben dargestellten PCR-Primer bla1 und bla2 dienten zur Vervielfältigung der codierenden DNA der beta-Lactamase. Template DNA war dabei das Plasmid pBSIISK+. Nach dem Einklonieren dieses PCR-Fragments entstand der Vektor pBS FRT KappaHEAbla.

### Figur 18

Weitere Beispiele für veränderte von Hybridomzellen präsentierte Antikörper.
A. Veränderte Antikörperspezifität mittels spezifischer Rekombination. Dargestellt ist der Vektor pBS FRT vKappa215, durch den mit Hilfe von Flp ein verändertes vKappa-Exon in den aktiven vKappa-Genlocus der Hybridomzelllinie HEA125-mhloxPmycFRTG418 einrekombiniert werden kann (Beispiel 14). Unter der Kontrolle des Kappa-Promotors (mit endogenem Leader-Exon) und des im anschliessenden Intron (vor dem endogenen cKappa-Exon) liegenden Kappa-Enhancers codiert dieses Exon die vKappa-Domäne des 215er-Antikörpers (Kontermann et al., 1995, Characterization of the epitope recognised by a monoclonal antibody directed against the largest subunit of Drosophila RNA polymerase II. Biol. Chem. Hoppe-Seyler 376, 473-481; grüne Kleinbuchstaben). Diese vKappa-Domäne wird mit der endogen von HEA125 codierten cKappa-Domäne zusammengespleisst. Restriktionsstellen sind unterstrichen. Die rot und kleingeschrieben dargestellten PCR-Primer K215-1 und K215-2 dienten zur Vervielfältigung der vKappa(215)-Domäne. Der PCR-Primer K215-1 bringt dabei eine stille Punktmutation mit, die zur Einführung einer EcoRV-Stelle dient. Template DNA war dabei das Plasmid pOPE101-215 (siehe Figur 17A). Dieses PCR-Fragment wurde in den EarI und AvaII geschnittenen Vektor pBS FRTvKappa (siehe Figur 15A) ligiert. Dadurch entstand der Vektor pBS FRT vKappa215.
B. Fab-Antikörper mittels spezifischer Rekombination. Dargestellt ist der Vektor pBS loxP-FdHEA, durch den mit Hilfe von Cre ein verändertes vH-Exon in den aktiven vH-Genlocus der Hybridomzelllinie HEA125-mhRek einrekombiniert werden kann (Beispiel 15). Unter der Kontrolle des vH-Promotors und des im anschliessenden Intron (vor dem endogenen CH1-Exon) liegenden IgG1-Enhancers codiert dieses Exon die vH-Domäne des HEA125-Antikörpers (grüne Kleinbuchstaben). Diese vH-Domäne wird mit der anschliessenden IgG1-CH1-Domäne (grüne Kleinbuchstaben) zusammengespleisst. Restriktionsstellen sind unterstrichen. Die rot und kleingeschrieben dargestellten PCR-Primer Fd1 und Fd2 dienten zur Vervielfältigung der IgG1-CH1-Domäne. Der PCR-Primer Fd2 bringt dabei ein Stopcodon und eine SalI-Stelle mit. Template DNA war dabei genomische DNA von HEA125. Dieses PCR-Fragment wurde in den SalI geschnittenen Vektor pBS loxPvH (siehe Figur 15B) ligiert. Dadurch entstand der Vektor pBS loxP-FdHEA.

### Abkürzungen

- Ag8: Myelomzelllinie X63AG8.653
Antikörper-Bank eine Vielzahl (>100) Antikörper-produzierender Zellen, bzw. der
- Antikörper-: entsprechenden Antikörper-Gene und -Proteine
- Bibliothek: siehe Antikörper-Bank
- bla: beta-Lactamase, Gen dafür
- Bp: Basenpaare
- Antigen: spezifisch einen Antikörper bindender Ligand; im Sinne dieser Erfindung auch
ein Ligand, der spezifisch an einen Rezeptor bindet
- Antikörper: spezifisch ein Antigen bindendes Protein; im Sinne dieser Erfindung auch allgemeiner als Protein zu verstehen, das spezifisch einen Liganden bindet
- cH: konstante Domänen der schweren Antikörperkette, Gen dafür
- CH1: erste konstante Domäne eines Immunglobulins (IgG, IgA, IgE, IgD, IgM), Exon dafür
- CH2: zweite konstante Domäne eines Immunglobulins (IgG, IgA, IgE, IgD, IgM), Exon dafür
- CH3: dritte konstante Domäne eines Immunglobulins (IgG, IgA, IgE, IgD, IgM), Exon dafür
- CH4: vierte konstante Domäne eines Immunglobulins (IgE, IgM), Exon dafür
- cL: konstante Domäne der leichten Antikörperkette, Gen dafür
- D: D-Segment der vH-Domäne
- DMSO: Dimethylsulfoxid
- downstream: ausgehend vom 3'-Ende einer DNA-Sequenz in 3'-Richtung
- e: Enhancer
- Fab: Antikörperteil bestehend aus leichter Kette, vH- und CH1-Domäne, Gen dafür
- FACS: Fluorescence Activated Cell Sorting
- FRT: Flp Recognition Targets
- FRT0: GAAGTTCCTATTCTCTAGAAAGTATAGGAACTTC
- FRT3: GAAGTTCCTATTCTTCAAATAGTATAGGAACTTC
- G418: siehe Neo
- H: Hinge, Hinge-Exon
- His-tag: eine Abfolge von 5 oder 6 Histidinen geeignet zur Affinitätsreinigung per NiChelat; DNA-Sequenzen dafür
- J_{H}: Joining-Segment der vH-Domäne, Gensegment dafür
- J_{Lambda}: Joining-Segment der vLambda-Domäne, Gensegment dafür
- J_{Kappa}: Joining-Segment der vKappa-Domäne, Gensegment dafür
- L: Leader, Signalpeptid, Exon dafür; die Leader-Exons der Antikörpergene codieren nur den N-terminalen Teil des Leaderpeptids
- loxP: DNA-Sequenzen, die von der Rekombinase Cre erkannt werden
- lox66-Stelle: TACCGTTCGTATAATGTATGCTATACGAAGTTAT
- lox71-Stelle: ATAACTTCGTATAATGTATGCTATACGAACGGTA
- loxP-Stelle: ATAACTTCGTATAATGTATGCTATACGAAGTTAT
- loxP511-Stelle: ATAACTTCGTATAATGTATACTATACGAAGTTAT
- loxG-Stelle: ATAACTTCGTATAGCATACATTATACGAAGTTGC
- M: Membran-Domäne eines Immunglobulins (IgA1, IgA2), Exon dafür
- M1: erste Membran-Domäne eines Immunglobulins (IgG, IgE, IgD, IgM), Exon dafür
- M2: zweite Membran-Domäne eines Immunglobulins (IgG, IgE, IgD, IgM), Exon dafür
- mIgG1: Membran-ständiges IgG1; mRNA dafür
- myc-tag: Peptidsequenz, die von dem monoklonalen Antikörper 1-9E10 erkannt
- wird;: DNA-Sequenzen dafür
- Myelom: Fusionspartner zur Herstellung eines Hybridoms; Abkömmling einer Plasmacytom-Zelllinie
- Neo: Das Neophosphoryltransferase II Gen vermittelt eine Resistenz gegen Neomycin bzw. gegen G418, Gen dafür
- P: Promotor
- pBS185: Cre-Expressionsvektor von Life Technologies (Gibco-BRL) #10347-011
- PEG: Polyethylenglykoll
- pGH-1: Neo-Gen und HSV-tk Gen flankiert von einer loxP-Stelle; Gu und Rajewsky, 1993, Cell 73, 1155-1164
- PGK: Phosphoglycerin Kinase
- PGKneo: Neo (G418)-Resistenzgen unter der Kontrolle des PGK-Promotors
- pMC-Cre: Cre-Expressionsvektor; Gu und Rajewsky, 1993, Cell 73, 1155-1164; siehe auch Transgenic Animal Web: www.med.umich.edu/tamc/mta.html
- pIC-Cre: Cre-Expressionsvektor; Gu und Rajewsky, 1993, Cell 73, 1155-1164
- pOG44: Flp-Expressionsvektor von Invitrogen; Bestellnummer V6005-20 oder aus dem Kit Flp-In™ pcDNA5/FRT Core Bestellnummer K6010-02
- polX mu: EMBL Datenbank accession number AJ251804 (Maus), AJ131891 (Homo Sapiens); komplette mRNA
- pOPE101-215: EMBL Datenbank accession number ASY14585; synthetisches Gen eines scFv(215)-Antikörpers; siehe auch Kontermann et al., 1995, Biol. Chem. Hoppe-Seyler 376, 473-481
- pREP4: Invitrogen V004-50; in Mammalliazellen episomal replizierendes Plasmid, durch Hygromycin selektionierbar
- pSH47: EMBL Datenbank accession numberAF298782; Cre Expressionsvektor für Hefe
- pSVlacZT: Cre-Rekombinationssubstrat-Vektor zum Nachweis von Cre-Aktivität; Torres und Kühn Laboratory Protocols for Conditional Gene Targeting, 1997, Oxford University Press, ISBN 0-19-963677-X
- Protein G: Antikörper-Bindeprotein zum Nachweis der konstanten Antikörperdomänen
- RAD 54: EMBL Datenbank accession number BC001965; komplette mRNA
- RAD51B: EMBL Datenbank accession number U84138; komplette mRNA
- RecQ4: EMBL Datenbank accession number AB006532; komplette mRNA
- scFv, scFvn: single chain Fv-Antikörper
- sIgG1: sekretiertes IgG1; mRNA dafür
- Stop: Stop-Codon
- upstream: ausgehend vom 5'-Ende einer DNA-Sequenz in 5'-Richtung
- vH, vH1, vHn: variable Domäne der schweren Antikörperkette
- vL, vL1, vLn: variable Domäne der leichten Antikörperkette
- vLambda: variable Domäne der leichten Lambda-Antikörperkette
- vKappa: variable Domäne der leichten Kappa-Antikörperkette
- XRCC2: EMBL Datenbank accession number AF035587; komplette mRNA
- XRCC3: EMBL Datenbank accession number AF035586; komplette mRNA

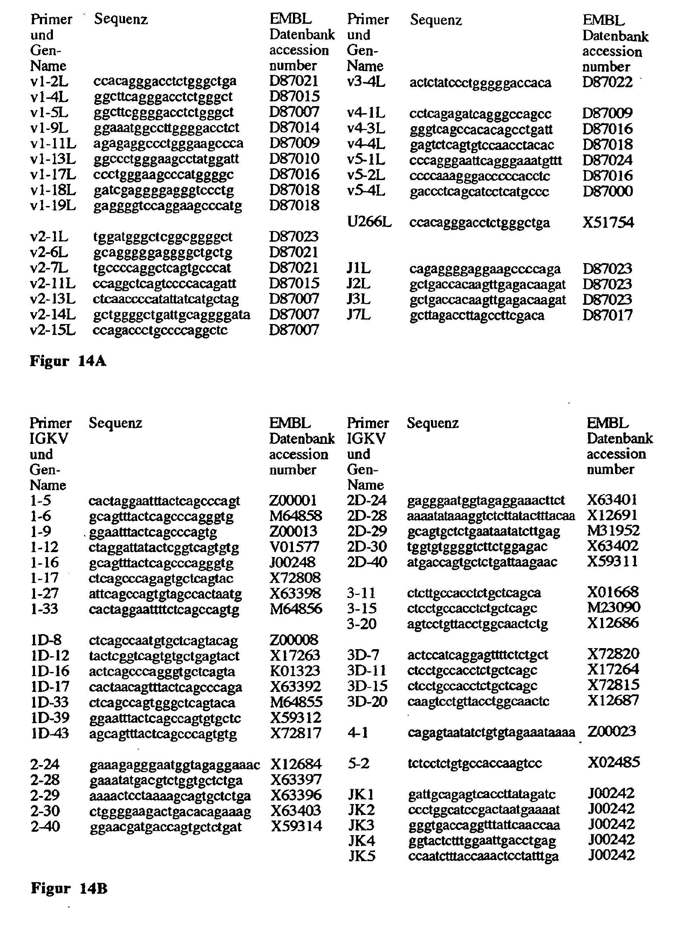

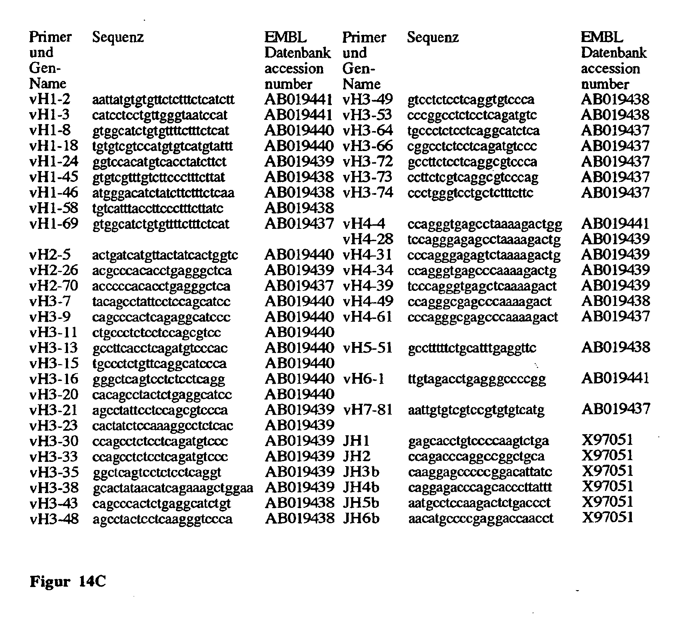

Das erfindungsgemäße Verfahren ist **dadurch gekennzeichnet**
■ **dass** in einen oder zwei aktive Genloci, insbesondere in die exprimierten vH- und vL-Gene einer B-Zelllinie spezifische Rekombinationssignale eingeführt werden (d.h., es werden Akzeptoren für DNA-Sequenzen generiert; Figur 3, 4, 6), insbesondere durch homologe Rekombination (Figur 3, 4);
■ dass die resultierende Zelllinie expandiert wird;
■ dass mit vergleichsweise wenigen Gensegment-spezifischen PCR-Primern, insbesondere mit vH, vKappa und vLambdaspezifischen Primern und mit J_{H}, Jₖₐₚₚₐ und J_{Lambda}-Segment spezifischen Gegenprimern eine Vielzahl von unterschiedlichen Genfragmenten vervielfältigt wird (Figur 14);
■ dass die Vielzahl der vervielfältigten Genfragmente jeweils mit spezifischen Rekombinationssignalen flankiert werden (d.h., es werden Donor-DNA-Sequenzen generiert; Figur 5, 6);
■ dass die genannte Vielzahl der vervielfältigten Genfragmente in die genannte Zelllinie transfiziert wird und dort unter Einfluss einer spezifischen Rekombinase, insbesondere von Cre oder Flp eine Vielzahl von spezifischen Rekombinationsereignissen (Figur 5, 6) innerhalb der genannten aktiven Genloci stattfinden und
■ dass auf Grund der spezifischen Rekombinationsereignisse viele unterschiedliche Zellen entstehen, die jeweils unterschiedliche, gegenüber der Ausgangszelle veränderte Antikörper auf der Oberfläche der jeweiligen Zelloberfläche präsentieren (Figur 9).

Die vorliegende Erfindung betrifft somit ein Verfahren, mit dem bereits existierende Maus Hybridomzellen vorteilhaft und einfach verändert humanisiert werden können (Figur 3, 7). Kennzeichen dieses Verfahrens ist,
■ dass die genannten Hybridomzellen durch eine homologe Rekombination innerhalb des aktiven Antikörperlocus verändert werden, ohne dass dabei ein störender Resistenzmarker eingesetzt wird;
■ dass die genannte homologe Rekombination zu einem humanisierten Antikörper führt;
■ dass der humanisierte Antikörper auf der Oberfläche der genannten durch homologe Rekombination veränderten Hybridomzelle präsentiert wird und
■ dass diese Oberflächenpräsentation zur Selektion der veränderten Zelle ausgenützt wird.

Zur Durchführung dieser Verfahrens und der nachstehend beschriebenen spezifischen Ausführungsformen kann der Fachmann gemäß allgemein bekannter Verfahren bzw. gemäß den nachstehend und in den Beispielen beschriebenen Methoden vorgehen.

### Einführung von Erkennungsstellen für Rekombinasen durch homologe Rekombination

Ein Kernelement der vorliegenden Erfindung ist die Einführung veränderter DNA-Sequenzen, insbesondere von Erkennungsstellen für Rekombinasen in das Genom von Zelllinien mittels homologer Rekombination (Figur 3, 4, 8). Dabei werden in einem definierten Genlocus DNA-Sequenzen ausgetauscht. Dies wird dadurch erreicht, dass die neu einzuführenden DNA-Sequenzen von i.d.R. >700 Bp langen zu dem definieren Genlocus homologen Bereichen flankiert werden und in dieser Form (meist als linearisierte DNA; siehe Hasty et al., 1992, Mol. ell. Biol. 12, 2464-2474) in die Zellen transfiziert werden. In einigen wenigen Fällen (ca. 1 aus 10⁷ Zellen) findet daraufhin die gewünschte homologe Rekombination statt. Diese seltenen Ereignisse bzw. homolog rekombinierten Zellen müssen anschliessend z.B. über einen Resistenzmarker, oder über einen FACS-Sorter (s.u.).oder ELISA-Test isoliert werden. Die dafür benötigten Verfahren (u.a. Klonierungsverfahren, PCR, Sequenzierung, Zellkultur von Hybridomzellen, homologe Rekombination, Selektion mittels G418, FACS) sind dem Fachmann bekannt und in einer Vielzahl von Laborhandbüchern beschrieben (u.a. Sambrook and Russell: Molecular Cloning, a laboratory manual, 3rd Edition, 2001, ISBN 0-87969-577-3). Eine Kombination dieser oder ähnlicher Verfahren mit dem Kernelement der homologen Rekombination von veränderten DNA-Sequenzen in das Genom einer Zelllinie wird zur Zeit v.a. für die Generierung von transgenen Mäusen, bzw. von den dafür benötigten veränderten ES-Zelllinien verwendet (Thomas und Capecchi, 1987, Cell 51, 503-512; Thompson et al., 1989, Cell 56, 313-321; Johnson et al., 1989, Science 245, 1234-1236; Doetschman et al., 1987, Nature 330, 576-578; "Transgene Tiere" von J. Schenkel, 1995, Spektrum-Verlag ISBN 3860252690; Vasquez et al., 2001, Manipulating the mammalian genome by homologous recombination. PNAS 98, 8403-8410; Torres und Kühn Laboratory Protocols for Conditional Gene Targeting, 1997, Oxford University Press, ISBN 0-19-963677-X). Spezifische Erkennungsstellen für Rekombinasen werden dabei u.a. dafür eingesetzt, um durch Gewebs-spezifisch induzierte Rekombinasen einzelne Exons definierter Gene in definierten Geweben zu deletieren. Für die vorliegende Erfindung werden diese Techniken auf geeignete Hybridomzelllinien adaptiert. Auch innerhalb von Hybridomzelllinien wurden bereits homologe Rekombinationen durchgeführt (Zou et al., 1994, Current Biuology 4, 1099-1103; Shulman et al., 1990, Mol. and Cell. Biology 10, 4466-4472; Sun et al., 1994, J. of Immunology, 152, 695-704; Baker et al., 1994, J. Immunological Methods 168, 25-32; Wood et al., 1991, PNAS 88, 8006-8010; Fell et al., 1989, PNAS 86, 8507-8511).

### Spezifische Rekombination

Der Fachmann kennt auch geeignete Erkennungsstellen und die dazu gehörigen Rekombinasen (u.a. Stricklett et al., 1999, The Cre/loxP system and gene targeting in the kidney. Am J Physiol. 276, F651-F657. Review.; Stricklett et al., 1998, Site-specific recombination using an epitope tagged bacteriophage P1 Cre recombinase. Gene. 215, 415-23; Van Duyne, 2001, A structural view of cre-loxp site-specific recombination. Annu Rev Biophys Biomol Struct. 30, 87-104. Review.; Theodosiou und Xu, 1998, Use of FLP/FRT system to study Drosophila development. Methods. 4, 355-65. Review.; Sadowski, 1995, The Flp recombinase of the 2-microns plasmid of Saccharomyces cerevisiae. Prog Nucleic Acid Res Mol Biol. 51, 53-91. Review.). Beispiele für geeignete Systeme sind:
■ das Cre-lox-System (Rekombinase Cre, spezifische Erkennungsstellen loxP; siehe u.a. Creator™ Kit von Clontech; US-Patent Nr. 4959317; Griffiths et al., 1994, EMBO Journal 13, 3245-3260);
■ das Flp-System (Rekombinase Flp, spezifische Erkennungsstellen FRT; O'Gorman et al. 1991, Science 251, 1351-1355; Flp-In™ pcDNA5/FRT Complete Kit von Invitrogen #K60101-01) und
■ das Gateway-System (Lambda-Integrase Int und Integration Host Factor IHF, spezifische Erkennungsstellen attB x attP und attL x attR; siehe u.a Gateway™ von GibcoBRL / Invitrogen),
wobei das Cre-lox-System und das Flp-System bevorzugt sind.

Der Einsatz mehrerer Systeme innerhalb einer Zelle ist dann vorzuziehen, wenn in mehr als einen Genlocus spezifische Rekombinationssignale eingeführt werden sollen, d.h., wenn unterschiedliche Akzeptoren für DNA-Sequenzen generiert werden sollen. Ein Beispiel sind die vH- und vKappa-Genloci einer Hybridomzelllinie, die jeweils mit spezifischen Erkennungsstellen für Rekombinasen flankiert werden sollen (Figur 5). Würden diese alle von derselben Rekombinase erkannt, käme es zu Interferenzen zwischen den verschiedenen Genloci, bzw. den verwendeten Austauschvektoren. Dies gilt nicht, wenn 4 verschiedene, nicht (oder wenig) miteinander rekombinierende Erkennungsstellen zur Verfügung stehen, wie z.B. die loxP-Stellen loxP, loxP511, loxG, lox66 und lox71. Dann kann eine Art von Rekombinase für zwei Genloci verwendet werden.

Die loxP-Stellen (FRT-Stellen sind sehr ähnlich aufgebaut) bestehen aus zwei 13Bp-langen inverted repeats, die von einem 8Bp-langen Spacer getrennt sind. Dieser Spacer gibt die Direktionalität der von loxP-Stellen flankierten einrekombinierten DNA vor, d.h., der Experimentator kann mit einer (genomischen) loxP- oder FRT-Stelle den genauen chromosomalen Genlocus und zugleich die Orientierung der einrekombinierten DNA vorausbestimmen. Höhere Rekombinationsfrequenzen als bei der homolgen Rekombination werden i.d.R. durch den Austausch von Gen-Kassetten erreicht. Dabei werden die auszutauschenden DNA-Sequenzen sowohl im Genom wie auch in den Austauschvektoren von zwei leicht unterschiedlichen loxP- oder FRT-Stellen flankiert, die auf Grund der Sequenzunterschiede zwar mit einem entsprechenden Partner, aber nicht miteinander rekombinieren (Figur 5). Diese Technik ist in dem US-Patent Nr. 5928914 und in der Publikation von Feng et al. (J. Mol. Biol. 1999, 292, 779-785) für das Cre-lox-System und in den Publikationen von Seibler und Bode (Biochemistry 1997, 36, 1740-1747; Biochemistry 1994, 33, 12746-12751) für das Flp-System beschrieben worden. Kassetten-Austausch findet auch bei Genen zwischen zwei invertierten Erkennungsstellen gleicher Identität statt (z.B. loxP Gen Pxol). Das Produkt einer spezifischen Rekombination kann in diesem Fall drei Formen haben: 1. Das Gen zwischen den loxP-Stellen kann invertiert werden. Ausserdem kann des Gen zwischen den loxP-Stellen gegen ein anderes reinrekombiniertes ersetzt werden, das 2. in richtiger Orientierung und 3. in falscher Orientierung zwischen den Erkennungsstellen integriert wird. Selbst wenn vor dem Austausch zwischen den Erkennungsstellen kein negativ selektionierbarer Marker zwischen den Erkennungsstellen vorhanden ist, findet in ca. 1% aller Zellen, die die Transfektion überlebt haben, eine spezifische Rekombination statt (Feng et al., 1999, J. Mol. Biol. 292, 779-785).

Gleichfalls bekannt sind Verfahren und Expressionsvektoren mit deren Hilfe die genannten Rekombinasen transient in eukaryontischen Zellen (Taniguchi et al., 1998, Efficient production of Cre-mediated site-directed recombinants through the utilization of the puromycin resistance gene, pac: a transient gene-integration marker for ES cells. Nucleic Acids Res 26, 679-680; Araki et al., 1997, Efficiency of recombination by Cre transient expression in embryonic stem cells: comparison of various promoters. J Biochem (Tokyo). 122, 977-82; Ludwig et al., 1996, FLP-mediated site-specific recombination in microinjected murine zygotes. Transgenic Res. 5, 385-395; Flp-Expressionsvektor pOG44 von Invirtogen, #V6005-20), stabil integriert oder episomal in Zelllininien (Seibler und Bode, 1997, Biochemistry 36, 1740-1747) in transgenen Tieren (Nelson et al., 1998, Expression of an AQP2 Cre recombinase transgene in kidney and male reproductive system of transgenic mice. Am J Physiol. 275, C216-226) oder gar Gewebs-spezifisch exprimiert werden können ("Transgene Tiere" von J. Schenkel, 1995, Spektrum-Verlag ISBN 3860252690). Auch die Rekombinasen selbst sind als gereinigte Proteine erhältlich (z.B. Creator™ Kit von Clontech) und können damit gegebenenfalls auch in dieser Form ins Zellinnere transfiziert werden.

### DNA-Sequenzen und geeignete Genloci

Ebenfalls bekannt sind die DNA-Sequenzen geeigneter Gene, Genfragmente (insbesondere vH, vLambda, vKappa und die dazugehörigen J-Segmente) und Genloci des Menschen (http://genome.ucsc.edu/goldenPath/hgTracks.html; www.gdb.org; www.gdb.org/hugo; www.ncbi.nlm.nih.gov; www.ncbi.nlm.nih.gov/LocusLink) oder der Maus (www.informatics.jax.org), insbesondere die aktiven Antikörper-Genloci und die Vielfalt der Antikörpergene (Immunoglobulin Facts Book, Lefranc und Lefranc, 2001, Academic Press, ISBN 0-12-441351-X; http://imgt.cines.fr; Kabat Datenbank: http://immuno.bme.nwe.edu) und die Genloci des T-Zellrezeptors (T-Cell Receptor Facts Book, Lefranc und Lefranc, 2001, Academic Press, ISBN 0-12-441352-8; http://imgt.cines.fr). Besonders bevorzugt im Sinne dieser Erfindung sind die aktiven Antikörper-Genloci, insbesondere der Maus-Hybridomzelllinie HEA125, die genomisch rekombinierte Antikörpergene exprimieren.

Es können jedoch auch zunächst unbekannte Genloci verwendet werden, z.B. durch die Selektion von Zellen mit einer möglichst hohen Expressionrate einer Antibiotikaresistenz (Figur 6). Vanin et al. (1997, Development of high-titer retroviral producer cell lines by using Cre-mediated recombination. J Virol 71, 7820-7826) beschreiben ein dafür geeignetes Verfahren, das dem in Figur 6 dargestellten ersten Verfahrensschritt sehr ähnlich ist (Integration statt Kassettenaustausch). Dabei ist das auf hohe Expressionsrate selektionierte Resistenzgen von spezifischen Rekombinationssignalen flankiert. Ein ähnliches System wird von Invitrogen verkauft (Flp-In™ pcDNA5/FRT Complete Kit #K6010-01; US-Patente 5654182 und 5677177). Dies ermöglicht den vergleichsweise einfachen Austausch des vorselektionierten Resistenzgens beispielsweise gegen eine Schar unterschiedlicher Antikörpergene. Vorzugsweise werden davor als Zwischenschritt zusätzliche andere Rekombinationssignale eingeführt, die einen variablen, d.h. austauschbaren Genanteil flankieren, insbesondere indem diese zusammen mit einem konstanten Genanteil in den vorselektionierten Genlocus einrekombiniert werden (Figur 6). In diesem Fall wird also:
1. Durch die Expression eines Resistenzgens die Integration spezifischer Rekombinationssignale in einen ersten aktiven Genlocus selektioniert (bzw. die entsprechende resistente Zelllinie),
2. Auf Grund der spezifischen Rekombinationssignale ein konstanter Proteinanteil einrekombiniert, insbesondere die differenziell gespleissten (optional mit CH1) CH2, CH3, M1 und M2-Domänen eines humanen IgG1-Gens,
3. Gleichzeitig damit werden die Gene für variable Proteinanteile einrekombiniert, insbesondere ein vH-Gen oder ein scFV-Gen eines Antikörpers, das zusätzlich von 2 weiteren spezifischen Rekombinationssignalen flankiert ist,
4. Das gewünschte Rekombinationsereignis auf Grund der Oberflächenpräsentation des exprimierten Antikörpers selektioniert,
5. Mit den Genen der leichten Antikörperkette wird anschliessend ggfs. analog verfahren und
6. Durch spezifische Rekombination die Vielfalt unterschiedlicher Antikörper in die genannten Genloci einrekombiniert.

### Herstellung komplexer DNA-Sequenzen

Ein weiteres Kernelement der vorliegenden Erfindung ist die Herstellung einer grossen Zahl unterschiedlicher DNA-Sequenzen, insbesondere von möglichst vielen unterschiedlichen Antikörpergenen. Jeweils flankiert von den erwähnten Erkennungsstellen für Rekombinasen dienen diese DNA-Sequenzen als Donoren für viele unterschiedliche DNA-Sequenzen, die unter dem Einfluss von Rekombinasen mit vergleichsweise hoher Effizienz in die erwähnten Genloci mit den erwähnten Akzeptoren für DNA-Sequenzen einrekombinieren. Auch die dafür benötigten Techniken, insbesondere PCR-Techniken (u.a. Sambrook and Russell: Molecular Cloning, a laboratory manual, 3rd Edition, 2001, ISBN 0-87969-577-3; insbesondere Kapitel 8 für PCR-Techniken) und DNA-Sequenzen (s.o.) sind dem Fachman bekannt und in einer Vielzahl von Publikationen v.a. zur Herstellung von Bibliotheken rekombinanter Antikörper beschrieben (u.a. "Rekombinante Antikörper", Kapitel 2.2 von Breitling und Dübel, 1997, Spektrum-Verlag ISBN 3-8274-0029-5). Insbesondere das Buch Immunoglobulin Facts Book (Lefranc und Lefranc, 2001, Academic Press, ISBN 0-12-441351-X) ist zusammen mit den dort angegebenen Acession Numbers für die Datenbanken eine ausgezeichnete Quelle der ca. 200 variablen (und konstanten) Antikörpergensequenzen des Menschen. Die Vervielfältigung der Vielzahl genomisch rekombinierter humaner Antikörpergene kann im Sinne der vorliegenden Erfindung z.B. vergleichsweise einfach durch die Kombination von wenigen (jeweils weniger als 50) vH, vKappa und vLambda-spezifischen PCR-Primern mit (jeweils weniger als 6) J_{H}, Jₖₐₚₚₐ und J_{Lambda-}Segment spezifischen Gegenprimern erfolgen (Figur 14), wobei die Primer vorzugsweise an evolutionär wenig konservierte genomische Bereiche hybridisieren. Ähnliches gilt für die Maus-Antikörpergene. Als Template der dafür benötigten weniger als 1.000 PCR-Reaktionen dient die genomische DNA einer Vielzahl von insbesondere aus menschlichem Blut gewonnenen B-Lymphocyten. Die genomische DNA dieser B-Lymphocyten enthält sehr viele unterschiedlich genomisch rekombinierte Antikörpergene (vH, vKappa, vLambda), die durch die beschriebene Verfahrensweise in ihrer Vielzahl sehr einfach vervielfältigt werden können. Ganz analog kann auch die Vielzahl genomisch rekombinierter T-Zellrezeptoren durch vergleichsweise wenige PCR-Reaktionen in eine Schar geeigneter DNA-Sequenzen überführt werden.

Um diese vielzähligen unterschiedlichen DNA-Sequenzen mit den erwähnten Erkennungsstellen für Rekombinasen zu flankieren, können diese entweder in einen vorgegebenen Klonierungsvektor kloniert werden, der diese Erkennungsstellen bereits enthält, oder aber eine nachgeschaltete zweite PCR bringt mit den verwendeten PCR-Primern diese Erkennungsstellen mit. Die Schar der unterschiedlichen PCR-Produkte wird anschliessend vorzugsweise in ein Plasmid kloniert (z.B. pUC19 von Biolabs, pBluescript von Stratagene, pCR-TOPO, pCR-XL-TOPO, pCR-Vector, pZErO-1, pCR-Blunt, pSinRep5 von Invitrogen). Optional kann dabei ein Replikationsursprung unter Selektionsdruck zu einer stabilen episomalen Replikation führen (z.B. die Hygromycinselektionierbaren episomal replizierenden Vektoren pCEP4, pREP7, pREP10, pEBVHis oder pREP4 von Invitrogen). Dadurch kann sehr einfach die Zeit verlängert werden, innerhalb derer es zu einer spezifischen Rekombination mit den erwähnten Genloci kommen kann. Hierzu ist natürlich zusätzlich die Anwesenheit der entsprechenden Rekombinasen, bzw. der entsprechenden Expressionsvektoren notwendig. In einer besonders bevorzugten Ausführungsform werden die Expressionskassetten für die genannten Rekombinasen in den gerade genannten Klonierungsvektor mit eingebaut.

Eine Schar unterschiedlicher Plasmide kann bei Bedarf auch in extrem hoher Komplexität (>10¹²) *in vitro* generiert werden, wobei vermieden wird diese vorher in Bakterien zu vervielfältigen (durch Elektroporation sind derzeit Komplexitäten von ca. 10⁹ unabhängigen Bakterienklonen pro *µ*g eingesetzter DNA routinemässig erreichbar; siehe z.B. Clontech #C2023-1 330, den *E.coli* Stamm KC8 >10⁹ cfu/µg pUC). Dazu werden die beschriebenen unterschiedlichen PCR-Produkte, die von den erwähnten Erkennungsstellen für Rekombinasen flankiert sind vorzugsweise zunächst mit Ligase zirkularisiert (Donoren für DNA-Sequenzen) und mit einem Klonierungsvektor vermischt, der dieselben Erkennungsstellen enthält (Akzeptoren für DNA-Sequenzen). Unter dem Einfluss der gereinigten Rekombinasen-Proteine entsteht dabei in vitro eine hochkomplexe Mischung von Klonierungsvektoren mit unterschiedlichen einrekombinierten DNA-Sequenzen. Auch dieses Verfahren ist dem Fachmann bekannt und bereits kommerziell erhältlich (siehe u.a. Creator™ Kit von Clontech; Gateway™ von GibcoBRL / Invitrogen).

Daneben existieren weitere dem Fachmann bekannte Verfahren, mit denen eine Vielzahl unterschiedlicher DNA-Sequenzen hergestellt werden können (z.B. In vitro DNA-Shuffeling, Stemmer, 1994, Nature 370, 389-391 oder die Vervielfältigung der Vielzahl von Antikörper cDNA oder ungerichtet kleingescherte genomische DNA). Erwähnt sei hier besonders die Herstellung sehr vieler unterschiedlicher Antikörpergene durch die kombinatorische Synthese von DNA-Sequenzen, insbesondere unterschiedlicher CDR-DNA-Sequenzen (Breitling et al., 1990, "synthetic human antibody libraries" Deutsches Patent Nr. P 40 02 897; siehe auch Firma Morphosys, HuCal-Antikörperbibliothek).

### Zelllinien

Die für das erfindungsgemäße Verfahren bevorzugte Maus-Hybridom Zelllinie HEA125 produziert einen Maus IgG1-Antikörper zusammen mit einer Maus-Kappa-Kette. Dieser monoklonale Antikörper erkennt das menschliche Tumorassoziierte Antigen Ep-CAM mit hoher Affinität und Spezifität (Moldenhauer et al., 1987, Br J Cancer 56, 714-722; Momburg et al., 1987, Cancer Research 47, 2883-2891). Eine davon abgeleitete Subpopulation präsentiert vergleichsweise viele Membran-gebundene Antikörper auf der Oberfläche. Andere Zelllinien, insbesondere andere Hybridomzellen oder lymphoide Zelllinien wie z.B. die humanen Linien:
■ U266, die eine Lambda-Kette und ein IgE produziert (Ikeyama et al., 1986, Purification and characterization of IgE produced by human myeloma cell line, U266. Mol Immunol 23, 159-167);
■ IM-9 (Lesniak und Roth, 1976, Regulation of receptor concentration by homologous hormone. Effect of human growth hormone on its receptor in IM-9 lymphocytes. J Biol Chem 251, 3720-3729); und
■ Die T-Zelllinie Jurkat (Gillis und Watson, 1980, Biochemical and biological characterization of lymphocyte regulatory molecules. V. Identification of an interleukin 2-producing human leukemia T cell line. J Exp Med 152, 1709-1719); oder
■ Die Hühnchen B-Zelllinie DT40 (Buerstedde und Takeda, 1991, Cell 67, 179-188)
sind gleichfalls für das erfindungsgemäße Verfahren geeignet. Humane Zelllinien haben dabei den zusätzlichen Vorteil, dass dadurch die etwas andere Glykosilierung von Mauszellen vermieden wird (Borrebaeck, 1999, Nat Biotechnol 17, 621). T-Zelllinien wie Jurkat sind dann vorzuziehen, wenn eine T-Zellrezeptorbibliothek hergestellt werden soll, bzw. wenn der aktive T-Zellrezeptor Genlocus verwendet werden soll. Die Hühnchen Zelllinie DT40 dagegen weist vergleichsweise sehr hohe Effizienzen der homologen Rekombination transfizierter DNA auf.

### Transfektion

Auch die Transfektion der eukaryontischen Zellen erfolgt mittels dem Fachmann bekannter Standardverfahren (Sambrook and Russell: Molecular Cloning, a laboratory manual, 3rd Edition, 2001, ISBN 0-87969-577-3; Kapitel 16) wie z.B. Elektroporation (Firma AGS / Hybaid bzw. BioRad, Handbuch der Elektroporatoren BTX bzw. BioRad GenePulser) oder der Transfektion z.B. mit LipofectAMINE™ 2000 Reagent (Invitrogen #1668-027), mit DMRIE-C Reagent (Invitrogen #10459-014), oder LipofectAMINE™ Reagent (Invitrogen #18324-012), FuGENE 6 Transfection Reagent (Roche #1815091), DOTAP Liposomal Transfection Reagent (Roche #1811177), oder DOSPER Liposomal Transfection Reagent (Roche #1811169). Der Anteil der erfolgreich elektroporierten Hybridome liegt dabei i.d.R. bei 30-40% der eingesetzten Zellen. Für Transfektionen mit dem Ziel einer homologen Rekombination der transfizierten DNA-Sequenzen, werden die genannten DNA-Sqeuenzen vorzugsweise linearisiert. Ist eine spezifische Rekombination der transfizierten DNA-Sequenzen gewünscht, so werden vorzugsweise circuläre DNA-Sequenzen eingesetzt, insbesondere sind dafür in Hybridomzellen episomal replizierende Vektoren geeignet (s.o.). Der Anteil einer erfolgreichen spezifischen Rekombination durch Kassettenaustausch innerhalb einer eukaryontischen Zelle liegt bei ca. 1% der erfolgreich elektroporierten Zellen (Feng et al., 1999, Site-specific chromosomal integration in mammalian cells: highly efficient CRE recombinase-mediated cassette exchange. J-Mol-Biol. 292, 779-785), so dass durch den Einsatz von 3x 10⁹ Zellen ca. 10⁷ unterschiedliche spezifische Rekombinationsereignisse erhalten werden.

### Oberflächenexpression / Anreicherungsverfahren

Ein weiteres Kernelement der vorliegenden Erfindung ist die Isolierung oder Anreicherung derjenigen Zellen (möglichst nach jedem Verfahrensschritt), bei denen das gewünschte Rekombinationsereignis stattgefunden hat (Figur 7, 8, 9). Die Erfindung ermöglicht dies durch den Nachweis veränderter Antikörper auf der Oberfläche der sie produzierenden lebenden Zellen. Auch dies erfolgt nach gängigen, dem Fachmann bekannten Verfahren wie dem Einsatz von Magnetobeads (Firma Dynal, Oslo, Norwegen; Technical Handbook: Cell Separation and Protein Purification von Dynal; Current Protocols in Immunology, John Wiley & Sons, New York, ISBN 0-471-52276-7 und Fa. MiltenyiBiotec MACS - System (www.miltenyibiotec.com)) oder eines FACS-Sorters (Shapiro, H.M. Practical Flow Cytometry, 3^{rd} edition 1995, Wiley-Liss., New York, ISBN 0-471-30376-3; Darzynkiewicz et al. Flow Cytometry, 2^{nd} edition 1994, Academic Press, ISBN 0-12-564142-7; Current Protocols in Immunology, John Wiley & Sons, New York, ISBN 0-471-52276-7) oder per ELISA und wiederholter Subklonierung. Die sehr seltenen (ca. 1 aus 10⁷ Zellen) homologen Rekombinationsereignisse können z.B. mit einem zum Turbosorter aufgerüsteten FACSVantage SE, oder einem FACSDiva (Becton-Dickinson), oder einem MoFlow (Cytomation) identifiziert werden, wobei bei der Einführung spezifischer Rekombinationssignale in definierte Genloci zusätzlich dazu eine Vorselektion auf Grund eines einrekombinierten Resistenzgens erfolgen kann (z.B. Geneticin (G418) von Sigma; Chauhan und Gottesman, 1992, Construction of a new universal vector for insertional mutagenesis by homologous recombination. Gene 120, 281). Diese Art von Vorselektion ist bei der vorliegenden Erfindung aber nicht essenziell und wird insbesondere dann vermieden, wenn das dafür chromosomal integrierte Resistenzgen die Oberflächenexpression des durch die homologe Rekombination veränderten Antikörpers behindern würde. Ein Beispiel für eine solche Behinderung der Oberflächenexpression ist die von Yarnold und Fell (1994, Cancer Research 54, 506-512) beschriebene Humanisierung von Hybridomzellen durch homologe Rekombination. Dabei integriert das Resistenzgen in das für das differenzielle Spleissen essenzielle Intron zwischen CH3 und M1.

Auch die häufigeren (ca. 1 aus 10² bis 10³ Zellen beim Kassettenaustausch) spezifischen Rekombinationsereignisse können mit Standardverfahren durch den Nachweis veränderter Antikörper auf der Oberfläche der Zellen mit einem FACS-Sorter oder Magnetobeads angereichert werden (Figur 9), wie in den nachstehenden Beispielen beschrieben.

Die veränderten Antikörper werden dazu vorzugsweise vor dem sortieren mit einem FACS mit Hilfe Fluoreszenz-markierter mono- oder polyklonaler Antikörper (oder Protein G o.ä.) angefärbt, die von vielen Firmen kommerziell erhältlich sind (z.B. Jackson ImmunoResearch, West Grove, PA, USA oder Dianova, Deutschland oder Southern Biotechnology Associates, Birmingham,AL, USA oder BIOZOL, Deutschland). Klare Signale werden dabei insbesondere durch eine Doppelfärbung erhalten, wobei zwei unterschiedliche Fluoreszenzen die durch die gegeneinander ausgetauschten DNA-Sequenzen codierten Proteine (bzw. deren Fehlen) nachweisen. Die dazu benötigten Verfahren sind dem Fachmann bekannt und in einer Vielzahl von ausführlichen Publikationen beschrieben (z.B. Scheffold und Kern, 2000, Recent developments in flow cytometry. J Clin Immunol. 20, 400-7. Review; Thiel A, Scheffold A, Radbruch, 1998, Immunomagnetic cell sorting--pushing the limits. Immunotechnology. 2, 89-96. Review; sowie die oben angegebenen Zytometrie-Handbücher). Beispielsweise kann der Austausch einer von der Maus stammenden gegen eine humane Kappa-Domäne (durch homologe oder spezifische Rekombination) durch die Färbung mit FITC-markiertem Ziege-anti-human-Kappa-Antikörper nachgewiesen und sortiert werden, wobei gleichzeitig mit PE-markiertem Ziege-anti-Maus-Kappa-Antikörper gegengefärbt wird. Es kann aber auch das Fehlen eines Signals nachgewiesen werden, wenn z.B. durch homologe Rekombination die vH-Domäne eines auf der Oberfläche eines Hybridoms präsentierten IgG Antikörpers gegen ein G418-Resistenzgen ausgetauscht wurde. Nach der Vorselektion mit G418 ergibt die Färbung mit FITC-markiertem Ziege-anti-IgG-Antikörper eine weitere Anreicherung homologer Rekombinationsereignisse mit dem FACS: Die Zellen mit grüner Fluoreszenz haben in diesem Fall ihre G418-Resistenz nicht durch eine homologe Rekombination erworben und werden deswegen im FACS abgereichert. Hilfreich ist auch der Einsatz Epitop-spezifischer monoklonaler Antikörper, wie z.B. ein Mycl-9E10-Epitop-spezifische Antikörper (Evan et al., 1985, Mol. Cell. Biol. 5, 3610-3616), mit dem z.B. veränderte vH-Domänen nachgewiesen werden können. Die ausgetauschten DNA-Sequenzen können auch direkt, ohne weitere Färbung im FACS nachgewiesen werden, falls dadurch z.B. das Gen für ein EGFP (enhanced Green fluorescent protein; Clontech) einrekombiniert und exprimiert wird.

Die Oberflächenpräsentation von Antikörpern wird in der vorliegenden Erfindung vorzugsweise durch das differenzielle Spleissen der mRNA für die schwere Antikörperkette (bzw. entsprechender chimärer mRNAs) erreicht (Figur 2; Immunologie von Janeway und Travers, Kapitel 3-24, 4. Auflage 1999, Spektrum-Verlag, ISBN 443062757). Dies bietet den Vorteil, dass neben einer kovalent an die Membran gebundenen Variante auch grosse Mengen der in das Kulturmedium sekretierten Form zur schnellen und einfachen Charakterisierung eines Zellklons oder einer Zell-Bibliothek zur Verfügung steht. Der Anteil Membran-gebundener Antikörper und damit die erwartete Signalstärke kann dadurch erhöht werden, dass das Intron zwischen der CH3-Domäne und der M1-Domäne eines IgG oder IgA (bzw. zwischen der CH4-Domäne und der M1-Domäne eines IgM), insbesondere um 300Bp bis 1.000Bp verkürzt wird (Peterson und Perry, 1986, PNAS 83, 8883-8887; Tsurushita und Korn, 1987, Molec. Cell. Biol. 7, 2602-2605; Galli et al., 1987, Genes Dev 1, 471-481; Seipelt und Peterson, 1995, Molecular Immunology 32, 277-285; Figur 3, III.). Alternativ können dazu aber auch andere Membrananker, wie z.B. des alpha-T-Zellrezeptors (EMBL accession number X02883, die Sequenzen des Exon 3) oder eines MHC-Moleküls verwendet werden, wodurch noch mehr Antikörper auf der Oberfläche präsentiert werden können. Auch eine nicht kovalente Kopplung von Antikörpern auf der Zelloberfläche durch Membran-ständiges Protein G ist möglich (Breitling et al., 1999, PCT DE00/00079), wobei dies den Nachteil des cross-talks beinhaltet: Dabei werden nicht alle Antikörper zwingend von der sie präsentierenden Zelle codiert.

Die Oberflächenpräsentation ist insbesondere dann von grossem Nutzen, wenn viele (>10²) durch die exprimierten Antikörper voneinander verschiedene Zellen nach einer bestimmten Proteinaktivität durchsucht werden sollen. Die vorliegende Erfindung erreicht dies ganz analog zur Technik der rekombinanten Antikörper, wo dies insbesondere durch die Präsentation der Antikörper auf einem Phagen (Breitling et al., 1991, Gene 104, 147-153) oder auf einem Bakterium (Fuchs et al., 1991, Bio/Technology 9, 1369-1372) erreicht wird. Wie dort beschrieben können dadurch wesentlich komplexere Protein-Bibliotheken durchsucht werden. Die vorliegende Erfindung hat dabei den zusätzlichen Vorteil einer vergleichsweise sehr hohen Signalintensität durch die grosse Zahl der präsentierten jeweils gleichartigen Antikörper. Ein FACS-Sorter erreicht heutzutage Sortiergeschwindigkeiten von ca. 10⁸ Zellen pro Stunde (z.B. FACSVantage SE, s.o.), wobei eine noch komplexere Antikörper-Bibliothek zusätzlich durch Magnetobeads vorher angereichert werden kann. Beschrieben ist die Technik der für diese Erfindung benötigten FACS-Sortierung von Kern et al. (1998, Nature Medicine 4, 975-978) und Maecker et al. (2001, J Immunol Methods 255, 27-40), die damit Epitop-spezifische T-Zellen identifizieren konnten.

### Hybridom Antikörper Bibliothek

In einer bevorzugten Ausführungsform des erfindungsgemäßen Verfahrens erfolgt nach jedem Transfektionsschritt mit einer Schar unterschiedlicher DNA-Sequenzen und anschliessender spezifischer Rekombination (Figur 5, 6) eine Anreicherung von Oberflächen-gebundene Antikörper produzierenden Zellen mit möglichst hoher Antikörper-Diversität (Figur 9). Dies kann wie vorstehend beschrieben erfolgen, d.h. die Schar von Hybridom-Zellen wird wie beschrieben mit 2 unterschiedlichen Färbereagenzien eingefärbt und anschliessend werden im FACS-Sorter möglichst viele Zellen sortiert. Abgereichert werden dabei die Zellen, die ein für die Ausgangszelle typisches Färbemuster zeigen. Dies geschieht z.B. durch den Nachweis eines myc-Epitops in der vH-Domäne der Ausgangszelle. Alternativ kann z.B. ausgenützt werden, dass die Ausgangszelle vor der spezifischen Rekombination keine Antikörper auf der Oberfläche präsentiert, sondern stattdessen eine G418-Resistenz in den Genlocus der monospezifischen Zelllinie eingebaut ist. Angereichert werden dagegen die Zellen, die eine, insbesondere humane IgG-Kette bzw. Kappa- oder Lambda-Kette präsentieren. Dies sind die Zellen, die eine produktive spezifische Rekombination durchgeführt haben.

Das Resultat dieser Vorgehensweise ist eine komplexe (>10⁶ verschiedene Hybridom-Spezifitäten) Hybridom-Bibliothek (Figur 5, 6, 9), deren einzelne Mitglieder grosse Mengen eines humanen Antikörpers auf der Oberfläche präsentieren (ca. 10⁴ bis 10⁶ Antikörpermoleküle pro Zelle). Die Zahl der präsentierten Antikörper pro Zelle bewegt sich in einem vergleichsweise engen Rahmen, da alle Zellen von demselben parentalen Hybridom abstammen. Gegenstand der vorliegenden Erfindung ist damit auch eine Antikörper-Bibliothek, die mit dem erfindungsgemäßen Verfahren erhältlich ist. Diese umfasst vorzugsweise eine Schar von mindestens .100 unterschiedlichen Zellen, mehr bevorzugt eine Schar von mindestens 1000 Zellen.

Die beschriebene Vorgehensweise zur Herstellung einer Hybridom Antikörper Bibliothek ist auch in den Figuren 3, 5 und 9 beschrieben:
■ Die vorstehend beschriebene, möglichst komplexe Schar von vL-Gen-Plasmiden wird in möglichst viele Zellen (ca. 3x 10⁹ Zellen) einer monospezifischen Zelllinie elektroporiert. Gleichzeitig wird in die Zellen z.B. ein Flp-Expressionsvektor elektroporiert;
■ Der Anteil der erfolgreich elektroporierten Hybridome liegt dabei i.d.R. bei 30-40% der überlebenden Zellen. Damit entsteht eine Schar von Hybridom-Zellen, die in einer Frequenz von etwa 1 aus 300 unterschiedliche vL-Domänen auf der Oberfläche präsentieren, so dass eine Hybridom-Bibliothek einer Komplexität von ca. 10⁷ unterschiedlicher Hybridome (Gesamtmenge 3x 10⁹ Zellen), d.h. unterschiedlicher Antikörper, entsteht;
■ Diese Schar von Hyridomzellen wird optional expandiert und danach wie beschrieben mit zwei unterschiedlichen Färbereagenzien eingefärbt;
■ Anschliessend werden im FACS-Sorter oder mit Magnetobeads möglichst viele Zellen sortiert. Abgereichert werden dabei die Zellen der monospezifischen Zelllinie sowie unproduktiv rekombinierte Zellen, angereichert werden die Zellen, die eine humane Kappa- oder Lambda-Kette präsentieren;
■ Die vorstehend beschriebene, möglichst komplexe Schar von vH-Gen-Plasmiden wird in möglichst viele Zellen der gerade beschriebenen Schar von Hybridom-Zellen elektroporiert. Gleichzeitig wird in die Zellen ein Cre-Expressionsvektor elektroporiert;
■ Damit entsteht eine Schar von Hybridom-Zellen, die in einer Frequenz von etwa 1 aus 300 unterschiedliche vH-Domänen auf der Oberfläche präsentieren;
■ Diese Schar von Hybridom-Zellen wird erneut wie beschrieben mit 2 unterschiedlichen Färbereagenzien eingefärbt und
■ Anschliessend werden im FACS-Sorter oder mit Magnetobeads möglichst viele Zellen sortiert. Abgereichert werden dabei die Zellen der für den vH-Genlocus monospezifischen Zelllinie sowie unproduktiv rekombinierte Zellen, angereichert werden die Zellen, die eine humane IgG-Kette präsentieren (Figur 9).

Besonders bevorzugt ist ein Verfahren, wobei die Genloci die Antikörperloci sind und das aktive vH-Gen, vLambda-Gen oder vKappa-Gen enthalten.

Besonders bevorzugt ist dabei eine Ausführungsform, bei der die von der jeweiligen Zelle exprimierten monoklonalen humanen Antikörper durch das differenzielle Spleissen der konstanten Domänen eines IgG, IgM, IgA, IgD oder IgE kovalent an die Oberfläche der sie exprimierenden Zelle gebunden sind.

Bei dem erfindungsgemäßen Verfahren werden vorzugsweise pro expandierter einzelner Zelle mehr als 10² verschiedene Zellen erhalten, die jeweils unterschiedliche Proteine exprimieren.

In einer weiteren bevorzugten Ausführungsform des erfindungsgemäßen Verfahrens erstrecken sich die homologen Bereiche der transfizierten DNA zu den jeweiligen Antikörper-Genloci der Zelle, die die spezifischen Rekombinationssignale flankieren, über mindestens 400 Basenpaare.

In einer noch mehr bevorzugten Ausführungsform des erfindungsgemäßen Verfahrens ist das Intron in Richtung 5'-Ende vor dem M1-Exon eines IgG, IgM, IgA, IgD oder IgE-Gens um mehr als 50 Basenpaare verkürzt.

In einer weiteren bevorzugten Ausführungsform des erfindungsgemäßen Verfahrens wird nach der Transfektion mit einer Vielzahl von unterschiedlichen DNA-Sequenzen aus der dadurch entstehenden Vielzahl von unterschiedlichen Zellen eine Anreicherung derjenigen Zellen durchgeführt, die auf der Zelloberfläche die an die Oberfläche der sie jeweils exprimierenden Zellen gebundenen Antikörper präsentieren, so dass eine Zellpopulation mit möglichst hoher Antikörper-Diversität entsteht.

Besonders bevorzugt ist eine Ausführungsform des erfindungsgemäßen Verfahrens, wobei bei den Transfektionsschritten mit der Vielzahl von unterschiedlichen DNA-Sequenzen in die Genloci, die jeweils flankiert von Erkennungsstellen für eine Rekombinase sind, ausserdem DNA-Sequenzen mit exprimierbaren, die entsprechenden Rekombinase codierenden DNA-Sequenzen transfiziert und/oder aktiviert werden.

### Selektion von Antikörpern

Die vorliegende Erfindung beschreibt auch ein Verfahren zur Isolierung eines monoklonalen Antikörpers mit einer gewünschten Spezifität (siehe Figur 9), wobei das Verfahren dadurch gekennzeichnet ist, dass man eine gemäß dem erfindungsgemäßen Verfahren hergestellte Antikörper-Bank mit dem entsprechenden Antigen inkubiert und im Anschluss daran die Zelllinie isoliert oder anreichert, die an ihrer Oberfläche das Antigen gebunden hat.

Der Fachmann kennt Verfahren hinsichtlich des Inkontaktbringens des Antigens mit einer Antikörper-Bibliothek und hinsichtlich der Selektion des gewünschten Antikörpers (Liddell und Weeks, 1996, "Monoclonal Antibodies: Principles and Practice." Spektrum-Verlag ISBN 3827400481; Goding, J. W., 1996, "Production and Application of Monoclonale Antibodies in Cell Biology, Biochemistry and Immunology. Third Edition.

Erschienen im Verlag Academic Press Limited, 24-28 Oval Road, London NW1 7DX; ISBN 0-12-287023-9). Diese Verfahren sind auch ausführlich für die Selektion Oberflächen-präsentierter rekombinanter Antikörper aus Phagenbibliotheken (u.a. de Kruif et al., 1995, PNAS 92, 3938-3942) und aus bakteriellen Bibliotheken (Fuchs et al., 1996, J. of Immunotechnology. 2, 97-102) beschrieben worden. Bei der vorliegenden Erfindung werden diese Verfahren insbesondere an das Screenen eukaryontischer Zellpopulationen mit Hilfe von Magnetobeads oder eines FACS-Sorters angepasst. Auch diese Verfahren sind dem Fachmann bekannt: Sie sind identisch mit dem Färben von lebenden Zellen für den FACS (Fluorescence Activated Cell Sorter) und in einer Vielzahl von speziellen Laborhandbüchern ausführlich beschrieben (s.o.).

Vorzugsweise werden die gemäß dem erfindungsgemäßen Verfahren hergestellten Zellen der Antikörper-Bibliothek mit biotinyliertem und danach mit Streptavidin-FITC-markiertem Antigen gefärbt (oder direkt FITC-markiertes Antigen) und die stärksten Färbeereignisse selektioniert. Falls gewünscht kann die Antikörper-Bibliothek auch mit einem Gemisch von optional unterschiedlich markierten Antigenen gefärbt werden und wieder die stärksten Färbeereignisse selektioniert werden. Danach werden die einzelnen Zellen in Zellkultur expandiert und produzieren jeweils den gewünschten, insbesondere humanen monoklonalen Antikörper. In der Regel wird für die Antigenspezifische Selektion nur eine einzige Selektionsrunde benötigt, da die sehr hohe Zahl der präsentierten Antikörper sehr klare Signale ergibt. Insbesondere besteht auch die Möglichkeit die auf Grund einer Spleissvariante in den Kulturüberstand sekretierten Antikörper einer Hybridom-Antikörper-Bibliothek, einer daraus gewonnenen Sub-Bibliothek oder von einzelnen Klonen zur Charakterisierung einer gesuchten Aktivität heranzuziehen. In der Antikörper-Bibliothek sollten auf Grund der Neukombination der vH- und vL-Domänen auch Antikörper-Spezifitäten gegen humane Antigene repräsentiert sein.

### Selektion von affineren Antikörpern

In einer besonders bevorzugten Ausführungsform ist das erfindungsgemäße Verfahren weiter dadurch gekennzeichnet, dass dabei hochaffine Antikörper erzeugt werden. Dabei kann der Fachmann gemäß den nachstehend beschriebenen Verfahren vorgehen.

Beispielsweise können affinere monoklonale Antikörper per FACS selektioniert werden. Dazu wird, wie vorstehend beschrieben, die Antikörper-Bibliothek oder eine davon abgeleitete Schar unterschiedlicher Antigen-spezifischer Hybridomzellen mit PE-markiertem Antigen gefärbt. Diese Zellen werden mit FITC-markiertem Protein G gegengefärbt. Im FACS-Sorter werden anschliessend die Färbeereignisse mit dem höchsten Quotienten PE-Färbung : FITC-Färbung selektioniert. Die einzelnen Zellen werden in Zellkultur expandiert und produzieren jeweils einen humanen monoklonalen Antikörper vergleichsweise hoher Affinität für das zur Selektion verwendete Antigen. Dies stellt eine sehr einfache Methode dar, um hochaffine monoklonale Antikörper aufzufinden. Dabei ermöglicht eine einfach durchführbare Normalisierung der Zahl der präsentierten Antikörper einen "on line"-Affinitätsvergleich der aufgefundenen Antikörper-Spezifitäten, da das Verhältnis Antikörper-gebundener und -ungebundener Antigene ein direktes Mass für die Affinität des Antikörpers an sein Antigen ist (Figur 10).

In einer bevorzugten Ausführungsform ist das vorstehende Verfahren dadurch gekennzeichnet, dass der Isolierung hochaffiner Antikörper die Einführung von Mutationen innerhalb der variablen Antikörpergene vorausgeht. Dies kann z.B. mittels der Durchführung einer somatischen Hypermutation bzw. einer Genkonversion der Antikörpergene erfolgen, die wie beschrieben gefolgt wird von der Selektion veränderter, insbesondere affinerer auf der Zelloberfläche präsentierter Antikörper (Figur 11).

Beispielsweise kann ein "Chain-shuffling" und eine anschliessende Selektion hochaffiner monoklonaler Antikörper durchgeführt werden. Dabei wird zunächst, wie beschrieben z.B. mit FITC-markiertem Antigen, aus der hergestellten Antikörper-Bibliothek eine davon abgeleitete Schar Antigen-spezifischer Hybridomzellen selektioniert. Diese Schar selektionierter Hybridomzellen wird in Zellkultur expandiert. Anschliessend wird eine der variablen Domänen, wie vorstehend beschrieben, durch ein spezifisches Rekombinationsereignis gegen eine Schar unterschiedlicher DNA-Sequenzen, insbesondere andere variable Domänen ausgetauscht, (Genkonversion). Die dabei entstandene Schar von Hybridomzellen wird in Zellkultur expandiert. Anschliessend werden daraus, wie vorstehend beschrieben, vergleichsweise (Figur 10) hochaffine monoklonale humane Antikörper mit Hilfe eines FACS-Sorters selektioniert. Dabei handelt es sich auch um eine sehr einfache Methode, um eine Schar von Hybridomen herzustellen, aus der hochaffine monoklonale Antikörper isoliert werden können.

Alternativ kann in eine Template-DNA, insbesondere in vorselektionierte variable Domänen, eine Vielzahl von ungerichteten Mutationen durch eine fehleranfällige-PCR eingeführt werden (Stemmer, 1994, Nature 370, 389-391). Anschliessend wird wie beschrieben eine möglichst grosse Zahl dieser mutierten variablen Antikörpergene mittels spezifischer Rekombinationssignale in den Antikörperlokus einrekombiniert und danach die Zellen z.B. im FACS selektioniert, die einen Antikörper höherer Affinität auf der Oberfläche präsentieren. Die ungerichteten Mutationen können dabei nach dem von Stemmer entwickelten Verfahren auch miteinander kombiniert werden (Genkonversion / somatische Hypermutation).

Ein weiterer erfindungsgemäßer Weg zur Erzeugung ungerichteter Mutationen erfolgt mit Hilfe eukaryontischer Expressionsvektoren, die die Gene RAD54, RecQ4, und/oder das Gen DNA PolX mu in lymphoiden Zellen exprimieren, insbesondere in Kombination mit anti-sense-RNA gegen XRCC2, XRCC3 oder RAD51B (Kitao et al., 1998, Cloning of two new human helicase genes of the RecQ family: biological significance of multiple species in higher eukaryotes. Genomics 54, 443-452; Aoufouchi et al., 2000, Two novel human and mouse DNA polymerases of the polX family, Nucleic Acids Res 28, 3684-3693; Sale et al., 2001 Ablation of XRCC2/3 transforms immunoglobulin v gene conversion into somatic hypermutation, 2001, Nature 412, 921-926; Expressionsvektoren z.B. pCEP4, pREP7, pREP10, pEBVHis oder pREP4 von Invitrogen). Die Genprodukte RAD54, RecQ4, und DNA PolX mu sind Teil des für die Einführung somatischer Hypermutationen in die aktiven Antikörpergene verantwortlichen Mutatorkomplexes, während die Unterdrückung der Expression von XRCC2, XRCC3 oder RAD51B durch vermehrte Einzelstrangbrüche die somatischen Hypermutationen offensichtlich initiiert. Dabei werden insbesondere beim Prozess der Bildung von Memory-Zellen des Organismus ca. 1,5 kb DNA-Sequenzen downstream des aktiven vH, vKappa oder vLambda Promotors ungerichtet mutiert, ungeachtet dessen, welche DNA-Sequenzen dort vorliegen. Zur Erzeugung einer somatischen Hypermutation kann Beispielsweise so vorgegangen werden, dass aus der erfindungsgemäßen Antikörper-Bibliothek eine davon abgeleitete Schar Antigen-spezifischer Hybridomzellen mit FITC-markiertem Antigen wie vorstehend beschrieben selektioniert wird. Diese Schar selektionierter Hybridomzellen wird in Zellkultur expandiert und anschliessend wird in diese Zellen ein Gemisch der vorstehend beschriebenen Expressionsvektoren elektroporiert. Die dabei entstandene Schar von Hybridomzellen wird in Zellkultur expandiert. Anschliessend werden daraus, wie vorstehend beschrieben, hochaffine monoklonale humane Antikörper mit Hilfe eines FACS-Sorters selektioniert.

Besonders bevorzugt ist eine Ausführungsform des vorstehenden Verfahrens, bei dem die Zelle mit einer Vielzahl von DNA-Sequenzen transfiziert wird, die jeweils von spezifischen Rekombinationssignalen flankiert sind und wobei die Vielzahl von DNA-Sequenzen mittels "error-prone"-PCR gewonnen wurde.

### Humanisierung bereits existierender Hybridome

Die vorliegende Erfindung betrifft somit Verfahren zur Humanisierung bereits existierender Maus-Hybridome mittels homologer Rekombination (Figur 3, 7). Dabei kann beispielsweise mit einem vorgegebenen immer gleichen DNA-Vektor jede beliebig wählbare Maus-IgG1-Hybridomzelle in eine Mensch- IgG1-Hybridomzelle umgewandelt werden (Figur 3, 7), oder mit anderen DNA-Vektoren in Mensch-IgG2, IgG3, IgG4, IgA1, IgA2, IgE oder IgM. Wieder andere DNA-Vektoren ermöglichen den Umbau eines Maus-IgM-Hybridoms usw. Dieses besonders einfache und vorteilhafte Verfahren kommt dabei ohne den Einsatz eines störenden Resistenzmarkers aus (siehe auch Baker et al., 1994, J. Immunological Methods 168, 25-32), da die Veränderung durch die Oberflächenpräsentation des veränderten Genprodukts zur Selektion der veränderten Zelle ausgenützt wird (Figur 7). Ermöglicht wird dieses Verfahrens neuerdings durch die Bereitstellung von sehr schnellen FACS-Sortierern (z.B. FACSVantage SE, Becton-Dickinson), die mittlerweile ca. 10⁸ Zellen pro Stunde sortieren können, wodurch auch die sehr seltenen homologen Rekombinationsereignisse aufgefunden werden können. Ansonsten werden hierzu dem Fachmann bekannte Standardtechniken angewendet.
Somit betrifft die vorliegende Erfindung ein Verfahren zur Humanisierung einer Hybridomzelle, das dadurch gekennzeichnet ist, dass
(a)eine eine oder mehrere humane konstante IgG-, IgM-, IgA-, IgD- oder IgE-Domänen codierende DNA-Sequenz in die Hybridomzelllinie transfiziert wird;
(b) die DNA-Sequenz der humanen konstanten IgG-, IgM-, IgA-, IgD- oder IgE-Domänen von DNA-Sequenzen flankiert wird, die zu den chromosomalen Genbereichen homolog sind, die die konstanten Domänen des aktiven, die schwere Kette des Antikörpers codierenden Genlocus der genannten Hybridomzelle flankieren;
(c) eine oder wenige Zellen expandiert werden, die auf Grund einer homologen Rekombination eine IgG-, IgM-, IgA-, IgD- oder IgE-schwere Kette mit einem humanisierten konstanten Anteil exprimieren;
(d) eine eine humane konstante Kappa- oder Lambda-Domäne codierende DNA-Sequenz in die Hybridomzelllinie transfiziert wird, wobei diese DNA-Sequenz von DNA-Sequenzen flankiert wird, die zu den chromosomalen Genbereichen homolog sind, die die konstante Kappa- oder Lambda-Domäne des aktiven Kappa- oder Lambda-Genlocus der Hybridomzelle flankieren;
(e) anschliessend eine oder wenige Zellen expandiert werden, die auf Grund einer homologen Rekombination eine Kappa- oder Lambda-Kette mit einem humanisierten konstanten Anteil exprimieren,
wobei die exprimierten Antikörper auf Grund der kovalenten Kopplung der membranständigen Spleissvariante der schweren Antikörperkette an die Oberfläche der sie jeweils exprimierenden Zellen gebunden sind, was den Nachweis und die Selektion derjenigen Zellen erlaubt, die humanisierte konstante Antikörperdomänen präsentieren.

In einer bevorzugten Ausführungsform dieses Verfahrens werden zunächst die konstanten Domänen der leichten Antikörperkette humanisiert und danach die konstanten Domänen der schweren Antikörperkette, wobei noch mehr bevorzugt die Vorgehensweise ist, dass nur die konstanten Domänen der schweren Antikörperkette oder nur die konstanten Domänen der leichten Antikörperkette humanisiert werden.

In einer weiteren bevorzugten Ausführungsform des vorstehenden Verfahrens ist das Intron in Richtung 5'-Ende vor dem M1-Exon des aktiven IgG-, IgM-, IgA-, IgD- oder IgE-Gens um mehr als 50 Basenpaare verkürzt.

In einer weiteren bevorzugten Ausführungsform des vorstehenden Verfahrens sind an die humanisierten konstanten Domänen zusätzliche Protein-codierende DNA-Sequenzen fusioniert, vorzugsweise codieren die zusätzlichen Protein-codierenden DNA-Sequenzen eine Linkersequenz und einen Einzelkettenantikörper, der C-terminal an die konstante Domäne der leichten Antikörperkette fusioniert ist.

In einer weiteren bevorzugten Ausführungsform des vorstehenden Verfahrens erstrecken sich die homologen Bereiche der transfizierten DNA-Sequenzen zu den jeweiligen Antikörper-Genloci der Hybridom-Zelle über mindestens 400 Basenpaare.

In einer weiteren bevorzugten Ausführungsform des vorstehenden Verfahrens werden bei den homologen Rekombinationen keine Resistenzmarker in die Zellen eingeführt, die zur Selektion homologer Rekombinationsereignisse eingesetzt werden.

Die Erfindung wird durch die nachstehenden Beispiele erläutert.

### Beispiel 1: Humanisierung eines Hybridom-Antikörpers

Als Beispiel für einen zu humanisierenden monoklonalen Antikörper diente die Hybridom Zelllinie HEA125. Diese Maus-Hybridom-Zelllinie produziert einen Maus IgG1-Antikörper zusammen mit einer Maus-Kappa-Kette. Dieser monoklonale Antikörper erkennt das menschliche Tumorassoziierte Antigen Ep-CAM mit vergleichsweise hoher Affinität und Spezifität (Moldenhauer et al., 1987, Br J Cancer 56, 714-722).

### a. Kulturbedingungen

Die Zelllinie HEA125 wurde in Kultur genommen und expandiert. Als Kulturmedium wurde RPMI 1640 (Gibco BRL #31870-025) mit einem Zusatz von 10% FCS, 1mM Pyruvat und 2mM Glutamin verwendet. Die sonstigen Kulturbedingungen (Plastikgefässe von Falcon 25cm³; 37°C Wärmeschrank; 5-7,5% CO₂-Begasung, maximal 10⁶ Zellen pro ml, etc.) sind dem Fachmann bekannt.

### b. Selektion einer Subpopulation von HEA125

Zunächst wurden die expandierten Zellen 2x mit eiskaltem Dulbeccos PBS (DPBS) gewaschen und ca. 10⁷ Zellen pro 400µl mit FITC-markiertem Ziege-anti-Maus IgG -Antikörper gefärbt (Dianova). Als Gegenfärbung zur Identifizierung toter Zellen wurde Propidium Jodid (1µg/ml) verwendet. Nach erneutem waschen mit eiskaltem DPBS wurden die 5% der Zellpopulation mit Hilfe eines FACS-Sorters sortiert (FACSVantage SE, Becton-Dickinson), die die stärkste grüne Fluoreszenz aufwiesen. Als Ergebnis wurde eine Subpopulation von HEA125-Zellen aufgefunden und unter den oben angegebenen Kulturbedingungen expandiert, die vergleichsweise viele Membran-gebundene Antikörper aufwies.

### c. Chimäre Maus-Mensch DNA-Sequenzen

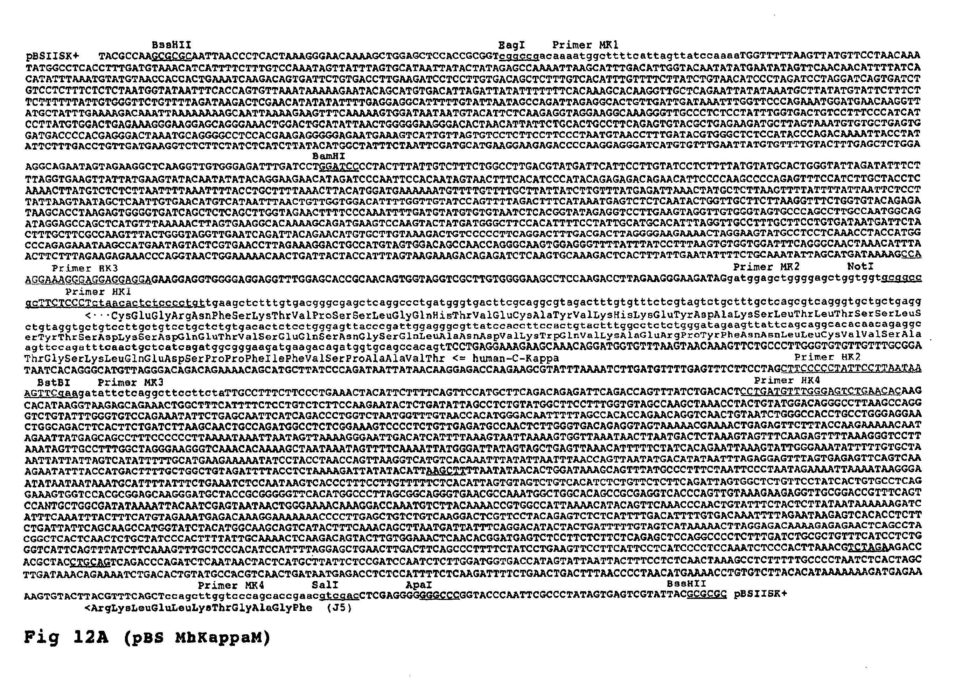

Mehrere einzelne Genfragmente wurden in dem Klonierungsvektor pBSIISK+ (Stratagene) zu chimären Maus-Mensch DNA-Sequenzen zusammengesetzt. Die einzelnen Genfragmente wurden dabei mittels PCR hergestellt (Roche Diagnostics; Expand Long Template PCR System; siehe auch für die PCR-Bedingungen). Als Template für die genomischen Maus-Gensequenzen diente genomische DNA der Maus Hybridomzelllinie HEA125, als Template für die genomischen Mensch-Gensequenzen diente genomische DNA menschlicher Blutzellen. Die Isolierung genomischer DNA und die benötigten Klonierungstechniken sind in diversen Laborhandbüchern beschrieben (siehe z.B. Sambrook and Russell: Molecular Cloning, a laboratory manual, 3rd Edition, 2001, ISBN 0-87969-577-3). In Figur 12 sind die resultierenden chimären DNA Sequenzen abgebildet, deren Sequenz überprüft wurde. Die verwendeten Primer für die Klonierung in pBSIISK+ sind in Figur 12 beschrieben. In Figur 12A ist die chimäre DNA-Sequenz des Vektors pBS MhKappaM abgebildet, der zur Humanisierung der konstanten Kappa-Kette von HEA125 verwendet wurde. In Figur 12B ist die chimäre DNA-Sequenz des Vektors pBS MhIgG1M abgebildet, der zur Humanisierung der konstanten IgG1 CH1, CH2 und CH3-Domänen von HEA125 verwendet wurde.

### d. Optimale Elektroporationsbedingungen für HEA125

Die optimalen Elektroporationsbedingungen für die Transfektion von HEA125 mit DNA wurden wie folgt ausgetestet:
■ Die Zellen wurden in RPMI-Medium + 10% FCS + 1mM Pyruvat + 2 mM Glutamin kultiviert;
■ anschliessend 2x in eiskaltem DPBS gewaschen;
■ in ca. 10⁷ Zellen pro 400µl DPBS-Puffer mit 0.5 mM Mg²⁺ + 0.1 mM Ca²⁺ aufgenommen;
■ zu 400µl Zellen je 10µg supercoiled Plasmid DNA pEGFP N3 MCS (Clontech) zugegeben;
■ in einer 4 mm breiten Elektroporationsküvette gemischt;
■ die Küvette mit den Zellen 10 min auf Eis inkubiert;
■ anschliessend mit dem BTX-Elektroporator (AGS) 1 Strompulse von je 2ms Dauer bei 500V - 450V gegeben;
■ je 10⁷ Zellen in 10ml RPMI + 10% FCS + 20% konditioniertes Medium dazu in 50cm³ Flaschen kultiviert;
■ 2 Tage später 2x in eiskaltem DPBS mit 2 % FCS gewaschen und
■ im FACS die durch EGFP hervorgerufene grüne Fluoreszenz gemessen.

Als Ergebnis wiesen 30-40% der im FACS untersuchten transfizierten Hybridomzellen eine vergleichsweise deutliche grüne Fluoreszenz auf (Kontrolle: Elektroporation mit irrelevanter Vektor DNA).

### e. Humanisierung der C-Kappa Domäne von HEA125

Der in Beispiel 1c und Figur 12A beschriebene chimäre Maus-Kappa konstante Domäne Humanisierungsvektor pBS MhKappaM wurde mit dem Restriktionsenzym BglI linearisiert und je 10µg linearisierte Plasmid DNA mit der in Beispiel 1b beschriebenen expandierten Subpopulation von HEA125 Zellen versetzt. Diese Zellen wurden mit den in Beispiel 1d beschriebenen optimierten Elektroporationsbedingungen transfiziert. Nach 2-4 Tagen in Kultur (beschrieben in Beispiel 1a) wurden die Zellen 2x mit eiskaltem DPBS gewaschen und ca. 10⁸Zellen pro 4ml mit FITC-markiertem Ziege-anti-Maus Kappa -Antikörper und gleichzeitig mit PE-konjugiertem Ziege-anti-Mensch Kappa -Antikörper gefärbt (Southern Biotechnology Associates). Als Gegenfärbung zur Identifizierung toter Zellen wurde Propidium Jodid verwendet. Nach erneutem waschen mit eiskaltem DPBS wurden die Zellen mit Hilfe eines FACS-Sorters sortiert, wobei ca. 10⁸Zellen in 2 Stunden sortiert wurden.

Als Ergebnis wurden 2-5 einzelne Zellen pro 10⁸HEA125-Zellen sortiert, die eine deutliche Phycoerythrin (PE)-spezifische rote Fluoreszenz aufwiesen. Die Einzelzellen wurden unter den in Beispiel 1a beschriebenen Kulturbedingungen 2-3 Wochen expandiert. Die resultierenden Klone wurden anschliessend erneut wie beschrieben mit FITC-markiertem Ziege-anti-Maus-Kappa-Antikörper und gleichzeitig mit PE-konjugiertem Ziege-anti-Mensch-Kappa-Antikörper gefärbt und im FACS analysiert. Zwei Klone mit deutlichem roten Fluoreszenz Signal wurden weiter propagiert. Die genomische konstante Kappa-Domäne dieser Klone wurde mittels PCR und der Primer HK3 und HK4 vervielfältigt (Primer siehe Figur 12A) und sequenziert. Als Ergebnis wurde der Klon HEA125-hKappa mit genomisch codierter konstanter humaner Kappa Domäne weiter propagiert. Die Sequenz des Übergangs von genomischer Maus Kappa-DNA zu genomischer Mensch Kappa-DNA dieses Klons ist in Figur 12A dargestellt.

Alternativ wurden Zellen in 96-Loch-Platten zu Pools von bis zu 1x10⁴ Zellen sortiert, bis 2x10⁵ Zellen hochwachsen gelassen und im ELISA nach positiven Pools, die humanisierte AK exprimierende Zellen enthalten, gescreent. Zum Nachweis diente der POX gekoppelte Ziege-anti-Mensch-Kappa-AK. Positive Pools wurden subkloniert und das Verfahren wurde wiederholt bis die Einzelklone HEA125-hKappa identifiziert waren.

### f. Humanisierung der konstanten IgG1 CH1, CH2 und CH3 Domänen

Der in Beispiel 1c und Figur 12B beschriebene chimäre Maus-IgG1 Humanisierungsvektor pBS MhIgG1M wurde mit dem Restriktionsenzym SspI linearisiert und je 10µg linearisierte Plasmid DNA mit Zellen des in Beispiel 1e beschriebenen teilweise humanisierten Subklons HEA125-hKappa versetzt. Zellkultur, FACS, Färbung der Zellen sind identisch wie in Beispiel 1e beschrieben, mit dem Unterschied, dass an Stelle des PE-konjugierten Ziege-anti-Mensch-Kappa -Antikörpers ein PE-konjugierter Ziege-anti-Mensch-IgG -Antikörper verwendet wurde. Als Ergebnis wurden zunächst 1-3 einzelne Zellen pro 10⁸ HEA125-hKappa-Zellen sortiert, die eine deutlich erhöhte Phycoerythrin (PE)-spezifische rote Fluoreszenz aufwiesen. Nach der Kontrollsequenzierung der homolog rekombinierten Bereiche mit Hilfe der PCR-Primer HG3 und HG4 (siehe Figur 12B) wurde die Zelllinie HEA125-hIgGlhKappa weiter propagiert, die neben einer konstanten humanen Kappa Domäne die konstanten IgG1 CH1, CH2 und CH3 Domänen genomisch codiert. Die Sequenz des Übergangs von genomischer Maus IgG1-DNA zu genomischer Mensch IgG1-DNA dieses Klons ist in Figur 12B dargestellt.

Damit sind bei einer im Prinzip beliebig wählbaren IgG1 produzierenden Maus Hybridom Zelle, die konstanten Domänen zu menschlichem IgG1 humanisiert worden. Maus Hybridom Zellen, die IgG2a, IgG2b, IgG3, IgA, IgE oder IgM produzieren, können mit anderen chimären DNA-Sequenzen ganz analog humanisiert werden. Je nachdem, welcher Humanisierungsvektor verwendet wird, können die einzelnen Antikörperklassen dabei auch umgewandelt oder verändert werden, wodurch Beispielsweise ein Maus IgM in Mensch IgG1, IgG2, IgG3, IgG4, IgA1, IgA2 oder IgE umgewandelt wird. Gleiches gilt für die Umwandlung einer konstanten Maus-Kappa-Domäne in eine konstante Mensch Lambda-Domäne.

### Beispiel 2: Erzielung einer verstärkten Oberflächenexpression von Antikörpern

Anstelle des in Beispiel 1f beschriebenen chimären Maus-IgG1 Humanisierungsvektors pBS MhIgG1M, wurde der Vektor pBS MhIgG1Mdelta350 verwendet, der die in Figur 12B beschriebene Deletion von ca. 350Bp im Intron zwischen der CH3 und der M1-Domäne aufweist, ansonsten aber identisch mit dem in Beispiel 1f und Figur 12B beschriebenen Vektor pBS MhIgG1M ist. Die Vorgehensweise war dieselbe wie in Beispiel 1f beschrieben. Auch hierbei wurden etwa 1-2 aus ca. 10⁸ Zellen mit einer homologen Rekombination im Bereich des Antikörper Genlocus der schweren Kette aufgefunden.

Als Ergebnis dieses Beispiels wurde die Zelllinie HEA125-mhIgG1hKappa propagiert, die verglichen mit der in Beispiel 1f beschriebenen Zelllinie HEA125-hIgGlhKappa auf Grund des verkürzten Introns zwischen der CH3 Domäne und der M1 Domäne von IgG1 deutlich mehr Membran gebundene humanisierte Antikörper aufweist (siehe auch Figur 7, Hybridomzelle 2H).

### Beispiel 3: Einführung spezifischer Rekombinationssignale in den vH-Genlocus von HEA125

### a. Chimäre DNA-Sequenzen

Mehrere einzelne Genfragmente wurden in dem Klonierungsvektor pBSIISK+ zu chimären DNA-Sequenzen zusammengesetzt. Die einzelnen Genfragmente wurden dabei mittels PCR hergestellt (Roche Diagnostics; Expand Long Template PCR System; siehe auch für die PCR-Bedingungen). Die dafür verwendeten PCR-Primer sind in Figur 13B dargestellt. Als Template für die genomischen Maus-Gensequenzen diente genomische DNA der Maus Hybridomzelllinie HEA125. Als Template für das Resistenzgen PGKneo diente der Vektor ploxPfrtPGKneofrtloxP (Erich Greiner, dkfz, Abteilung Molekularbiologie der Zelle I). In Figur 13B ist der resultierende Vektor pBS MvHG418M abgebildet, dessen Sequenz anschliessend überprüft wurde.

### b. G418-Vorselektion

Die in Beispiel 2 erhaltene Zelllinie HEA125-hIgGlhKappa wurde mit dem in Figur 13B beschriebenen linearisierten Vektor pBS MvHG418M transfiziert, wobei die in Beispiel 1d beschriebenen Elektroporationsbedingungen angewendet wurden. Zwei Tage nach der Elektroporation wurden die mit DNA transfizierten Zellen zunächst einer Vorselektion mit G418 unterzogen (je nach Charge 200-800µg G418 pro ml für 14 Tage; Zellkulturbedingungen ansonsten wie in Beispiel 1). Die expandierten G418-resistenten Zellen wurden anschliessend wie in Beispiel 1b beschrieben mit FITC-markiertem Ziege-anti-Mensch-IgG-Antikörper gefärbt und im FACS sortiert. Dabei wurden als Ergebnis wie oben beschrieben ca. 2.000 Einzelzellen sortiert, die keine Antikörper-spezifische Färbung aufwiesen (d.h., möglichst geringe grüne Fluoreszenz). Diese Einzelzellen wurden unter den in Beispiel 1a beschriebenen Kulturbedingungen 2-3 Wochen expandiert.

### c. Selektion von Klonen mit loxP Stellen im vH-Genlocus

Aus den in Beispiel 3b erhaltenen Klonen wurde die genomische DNA isoliert, die jeweils als Template für eine PCR diente (Roche Diagnostics; Expand Long Template PCR System; siehe auch für die PCR-Bedingungen). Die dafür verwendeten PCR-Primer vHG418-3 und vHG418-4 sind in Figur 13B beschrieben. Die jeweiligen PCR-Banden wurden in einem 0,8% TAE-Agarosegel nach Grösse aufgetrennt und mit dem erwarteten Ergebnis verglichen. Als Ergebnis wiesen 8 der 2.000 untersuchten Klone eine PCR-Bande der erwarteten Grösse von ca. 1,85 kb auf. Nach der Kontrollsequenzierung der genomischen DNA (bzw. der beschriebenen PCR-Bande) wurde als Ergebnis der Klon HEA125-mhIgG1hKappa-loxPG418 mit genomisch codierten spezifischen Rekombinationssignalen (loxP und loxP511) im Bereich des aktiven vH-Genlocus weiter propagiert.

### d. Spezifische Rekombination

Zellen der in Beispiel 3b beschriebenen ca. 2.000 Klone wurden gepoolt, alternativ wurde der in Beispiel 3c beschriebene expandierte Klon HEA125-mhIgGlhKappa-loxPG418 verwendet. In diese Zellen wurde der in Figur 15B dargestellte Vektor pBS loxPvHmyc elektroporiert. Dieser Vektor codiert das genomisch rekombinierte vH-Gen von HEA125, das von einer loxP und einer loxP511 Stelle flankiert wird. Zusätzlich zu dem vH-Gen von HEA125 ist in die CDR3 Region der vH-Domäne ein myc-tag eingebaut. Gleichzeitig wurde der Cre-Expressionsvektor pMC-Cre in die Zellen elektroporiert (Bedingungen dafür siehe Beispiel 1d).

Nach 2-4 Tagen in Kultur (beschrieben in Beispiel 1a) wurden die Zellen 2x mit eiskaltem DPBS gewaschen und ca. 10⁸Zellen pro ml mit FITC-konjugiertem Ziege-anti-Mensch IgG -Antikörper gefärbt (Dianova). Alternativ wurde mit FITC-markiertem 1-9E10 anti-myc Antikörper gefärbt. Als Gegenfärbung zur Identifizierung toter Zellen wurde Propidium Jodid verwendet. Nach erneutem Waschen mit eiskaltem DPBS wurden mit Hilfe eines FACS-Sorters Einzelzellen mit starker FITC-Fluoreszenz sortiert.

Alternativ wurden nach 2-4 Tagen in Kultur (beschrieben in Beispiel 1a) die Zellen in je 1x10⁴* Zellen/Loch in 96-Loch-Platten sortiert und nach 5 weiteren Tagen in Kultur im ELISA das Wiedervorhandensein von Antikörpern, die von den Zellen ins Medium abgegeben wurden, mit einem POXgekoppelten GAM Ig61Fc AK detektiert. Kulturen, die positive Signale zeigten, wurden bis zu Einzelzellklonen subkloniert.

Es ergaben:
■ Der in Beispiel 3c beschriebene Ausgangsklon HEA125-mhIgGlhKappa-loxPG418 ca. 1% Zellen mit vergleichsweise starker FITC-Fluoreszenz;
■ Der in Beispiel 3b beschriebene Pool von Zellen ca. 15 Zellen mit vergleichsweise starker FITC-Fluoreszenz pro 10⁸ sortierter Zellen.

Insgesamt 20 sortierte Einzelzellen wurden unter den in Beispiel 1a beschriebenen Kulturbedingungen 2-3 Wochen expandiert. Anschliessend wurden die Einzelklone auf G418-Resistenz getestet wie in Beispiel 3b beschrieben. Im Ergebnis waren 17 der 20 getesteten Klone sensitiv gegen G418. Nach PCR und Sequenzierung des vH-Genlocus mit Hilfe der PCR-Primer vHG418-3 und vHG418-4 (siehe Figur 13B), wurde die G418-sensitive Zelllinie HEA125-mhloxPmyc weiter expandiert. Zellen dieses Klons wurden 2x mit eiskaltem DPBS gewaschen und ca. 10⁸Zellen pro ml mit FITC-markiertem anti-myc-Antikörper (Mycl-9E10-Epitop; Evan et al., 1985, Mol. Cell. Biol. 5, 3610-3616) gefärbt. Als Gegenfärbung zur Identifizierung toter Zellen wurde Propidium Jodid verwendet. Es zeigte sich im FACS eine starke grüne Fluoreszenz der Zelllinie HEA125-mhloxPmyc.

Die resultierende G418-sensitive Zelllinie HEA125-mhloxPmyc produziert einen monoklonalen Hybridom IgG1 Antikörper einer definierten Spezifität (im Beispiel die vH-Domäne von HEA125 mit einem zusätzlichen c-myc-tag im CDR3 der vH-Domäne) mit humanisierten konstanten Domänen. Zusätzlich dazu ist das vH-Exon innerhalb des vH-Genlocus von 2 unterschiedlichen loxP-Stellen flankiert. Ein grosser Teil der produzierten Antikörper (in der Grössenordnung von 10⁴ bis 10⁶ Antikörper Moleküle) befindet sich kovalent an einen Membran Anker gebunden auf der Oberfläche der Hybridom Zellen.

### Beispiel 4: Einführung spezifischer Rekombinationssignale in den vKappa-Genlocus von HEA125

### a. Chimäre DNA-Sequenzen

Wie in Beispiel 3 beschrieben wurden mehrere einzelne Genfragmente in dem Klonierungsvektor pBS zu chimären DNA-Sequenzen zusammengesetzt. In Figur 13A ist der resultierende DNA-Vektor pBS MKappaG418M abgebildet, dessen Sequenz anschliessend überprüft wurde.

### b. G418-Vorselektion

Die in Beispiel 3d erhaltene Zelllinie HEA125-mhloxPmyc wurde mit dem in Beispiel 4a beschriebenen linearisierten Vektor pBS MKappaG418M wie in Beispiel 3b beschrieben elektroporiert, anschliessend wie beschrieben eine G418-Selektion durchgeführt und Zellen ohne exprimierte Kappa Kette im FACS sortiert. Bei der Färbung wurde im Unterschied zu Beispiel 3b jedoch mit FITC-markiertem Ziege-anti-Mensch Kappa -Antikörper gefärbt. Dabei wurden als Ergebnis wie in Beispiel 3b beschrieben ca. 2.000 Einzelzellen sortiert, die keine Kappa-spezifische Färbung aufwiesen (d.h., möglichst geringe grüne Fluoreszenz). Diese Einzelzellen wurden unter den in Beispiel 1a beschriebenen Kulturbedingungen 2-3 Wochen expandiert.

### c. Einführung von FRT Stellen in den vKappa-Genlocus

Aus den in Beispiel 4b beschriebenen Klonen wurde wie beschrieben die genomische DNA isoliert, eine PCR durchgeführt und die erwartete Grösse der PCR-Bande überprüft. Die dafür verwendeten PCR-Primer KG418-3 und KG418-4 sind in Figur 13A beschrieben. Als Ergebnis wiesen 14 der 2.000 untersuchten Klone eine PCR-Bande der erwarteten Grösse von ca. 1,9 kb auf. Nach der Kontrollsequenzierung der genomischen DNA (bzw. der beschriebenen PCR-Bande) wurde als Ergebnis der Klon HEA125-mhloxPmycFRTG418 mit genomisch codierten spezifischen Rekombinationssignalen (FRT0 und FRT3) im Bereich des aktiven vKappa-Genlocus weiter propagiert. Die resultierende Zelllinie HEA125-mhloxPmycFRTG418 produziert einen monoklonalen Hybridom IgG1 Antikörper einer definierten Spezifität (im Beispiel die vH-Domäne von HEA125 mit einem zusätzlichen c-myc-tag im CDR3 der vH-Domäne) mit humanisierten konstanten Domänen. Zusätzlich dazu ist das vH-Exon innerhalb des vH-Genlocus von 2 unterschiedlichen loxP-Stellen flankiert. An Stelle der vKappa-Domäne befindet sich ein G418 Resistenzgen, das von 2 verschiedenen FRT-Stellen flankiert ist.

### d. Spezifische Rekombination

Zellen der in Beispiel 4b beschriebenen ca. 2.000 Klone wurden gepoolt, alternativ wurde der in Beispiel 4c beschriebene expandierte Klon HEA125-mhloxPmycFRTG418 verwendet. In diese Zellen wurde der in Figur 15A dargestellte Vektor pBS FRTvKappa elektroporiert. Dieser Vektor codiert für das genomisch rekombinierte vKappa-Gen von HEA125 (ohne Leader-Exon), das von einer FRT0 und einer FRT3 Stelle flankiert wird. Gleichzeitig wurde der Flp-Expressionsvektor pOG44 in die Zellen elektroporiert (Bedingungen dafür siehe Beispiel 1d). Nach 2-4 Tagen in Kultur (beschrieben in Beispiel 1a) wurden die Zellen 2x mit eiskaltem DPBS gewaschen und ca. 10⁸Zellen pro ml mit PE-konjugiertem Ziege-anti-Mensch Kappa-Antikörper gefärbt (Dianova). Als Gegenfärbung zur Identifizierung toter Zellen wurde Propidium Jodid verwendet. Nach erneutem Waschen mit eiskaltem DPBS wurden mit Hilfe eines FACS-Sorters Einzelzellen mit starker PE-Fluoreszenz sortiert.

Es ergaben:
■ Der in Beispiel 4c beschriebene Ausgangsklon HEA125-mhloxPmycFRTG418 ca. 1% Zellen mit vergleichsweise starker PE-Fluoreszenz;
■ Der in Beispiel 4b beschriebene Pool von Zellen ca. 20 Zellen mit vergleichsweise starker PE-Fluoreszenz pro 10⁸ sortierter Zellen.

Insgesamt ca. 20 sortierte Einzelzellen wurden unter den in Beispiel 1a beschriebenen Kulturbedingungen 2-3 Wochen expandiert. Anschliessend wurden die Einzelklone auf G418-Resistenz getestet wie in Beispiel 4b beschrieben. Im Ergebnis waren 18 der 20 getesteten Klone sensitiv gegen G418, darunter alle von dem Klon HEA125-mhloxPmycFRTG418 abstammenden Subklone. Nach PCR und Sequenzierung des vKappa-Genlocus mit Hilfe der PCR-Primer KG418-3 und KG418-4 (siehe Figur 13A), wurde die G418-sensitive Zelllinie HEA125-mhRek weiter expandiert.

Die resultierende Zelllinie HEA125-mhRek produziert einen monoklonalen Hybridom IgG1 Antikörper einer definierten Spezifität mit humanisierten konstanten Domänen. Im Beispiel codiert die vH-Domäne von HEA125 ein zusätzliches c-myc-tag im CDR3 der vH-Domäne, das mit dem monoklonalen Antikörper 1-9E10 im FACS nachgewiesen werden kann. Zusätzlich dazu ist das aktive vH-Exon innerhalb des vH-Genlocus von 2 unterschiedlichen loxP-Stellen flankiert. Das aktive vKappa-Exon innerhalb des vKappa-Genlocus ist von 2 unterschiedlichen FRT-Stellen flankiert. Ein grosser Teil der produzierten Antikörper befindet sich kovalent an einen Membran Anker gebunden auf der Oberfläche der Hybridom Zellen.

### Beispiel 5: Herstellung einer vLambda-Gen-Bibliothek

### a. Computeranalyse menschlicher vLambda-Gene

Die genomischen Sequenzen des humanen vLambda Genlocus sind bekannt (One-megabase sequence analysis of the human immunoglobulin lambda gene locus"; Genome Res. 7:250-261(1997); Accession Numbers: D87000; D87007; D87009; D87010; D87014; D87015; D87016; D87017; D87018; D87021; D87022; D87023; D87024; X51755; X51754; siehe auch Immunoglobulin Facts Book, Lefranc und Lefranc, wo 33 funktionelle vLambda-Gene beschrieben sind). Die Computeranalyse von 24 bekannten humanen vLambda Gene ergab keine BstBI, BssHII, ClaI, DraI, HpaI, MluI, NotI, NruI, SacII, SalI, SnaBI, XhoI, EagI und keine SwaI Restriktionsstellen innerhalb eines Bereiches von ca. 300Bp upstream (in 5'-Richtung) von dem Startcodon des Leader-Exons der untersuchten Gene bis zu dem jeweiligen genomischen Rekombinationssignal am Ende des CDR3. Zusätzlich wurde die Region der J-Segmente untersucht, jeweils ab dem 5'-Ende der 4 aktiven J_{Lambda}-Gensegmente aus gerechnet bis jeweils ca. 200Bp downstream (in 3'-Richtung) vom 3'-Ende der J-Segmente (siehe D87018 und D87023). Im Ergebnis eignen sich damit insbesondere die Restriktionsstellen BssHII, MluI, NotI, SalI, XhoI und EagI zur Klonierung der Vielfalt humaner vLambda-Gene.

### b. vLambda-Gen-spezifische PCR-Primer

Hergestellt wurden vLambda-Gen-spezifische PCR-Primer, die jeweils an das Intron zwischen dem Leader Exon und dem jeweiligen vLambda-Exon hybridisieren (Figur 14A). Der vergleichbare Bereich des aktiven vKappa-Genlocus von HEA125 liegt etwa bei der SwaI-Schnittstelle im Intron zwischen Leader-Exon und vKappa-Exon. Die Primer hybridisieren an Bereiche, die jeweils ca. 130-170Bp upstream von dem 5'-Ende des jeweiligen vLambda-Exons liegen. Die Hybridisierungstemperatur der Primer wurde auf jeweils ca. 65°C berechnet. Zur Berechnung der Hybridisierungstemperatur dient dabei die Formel

Hybridisierungstemperatur = (2°C x Zahl der AT Bp + 4°C x Zahl der GC Bp) -5°C.

### c. J_{Lambda}-Gensegment-spezifische PCR-Primer

Die insgesamt 4 aktiven menschlichen J_{Lambda}-Gensegmente sind jeweils durch ein Intron von den benachbarten konstanten cLambda-Exons getrennt. Jeweils ca. 89Bp (im Bereich der BsaI Schnittstelle bei HEA125) downstream von den 3'-Enden der J_{Lambda}-Segmente gerechnet wurden insgesamt 4 J_{Lambda}-Gensegment-spezif ische PCR-Primer mit einer berechneten Hybridisierungstemperatur von jeweils ca. 65°C hergestellt (Figur 14A).

### d. Vervielfältigung menschlicher vLambda-Gene mittels PCR

Als Template-DNA wurde die genomische DNA menschlicher B-Lymphocyten verwendet. Die Isolierung genomischer DNA ist in diversen Laborhandbüchern beschrieben (siehe z.B. Sambrook and Russell: Molecular Cloning, a laboratory manual, 3rd Edition, Kapitel 6, 2001, ISBN 0-87969-577-3). Die PCR-Primer sind in Beispiel 5b und 5c beschrieben. Die Vielfalt der genomisch rekombinierten menschlichen vLambda-Gene wurde durch die Kombination der 4 unterschiedlichen J_{Lambda}-Gensegment-Primer mit den 24 verschiedenen vLambda-Gen-spezifischen Primern erhalten, d.h., es wurden 96 verschiedene PCR-Reaktionen durchgeführt. Die PCR-Bedingungen dafür sind in dem Expand Long Template PCR System beschrieben (Roche Diagnostics). Anschliessend wurden PCR-Banden von ca. 560Bp Länge getrennt nach Grösse selektioniert und gereinigt (Qiagen Gel Purifica.tion Kit). Diese insgesamt 96 PCR-Banden dienten jeweils als Template für eine weitere PCR mit den beschriebenen Bedingungen, wobei diesmal anstelle der in Beispiel 5b beschriebenen Primer, PCR-Primer verwendet wurden, deren Sequenzen an ihrem 5'-Ende um folgende Basen verlängert sind:
■ 5' attataACGCGT... (hier folgen jeweils die Sequenzen der in Beispiel 5b beschriebenen vLambda-Gen-spezifischen PCR-Primer (in das PCR-Produkt wird dadurch eine zur Klonierung benötigte MluI-Restriktionsstelle eingeführt);
■ 5' ttcGAAGTTCCTATTCTCTAGAAAGTATAGGAACTTC... (hier folgen jeweils die Sequenzen der in Beispiel 5b beschriebenen vLambda-Gen-spezifischen PCR-Primer (in das PCR-Produkt wird dadurch eine für eine alternative Klonierungsstrategie benötigte FRT0-Stelle eingeführt);
■ 5' attata GCGGCCGC... (hier folgen jeweils die Sequenzen der in Beispiel 5c beschriebenen J_{Lambda}-Gensegment - spezifischen PCR-Primer (in das PCR-Produkt wird dadurch eine zur Klonierung benötigte NotI-Restriktionsstelle eingeführt) und
■ 5' ttcGAAGTTCCTATACTATTTGAAGAATAGGAACTTC... (hier folgen jeweils die Sequenzen der in Beispiel 5c beschriebenen J_{Lambda}-Gensegment-spezifischen PCR-Primer (in das PCR-Produkt wird dadurch eine für eine alternative Klonierungsstrategie benötigte FRT3-Stelle eingeführt).

Wie beschrieben wurden die PCR-Banden nach Grösse selektioniert und gereinigt, so dass als Ergebnis 96 PCR-Banden vorlagen, jeweils flankiert von FRT0 und FRT3-Sequenzen. Weitere 96 PCR-Banden wurden erhalten, jeweils flankiert von MluI und NotI Restriktionsstellen.

### e. Klonierung menschlicher vLambda-Gene

In dem Klonierungsvektor pBS FRTvKappa flankieren 2 verschiedene FRT-Stellen das in HEA125 aktive vKappa-Exon (Figur 15A). Dies ermöglicht in Anwesenheit der FRTspezifischen Rekombinase Flp den Austausch der von den beschriebenen FRT-Stellen flankierten DNA-Sequenz *in vitro*. Zunächst wurde in den mit AatII geschnittenen Klonierungsvektor pBS FRTvKappa die gleichfalls AatII geschnittene DNA-Sequenz einkloniert. Nach der Transfektion der resultierenden rekombinierten DNA in *E.coli* wurden Einzelklone isoliert und deren Sequenz überprüft. Dabei wurde der Klonierungsvektor pBS FRTklon (Figur 15C) erhalten, dessen FRT-Stellen anstelle des in Figur 15A dargestelltenvKappa-Exons die oben, bzw. in Figur 15C beschriebenen Restriktionsstellen MluI, SalI und NotI flankieren.
Nach Verdau der isolierten Vektor DNA (Qiagen Plasmid Purification Kit) mit den Restriktionsenzymen MluI und NotI wurden die entsprechend verdauten in Beispiel 5d beschriebenen 96 verschiedenen PCR-Banden einligiert, die resultierenden Ligationsprodukte in *E.coli* transfiziert und anschliessend ein hochkomplexes Gemisch (>10⁶ verschiedene Transformanden) von Vektor-DNA (pBS FRTklon-vLambda) isoliert (Qiagen Plasmid Purification Kit).
Alternativ dazu wurden die von FRT-Stellen flankierten in Beispiel 5d beschriebenen 96 verschiedenen mit Hilfe von T4-DNA-Ligase zirkularisierten PCR-Banden zusammen mit Flp-Rekombinase und mit dem Vektor pBS FRTklon inkubiert, die resultierenden Rekombinationsprodukte mit dem Restriktionsenzym SalI geschnitten und wie gerade beschrieben in *E.coli* transfiziert und anschliessend ein hochkomplexes Gemisch von Vektor-DNA (pBS FRTklon-vLambda) isoliert.

### f. Spezifische Rekombination menschlicher vLambda-Gene in HEA125

Die in Beispiel 5e beschriebenen hochkomplexen Gemische von humaner vLambda-Vektor-DNA (pBS FRTklon-vLambda) wurden zusammen mit dem Flp-Expressionsvektor pOG44 in die Zellen des expandierten Klons HEA125-mhloxPmycFRTG418 (siehe Beispiel 4c) elektroporiert, die Zellen nach 3 Tagen mit FITC-markiertem Ziege-anti-Mensch-Kappa - Antikörper gefärbt und wie beschrieben mittels FACS-Sorter Zellen isoliert. Als Ergebnis wurden dabei ca. 0,7% der sortierten Zellen (bzw. ca. 10⁶ Zellen) erhalten und miteinander vereinigt, die im FACS eine deutliche grüne Fluoreszenz aufwiesen. Diese vLambda Zellbibliothek wurde in Zellkultur expandiert.

Das Resultat dieser Vorgehensweise ist eine komplexe (>10⁶ verschiedene Hybridom Spezifitäten) Hybridom Bibliothek (Figur 9), deren einzelne Mitglieder grosse Mengen jeweils unterschiedlicher humaner vLambda-Ketten jeweils fusioniert an eine konstante humane Kappa-Domäne auf der Oberfläche präsentieren.
Nicht dargestellt ist eine bevorzugte Ausführung mit der ein hochkomplexes Gemisch wie das beschriebene pBS FRTklon-vLambda erhalten werden kann. Dabei wird an Stelle des Klonierungsvektors pBSIISK+ ein in eukaryontischen Zellen episomal replizierender Vektor verwendet (basierend z.B. auf pCEP4, pREP7, pREP10, pEBVHis oder pREP4 von Invitrogen). Die Rekombinase Flp wird dabei von einer regulierbaren Expressionskassette codiert, die stabil chromosomal integriert ist. Systeme dieser Art werden u.a. von der Firma Invitrogen angeboten (GeneSwitch™ System K1060-01; T-Rex™ System K1020-01).

### Beispiel 6: Herstellung einer vKappa-Gen-Bibliothek

### a. Computeranalyse menschlicher vKappa-Gene

Die genomischen Sequenzen des humanen vKappa Genlocus sind bekannt (Immunoglobulin Facts Book, Lefranc und Lefranc, 2001, Academic Press, ISBN 0-12-441351-X). Die Computeranalyse von 34 bekannten funktionellen humanen vKappa Genen ergab keine BglI, BssHII, BstBI, ClaI, EagI, HindIII, MluI, NotI, NruI, PvuI, SacII, SfiI, SnaBI, SpeI und keine StuI Restriktionsstellen innerhalb eines Bereiches von ca. 200-300Bp upstream von dem Leader-Exon der untersuchten Gene bis zu dem jeweiligen genomischen Rekombinationssignal am Ende des CDR3. Zusätzlich wurde die Region der J-Segmente untersucht, vom 5'-Ende des aktiven J1_{Kappa}-Gensegment aus bis ca. 200Bp downstream vom 3'-Ende des aktiven J5_{Kappa}-Gensegmentes gerechnet (siehe Acession number J00242). Im Ergebnis eignen sich damit insbesondere die Restriktionsstellen BssHII, EagI, HindIII, MluI, NotI, SfiI und SpeI zur Klonierung der Vielfalt humaner vKappa-Gene. Das Restriktionsenzym SalI schneidet nur einmal ausserhalb des Gens IGKV1D-43.

### b. vKappa-Gen-spezifische PCR-Primer

Hergestellt wurden vKappa-Gen-spezifische PCR-Primer, die jeweils an das Intron zwischen dem Leader Exon und dem jeweiligen vKappa-Exon hybridisieren (Figur 14B). Der vergleichbare Bereich des aktiven vKappa-Genlocus von HEA125 liegt etwa bei der SwaI-Schnittstelle im Intron zwischen Leader-Exon und vKappa-Exon. Die Primer hybridisieren an Bereiche, die jeweils ca. 130-170Bp upstream von dem Beginn des jeweiligen vKappa-Exons liegen. Die Hybridisierungstemperatur der Primer wurde auf jeweils ca. 65°C berechnet.

### c. J_{Kappa}-Gensegment-spezifische PCR-Primer

Die insgesamt 5 aktiven menschlichen J_{Kappa}-Gensegmente sind im Unterschied zu den J_{Lambda}-Gensegmenten geclustert, wodurch immer dasselbe konstante cKappa-Exon verwendet wird, das durch ein Intron von den geclusterten J_{Kappa-}Segmenten getrennt ist. Jeweils ca. 89Bp (der vergleichbare Bereich liegt bei HEA125 bei der BsaI Schnittstelle) downstream von den 3'-Enden der unterschiedlichen J_{Kappa}-Segmente gerechnet wurden insgesamt 5 J_{Kappa}-Gensegment-spezifische PCR-Primer mit einer berechneten Hybridisierungstemperatur von jeweils ca. 65°C hergestellt (Figur 14B).

### d. Vervielfältigung menschlicher vKappa-Gene mittels PCR

Wie in Beispiel 5d für vLambda beschrieben, wurde die Vielfalt der genomisch rekombinierten menschlichen vKappa-Gene durch die Kombination der 5 unterschiedlichen J_{Kappa-}Gensegment-Primer mit 34 verschiedenen funktionellen vKappa-Gen-spezifischen Primern erhalten, d.h., es wurden 170 verschiedene PCR-Reaktionen durchgeführt. Diese insgesamt 170 PCR-Banden dienten jeweils als Template für weitere PCR-Reaktionen, wobei diesmal wie in Beispiel 5d beschrieben die verwendeten Primer an ihrem 5'-Ende um eine Restriktionsstelle, bzw. um FRT0 oder FRT3 verlängert wurden. Wie beschrieben wurden die PCR-Banden nach Grösse selektioniert und gereinigt, so dass als Ergebnis 170 PCR-Banden vorlagen (ca. 600Bp), jeweils flankiert von FRT0 und FRT3-Sequenzen. Weitere 170 PCR-Banden wurden erhalten (ca. 550Bp), jeweils flankiert von MluI und NotI Restriktionsstellen.

### e. Klonierung menschlicher vKappa-Gene

Nach Verdau der in Beispiel 5e beschriebenen isolierten Vektor DNAvon pBS FRTklon (Figur 15C) mit den Restriktionsenzymen MluI und NotI wurden die entsprechend verdauten in Beispiel 6d beschriebenen mit MluI und NotI-Stellen flankierten 170 verschiedenen PCR-Banden einligiert, die resultierenden Ligationsprodukte in *E.coli* transfiziert und anschliessend wie in Beispiel 5e beschrieben ein hochkomplexes Gemisch (>10⁶ verschiedene Transformanden) von Vektor-DNA (pBS FRTklon-vKappa) isoliert.
Alternativ dazu wurden die von FRT-Stellen flankierten in Beispiel 6d beschriebenen 170 verschiedenen jeweils zirkularisierten PCR-Banden zusammen mit Flp-Rekombinase mit dem Vektor pBS FRTvKappa inkubiert, die resultierenden Rekombinationsprodukte mit dem Restriktionsenzym SalI geschnitten und wie in Beispiel 5e beschrieben in *E.coli* transfiziert und anschliessend ein hochkomplexes Gemisch von Vektor-DNA (pBS FRTklon-vKappa) isoliert.

### f. Spezifische Rekombination menschlicher vKappa-Gene in HEA125

Wie in Beispiel 5f für vLambda beschrieben, wurden die in Beispiel 6e beschriebenen hochkomplexen Gemische von humaner vKappa-Vektor-DNA (pBS FRTklon-vKappa) zusammen mit dem Flp-Expressionsvektor pOG44 in die Zellen der expandierten Zelllinie HEA125-mhloxPmycFRTG418 elektroporiert, mit FITC-markiertem Ziege-anti-Mensch Kappa -Antikörper gefärbt und wie beschrieben mittels FACS-Sorter Zellen isoliert. Als Ergebnis wurden dabei ca. 0,8% der sortierten Zellen (bzw. ca. 10⁶ Zellen) erhalten und miteinander vereinigt, die im FACS eine deutliche grüne Fluoreszenz aufwiesen. Diese vKappa Zellbibliothek wurde in Zellkultur expandiert.

Das Resultat dieser Vorgehensweise ist eine komplexe (>10⁶ verschiedene Hybridom Spezifitäten) Hybridom Bibliothek (Figur 9), deren einzelne Mitglieder grosse Mengen jeweils unterschiedlicher humaner vKappa-Ketten jeweils fusioniert an eine konstante humane Kappa-Domäne auf der Oberfläche präsentieren.

### Beispiel 7: Herstellung einer Antikörper-Bibliothek

### a. Computeranalyse menschlicher vH-Gene

Die genomischen Sequenzen des humanen vH-Genlocus sind bekannt (EMBL Datenbank Accession numbers X97051; S64822; AB019437; AB019438; AB019439; AB019440; AB019441; siehe auch das Immunoglobulin Facts Book, Lefranc und Lefranc, 2001, Academic Press, ISBN 0-12-441351-X). Die Computeranalyse der in den Accession numbers X97051; S64822; AB019437; AB019438; AB019439; AB019440 und AB019441 aufgeführten 44 funktionellen humanen vH-Gene ergab keine BssHII, ClaI, MluI, NheI, NotI, NruI, PvuI, SalI, SfiI, SwaI und keine XhoI Restriktionsstellen innerhalb eines Bereiches von 300Bp upstream von dem 5'-Ende des Leader-Exon der untersuchten Gene bis zu dem jeweils das vH-Gen flankierenden genomischen Rekombinationssignal. Zusätzlich wurde die Region der J_{H-}Segmente untersucht, vom 5'-Ende des aktiven J1_{H}-Gensegment aus bis ca. 200Bp downstream vom 3'-Ende des aktiven J6_{H-}Gensegmentes gerechnet (siehe Acession numbers X97051; S64822). Im Ergebnis eignen sich damit insbesondere die Restriktionsstellen BssHII, MluI, NheI, NotI, SalI, SfiI und XhoI zur Klonierung der Vielfalt humaner vH-Gene. Die Restriktionsstellen BssHII, MluI, NotI und SalI (mit Ausnahme des Gens IGKV1D-43) kommen auch nicht in den untersuchten vLambda und vKappa-Genbereichen vor.

### b. vH-Gen-spezifische PCR-Primer

Hergestellt wurden vH-Gen-spezifische PCR-Primer, die jeweils ca. 182 Bp upstream von dem 5'-Ende des jeweiligen vH-Leader-Exons an die jeweiligen vH-Gene hybridisieren (Figur 14C). Die Hybridisierungstemperatur der Primer wurde auf jeweils ca. 65°C berechnet.

### c. J_{H}-Gensegment-spezifische PCR-Primer

Die insgesamt 6 aktiven menschlichen J_{H}-Gensegmente sind wie die J_{Kappa}-Gensegmente geclustert, wodurch immer dieselben konstanten CH-Exons verwendet werden (allerdings in Abhängigkeit von einem evtl. durchgeführten classswitch). Das CH1-Exon ist dabei durch ein Intron von den geclusterten J_{H}-Gensegmenten getrennt. Jeweils ca. 83Bp (der vergleichbare Bereich bei HEA125liegt bei der Bsu36I Schnittstelle) downstream vom 3'-Ende der jeweiligen J_{H-}Gensegmente gerechnet wurden insgesamt 6 J_{H}-Gensegment-spezifische PCR-Primer mit einer berechneten Hybridisierungstemperatur von jeweils ca. 65°C hergestellt (Figur 14C).

### d. Vervielfältigung menschlicher vH-Gene mittels PCR

Wie in Beispiel 5d für vLambda beschrieben, wurde die Vielfalt der genomisch rekombinierten menschlichen vH-Gene durch die Kombination der 6 unterschiedlichen J_{H-}Gensegment-Primer mit den 44 verschiedenen vH-Gen-spezifischen Primern erhalten, d.h., es wurden 6x44=264 verschiedene PCR-Reaktionen durchgeführt. Dabei zeigten 5 der 6 J_{H}-Gensegment-spezifischen PCR-Primer zusätzliche höhermolekulare PCR-Banden, die im TAE-Agarosegel von der jeweils ca. 790 Bp grossen Bande abgetrennt wurden. Dies liegt daran, dass z.B. der J2_{H}-PCR-Primer sowohl genomisch rekombinierte vHJ2_{H}-Fusionen, wie auch vHJ1_{H}-Fusionen vervielfältigt.
Wie in Beispiel 5d beschrieben dienten diese insgesamt 264 PCR-Banden einer Grösse von ca. 790 Bp jeweils als Template für eine weitere PCR mit den beschriebenen Bedingungen, wobei diesmal anstelle der in Beispiel 7b beschriebenen Primer, PCR-Primer verwendet wurden, deren Sequenzen an ihrem 5'-Ende um folgende Basen verlängert sind:
■ 5' attata ACGCGT... (hier folgen jeweils die Sequenzen der in Beispiel 7b beschriebenen vH-Gen-spezifischen PCR-Primer (in das PCR-Produkt wird dadurch eine zur Klonierung benötigte MluI-Restriktionsstelle eingeführt);
■ 5' ttcATAACTTCGTATAATGTATGCTATACGAAGTTAT... (hier folgen jeweils die Sequenzen der in Beispiel 7b beschriebenen vH-Gen-spezifischen PCR-Primer (in das PCR-Produkt wird dadurch eine für eine alternative Klonierungsstrategie benötigte loxP-Stelle eingeführt);
■ 5' attata GCGGCCGC... (hier folgen jeweils die Sequenzen der in Beispiel 7c beschriebenen J_{H}-Gensegment - spezifischen PCR-Primer (in das PCR-Produkt wird dadurch eine zur Klonierung benötigte NotI-Restriktionsstelle eingeführt) und;
■ 5' cctATAACTTCGTATAATGTATACTATACGAAGTTAT... (hier folgen jeweils die Sequenzen der in Beispiel 7c beschriebenen J_{H}-Gensegment -spezifischen PCR-Primer (in das PCR-Produkt wird dadurch eine für eine alternative Klonierungsstrategie benötigte loxP511-Stelle eingeführt).

Wie beschrieben wurden die PCR-Banden nach Grösse selektioniert und gereinigt, so dass als Ergebnis 264 PCR-Banden einer Grösse von ca. 860 Bp vorlagen, jeweils flankiert von loxP und loxP511-Sequenzen. Weitere 264 PCR-Banden wurden erhalten, jeweils flankiert von MluI und NotI Restriktionsstellen.

### e. Klonierung menschlicher vH-Gene

Nach Verdau der in Figur 15D beschriebenen isolierten Vektor DNA pBS loxPklon (Qiagen Plasmid Purification Kit) mit den Restriktionsenzymen MluI und NotI wurden die entsprechend verdauten in Beispiel 7d beschriebenen 264 verschiedenen PCR-Banden einligiert, die resultierenden Ligationsprodukte in *E.coli* transfiziert und anschliessend wie in Beispiel 5e beschrieben ein hochkomplexes Gemisch (>10⁶ verschiedene Transformanden) von Vektor-DNA isoliert (pBS loxPklon-vH).
Alternativ dazu wurden die von loxP-Stellen flankierten in Beispiel 7d beschriebenen 264 verschiedenen PCR-Banden zusammen mit Cre-Rekombinase mit dem Vektor pBS loxPklon inkubiert, die resultierenden Rekombinationsprodukte mit dem Restriktionsenzym SalI geschnitten und wie gerade beschrieben in *E.coli* transfiziert und anschliessend ein hochkomplexes Gemisch von Vektor-DNA (pBS loxPklon-vH) isoliert.

### f. Spezifische Rekombination menschlicher vH-Gene in HEA125

Wie in Beispiel 5f für vLambda beschrieben, wurden die in Beispiel 7e beschriebenen hochkomplexen Gemische von humaner vH-Vektor-DNA (pBS loxPklon-vH) zusammen mit dem Cre-Expressionsvektor pMC-Cre in die Zellen elektroporiert. Verwendet wurden dazu die in Beispiel 5f und 6f beschriebenen vLambda bzw. vKappa Zellbibliotheken.

Die Zellen wurden mit PE-konjugiertem Ziege-anti-Mensch IgG -Antikörper und gleichzeitig mit FITC-markiertem monoklonalem anti myc1-9E10-Antikörper gefärbt. Wie beschrieben wurden mittels FACS-Sorter Zellen isoliert. Als Ergebnis wurden dabei ca. 0,8% der sortierten Zellen (bzw. ca. 10⁷ Zellen) erhalten und miteinander vereinigt, die im FACS eine starke rote Fluoreszenz bei gleichzeitig möglichst geringer grüner Fluoreszenz aufwiesen. Diese Antikörper Zellbibliothek wurde in Zellkultur expandiert und Aliquots davon in flüssigem Stickstoff eingefroren.

Das Resultat dieser Vorgehensweise ist eine komplexe (>10⁷ verschiedene Hybridom Spezifitäten) Antikörper Bibliothek (Figur 9), deren einzelne Mitglieder grosse Mengen jeweils unterschiedlicher humaner IgG1-Antikörper jeweils fusioniert an konstante humane Domänen auf der Oberfläche präsentieren. Gleichzeitig sekretieren die Hybridomzellen den grösseren Teil der produzierten Antikörper ins Umgebungsmedium. Die einzelnen Verfahrensschritte orientierten sich dabei weitgehend an dem bekannten Stand der Technik.
Die vLambda-Domänen dieser Antikörper Bibliothek sind im Beispiel an die konstante Kappa-Domäne fusioniert. In einer nicht dargestellten weiteren bevorzugten Ausführungsform wurde deswegen das Beispiel 1e dahingehend geändert, dass an Stelle des in Figur 12A dargestellten Vektors pBS MhKappaM ein Vektor verwendet wurde, der nach der homologen Rekombination eine konstante Mensch-Lambda-Domäne codiert.

### Beispiel 8: Selektion monoklonaler Antikörper mittels FACS / Magnetobeads

Die in Beispiel 7f beschriebene Antikörper-Bibliothek wurde wie in Beispiel 1a beschrieben in Kultur genommen. Die expandierten Zellen wurden 2x mit eiskaltem DPBS gewaschen und ca. 10⁷ Zellen pro 400µl mit FITC-konjugiertem BSA gefärbt. Als Gegenfärbung zur Identifizierung toter Zellen wurde Propidium Jodid (1µg/ml) verwendet. Nach erneutem Waschen mit eiskaltem DPBS wurden die Zellen mit Hilfe eines FACS-Sorters sortiert. Als Ergebnis wurden ca. 15 pro 10⁷ Hybridomzellen aufgefunden, die eine vergleichsweise starke grüne Fluoreszenz aufwiesen. Die sortierten Einzelzellen wurden wie in Beispiel 1a beschrieben expandiert (u.U. zusammen mit Feeder-Zellen) und der Überstand mit den darin enhaltenen sekretierten Antikörpern im Western Blot auf Antigen-Spezifität hin untersucht. Als Ergebnis reagierte der Überstand von 2 Klonen mit BSA (MG ca. 68kd), 5 weitere Klone wiesen eine FITC-BSA spezifische Färbung auf (MG ca. 72kd).

Parallel dazu wurden ca. 10⁸ Zellen der in Beispiel 7f beschriebenen Antikörper-Bibliothek 2x mit eiskaltem DPBS gewaschen und anschliessend in 5ml DPBS-Puffer für 5 Minuten mit BSA, bzw. mit Ovalbumin gecoateten Magnetobeads inkubiert. Ungebundene Zellen wurden mit Hilfe eines Magneten weggewaschen und die übrig gebliebenen Magnetobead-gebundenen Zellen wie in Beispiel 1a beschrieben in Kultur genommen. Als Ergebnis reagierte der Überstand der mit BSA-Magnetobeads angereicherten Zellen im Western Blot mit BSA (MG ca. 68kd) nicht aber mit Ovalbumin, während der Überstand der mit Ovalbumin-Magnetobeads angereicherten Zellen mit BSA keine Färbung aufwies.

### Beispiel 9: Selektion affinerer monoklonaler Antikörper mittels FACS

Die von den fünf in Beispiel 8 beschriebenen FITC-BSAspezifischen Klonen sekretierten anti-FITC-Antikörper wurden jeweils mit Hilfe von Protein G-Sepharose gereinigt und jeweils ein Teil davon mit Meerrettichperoxidase konjugiert (anti-FITC-POX-Antikörper). Die Konzentration der verschiedenen gereinigten Antikörper wurde auf jeweils 1mg/ml in PBS eingestellt.

Eine ELISA-Platte wurde mit FITC-BSA gecoatet (jeweils 0,1µg in 100µl PBS), geblockt (mit 1% Milchpulver in 200µl PBS) und die 5 verschiedenen Peroxidase-konjugierten Antikörper (jeweils 1:2000 verdünnt in 100µl PBS-Tween20) mit ansteigenden Mengen der nicht Peroxidase-konjugierten Antikörper kompetitiert. Die nicht Peroxidase-konjugierten Antikörper wurden dabei jeweils 10 Minuten mit dem gecoateten FITC-Antigen vorinkubiert. Nach zwischen geschalteten Waschschritten wurde residuale gebundene Peroxidase mit dem Substrat OPD / H₂O₂ nachgewiesen.

In diesen Kompetitionsexperimenten erwies sich der vom Klon anti FITC2 produzierte anti-FITC2-Antikörper als der vergleichsweise affinste Antikörper. Wurde jeweils 1:2000 verdünnter Peroxidase-konjugierter anti-FITC1, 2, 3, 4 oder 5-Antikörper vorgegeben, so ergab sich eine halb maximale Hemmung des ELISA Signals von dem:
■ anti-FITC1-POX-Antikörper bei 1:1000 verdünntem anti-FITC2-Antikörper;
■ anti-FITC2- POX-Antikörper bei 1:500 verdünntem anti-FITC2-Antikörper;
■ anti-FITC3- POX-Antikörper bei 1:2000 verdünntem anti-FITC2-Antikörper;
■ anti-FITC4- POX-Antikörper bei 1:2000 verdünntem anti-FITC2-Antikörper;
■ anti-FITC5- POX-Antikörper bei 1:5000 verdünntem anti-FITC2-Antikörper.

Daraufhin wurden 10⁶ Zellen des anti-FITC2-Klons mit ca. 10⁷ Zellen des anti-FITC5-Klons gemischt, 2x mit eiskaltem DPBS gewaschen, mit FITC-BSA (10µg/ml) und gleichzeitig mit PE-konjugiertem ProteinG (10µg/ml) gefärbt. Zum Nachweis toter Zellen wurde wie beschrieben Propidium Jodid eingesetzt. Nach erneutem Waschen mit DPBS wurden die Zellen im FACS analysiert (Figur 10). Dabei wiesen ca. 10% der lebenden Zellen ein Verhältnis von grüner zu roter Fluoreszenz von ca. 0,8 auf (+/- 0,2) während ca. 90% der lebenden Zellen ein Verhältnis von grüner zu roter Fluoreszenz von ca. 0,08 aufwiesen (+/-0,03).

Wie oben beschrieben wurden ca. 10⁶ Zellen dieser Zellpopulationen jeweils getrennt sortiert, daraus die genomische DNA isoliert und als Template für eine PCR mit den Primern vHG418-3 und -4 verwendet (Figur 13B). Genau gleich wurde mit Zellen der Klone anti FITC2 und anti FITC5 verfahren. Die PCR-Banden wurden in einem 1% TAE Agarosegel nach Grösse aufgetrennt, wobei die unterscheidbar leicht grössere PCR-Bande der sortierten Zellpopulation mit einem Verhältnis von grüner zu roter Fluoreszenz von ca. 0,8 dem Klon anti-FITC2 entsprach (ca. 1,95kb), während die gleichfalls korrespondierende leicht kleinere PCR-Bande der sortierten Zellpopulation mit einem Verhältnis von grüner zu roter Fluoreszenz von ca. 0,08 dem Klon anti-FITC5 entsprach.

Dieses Beispiel zeigt eine sehr einfache Methode, um hoch affine monoklonale Antikörper aufzufinden. Dabei ermöglicht eine einfach durchführbare Normalisierung der Zahl der präsentierten Antikörper einen "on line" Affinitätsvergleich der aufgefundenen Antikörper Spezifitäten (Figur 10).

### Beispiel 10: "Chain-shuffling" und Selektion hochaffiner monoklonaler Antikörper

In die in Beispiel 9 beschriebene expandierte Zelllinie anti-FITC5 wurde die in Beispiel 7e beschriebene Vielfalt der vH-Gene wie in Beispiel 7f beschrieben mit Hilfe eines Cre-Expressionsvektors einrekombiniert. Die resultierende anti-FITC-Antikörper Bibliothek wurde wie in Beispiel 9 beschrieben mit FITC-BSA und gleichzeitig mit PE-konjugiertem ProteinG gefärbt.

Als Resultat wiesen ca. 70% der lebenden Zellen ein Verhältnis von grüner zu roter Fluoreszenz von ca. 0,08 auf (+/- 0,03) während ca. 0,02% der lebenden Zellen ein Verhältnis von grüner zu roter Fluoreszenz von 0,2 bis 0,7 aufwiesen (+/-0,1).

Dieses Beispiel zeigt eine sehr einfache Methode, um eine Schar von Hybridomen herzustellen, aus der vergleichsweise hochaffine monoklonale Antikörper isoliert werden können.

### Beispiel 11: Durchführung einer somatischen Hypermutation zum Erhalt hochaffiner Antikörper

Die cDNA-Sequenzen der Gene RAD 54, RecQ4, polX mu, RAD51B, XRCC2 und XRCC3 sind bekannt. Zunächst wurden die cDNA-Sequenzen der Gene RAD54, RecQ4 bzw. polX mu jeweils unter der Kontrolle eines RSV-Promotors in den eukaryontischen Expressionsvektor pREP4 (Invitrogen) einkloniert und die korrekte Sequenz überprüft. Dabei wurden die Expressionsvektoren pREP4-RAD54, pREP4-RecQ4 und pREP4-polXmu und die anti-sense-RNA Expressionsvektoren pREP4-RAD51B, pREP4-XRCC2 und pREP4-XRCC3 erhalten.

Zirkuläre DNA der Vektoren pREP4-XRCC2, pREP4-RAD54, pREP4-RecQ4 und pREP4-polXmu wurde im Verhältnis 1:1:1:1 gemischt und unter den in Beispiel 1d beschriebenen optimierten Elektroporationsbedingungen in die in Beispiel 9 beschriebene expandierte Zelllinie anti-FITC5 elektroporiert. Anschliessend wurden die Zellen wie in Beispiel 1a beschrieben für 2-3 Tage in Kultur genommen. Optional können Zellen, die die episomal replizierenden pREP4-Vektoren aufgenommen haben anschliessend mit Hygromycin selektioniert werden. Anschliessend wurden die Zellen wie in Beispiel 9 beschrieben mit FITC-BSA und gleichzeitig mit PE-konjugiertem ProteinG gefärbt. Als Resultat wiesen ca. 90% der lebenden Zellen ein Verhältnis von grüner zu roter Fluoreszenz von ca. 0,08 auf (+/- 0,03) während ca. 0,002% der lebenden Zellen ein Verhältnis von grüner zu roter Fluoreszenz von 0,2 bis 0,7 aufwiesen (+/-0,2).

Dieses Beispiel zeigt eine weitere sehr einfache Methode, um eine Schar von Hybridomen herzustellen, aus der vergleichsweise hochaffine monoklonale Antikörper isoliert werden können.

### Beispiel 12: Erzeugung eines bispezifischen Antikörpers

### a. Chimäre DNA-Sequenzen

Mehrere einzelne Genfragmente wurden in dem Klonierungsvektor pBSIISK+ zu chimären DNA-Sequenzen zusammengesetzt. Die einzelnen Genfragmente wurden dabei mittels PCR hergestellt (Roche Diagnostics; Expand Long Template PCR System; siehe auch für die PCR-Bedingungen).

Als Template für die Gensequenzen diente cDNA der Maus Hybridomzelllinie HEA125, sowie einExpressionsvektor für den scFv-Antikörper 215 (Kontermann et al., 1995, Characterization of the epitope recognised by a monoclonal antibody directed against the largest subunit of *Drosophila* RNA polymerase II. Biol. Chem. Hoppe-Seyler 376, 473-481). Der Bereich der Linkersequenz zwischen cKappa(HEA) und dem scFv(215) Antikörper wurde durch ein synthetisches überhängendes PCR-Oligonucleotid hergestellt. Als Ergebnis wurde der Vektor pBS FRT KappaHEAscFv215 erhalten. In Figur 17A ist die resultierende chimäre DNA Sequenz abgebildet, deren Sequenz anschliessend überprüft wurde.

### b. Spezifische Rekombination in den vKappa-Genlocus

In die Zelllinie HEA125-mhloxPmycFRTG418 wurde der beschriebene Vektor pBS FRT KappaHEAscFv215 elektroporiert. Gleichzeitig wurde der Flp-Expressionsvektor pOG44 in die Zellen elektroporiert (Bedingungen dafür siehe Beispiel 1d). Nach 2-4 Tagen in Kultur (Bedingungen dafür siehe Beispiel 1a) wurden die Zellen 2x mit eiskaltem DPBS gewaschen und ca. 10⁸Zellen pro ml mit PE-konjugiertem Ziege-anti-Maus-Kappa-Antikörper gefärbt (Southern Biotechnology Associates). Als Gegenfärbung zur Identifizierung toter Zellen wurde Propidium Jodid verwendet. Nach erneutem Waschen mit eiskaltem DPBS wurden mit Hilfe eines FACS-Sorters Einzelzellen mit starker PE-Fluoreszenz sortiert. Dabei ergab der in Beispiel 12a beschriebene Ausgangsklon HEA125-mhloxPmycFRTG418 ca. 0,1% Zellen mit vergleichsweise starker PE-Fluoreszenz. Anschliessend wurden 5 sortierte Einzelzellen unter den in Beispiel 1a beschriebenen Kulturbedingungen 2-3 Wochen expandiert. Die resultierenden Klone wurden anschliessend erneut wie beschrieben mit PE-markiertem Ziege-anti-Maus-Kappa-Antikörper gefärbt und im FACS analysiert. Zwei Klone mit deutlichem roten Fluoreszenz Signal wurden weiter propagiert. Die genomische DNA dieser Klone wurde mittels PCR und den Primern KG418-3 und KG418-4 vervielfältigt (Primer siehe Figur 13A; siehe auch Figur 17A) und sequenziert.

Die im Ergebnis erhaltene Zelllinie HEA125-mhloxPmycFRTscFv215 produziert einen bispezifischen monoklonalen Hybridom IgG1 Antikörper einer definierten Spezifität (im Beispiel die vH-Domäne von HEA125 mit einem zusätzlichen c-myc-tag im CDR3 der vH-Domäne) mit humanisierten konstanten IgG1-Domänen. Zusätzlich dazu ist das vH-Exon innerhalb des vH-Genlocus von 2 unterschiedlichen loxP-Stellen flankiert. Im Bereich des von FRT-Stellen flankierten aktiven Maus vKappa-Genlocus wird die vKappa-Domäne von HEA125 codiert, die mit der Maus cKappa Domäne, einer Linkersequenz und dem scFv215 Antikörper fusioniert ist.

### Beispiel 13: Herstellung eines bifunktionellen Antikörpers

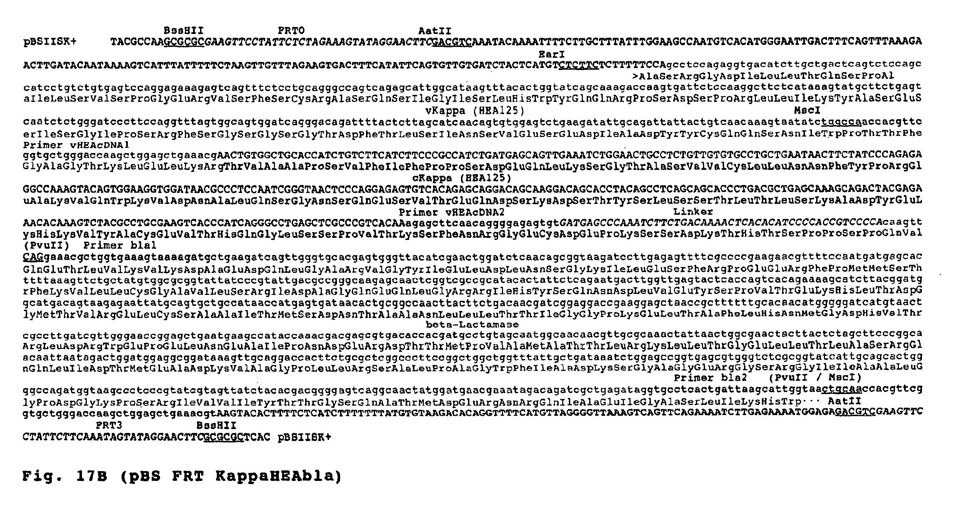

Wie im Beispiel 12 beschrieben wurde die Zelllinie HEA125-mhloxPmycFRTG418 mit chimärer DNA elektroporiert, mit PE-konjugiertem Ziege-anti-Maus-Kappa -Antikörper gefärbt, einzelne Zellen mit vergleichsweise starker PE-Fluoreszenz sortiert, Einzelklone expandiert und die genomische Sequenz überprüft. Im Unterschied zu Beispiel 12 wurde dabei an Stelle des beschriebenen Vektors pBS FRT KappaHEAscFv215 der Vektor pBS FRT KappaHEAbla verwendet (Figur 17B). Dabei ergab der beschriebene Ausgangsklon HEA125-mhloxPmycFRTG418 ca. 0,1% Zellen mit vergleichsweise starker PE-Fluoreszenz.

Die im Ergebnis erhaltene Zelllinie HEA125-mhloxPmycFRTbla produziert einen monoklonalen Hybridom IgG1 Antikörper einer definierten Spezifität (im Beispiel die vH-Domäne von HEA125 mit einem zusätzlichen c-myc-tag im CDR3 der vH-Domäne) mit humanisierten konstanten IgG1-Domänen. Zusätzlich dazu ist das vH-Exon innerhalb des vH-Genlocus von 2 unterschiedlichen loxP-Stellen flankiert. Im Bereich des von FRT-Stellen flankierten aktiven Maus vKappa-Genlocus wird die vKappa-Domäne von HEA125 codiert, die mit der Maus cKappa Domäne, einer Linkersequenz und dem Gen für die beta-Lactamase fusioniert ist.

### Beispiel 14: Änderung der Antikörperspezifität mittels spezifischer Rekombination

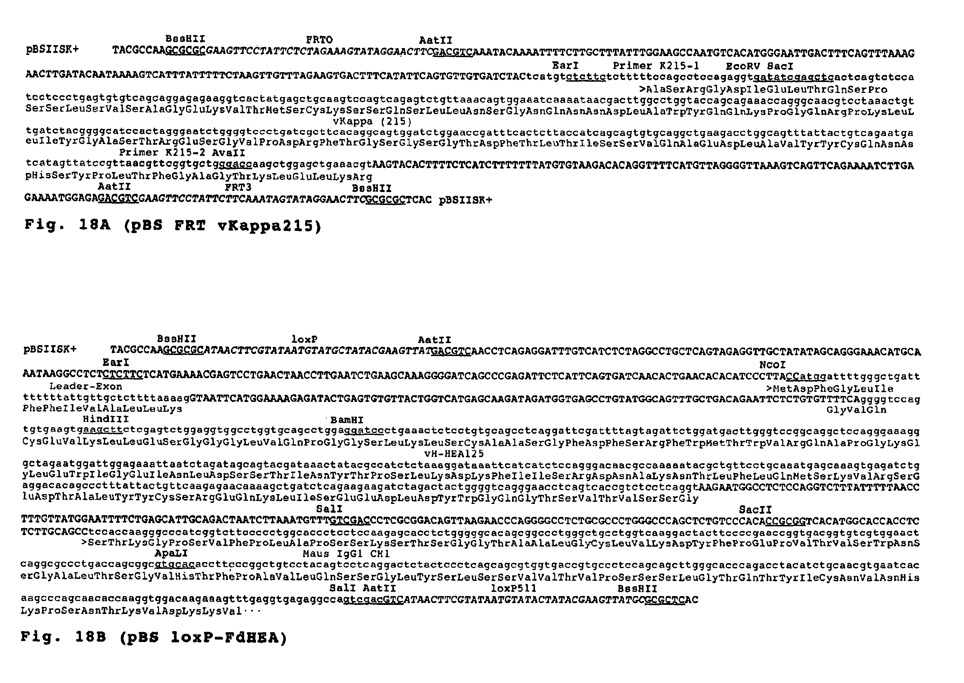

Wie im Beispiel 12 beschrieben wurde die Zelllinie HEA125-mhloxPmycFRTG418 mit chimärer DNA elektroporiert, mit PE-konjugiertem Ziege-anti-Mensch-Kappa -Antikörper gefärbt, einzelne Zellen mit vergleichsweise starker PE-Fluoreszenz sortiert, Einzelklone expandiert und die genomische Sequenz überprüft. Im Unterschied zu Beispiel 12 wurde dabei an Stelle des beschriebenen Vektors pBS FRT KappaHEAscFv215 der Vektor pBS FRT Kappa215 verwendet (Figur 18A). Dabei ergab der beschriebene Ausgangsklon HEA125-mhloxPmycFRTG418 ca. 0,1% Zellen mit vergleichsweise starker PE-Fluoreszenz.

Die im Ergebnis erhaltene Zelllinie HEA125-mhloxPmycFRT215 produziert einen monoklonalen Hybridom IgG1 Antikörper einer definierten Spezifität (im Beispiel die vH-Domäne von HEA125 mit einem zusätzlichen c-myc-tag im CDR3 der vH-Domäne) mit humanisierten konstanten Domänen. Zusätzlich dazu ist das vH-Exon innerhalb des vH-Genlocus von 2 unterschiedlichen loxP-Stellen flankiert. Im Bereich des von FRT-Stellen flankierten aktiven Maus vKappa-Genlocus wird die vKappa-Domäne von dem Antikörper 215 codiert. Eine analoge Vorgehensweise ergibt eine veränderte vH-Domäne.

### Beispiel 15: Herstellung eines Fab-Antikörpers mittels spezifischer Rekombination

### a. Chimäre DNA-Sequenzen

Mehrere einzelne Genfragmente wurden in dem Klonierungsvektor pBSIISK+ zu chimären DNA-Sequenzen zusammengesetzt. Die einzelnen Genfragmente wurden dabei mittels PCR hergestellt (Roche Diagnostics; Expand Long Template PCR System; siehe auch für die PCR-Bedingungen). Als Template für die Gensequenzen diente cDNA der Maus Hybridomzelllinie HEA125. Als Ergebnis wurde der Vektor pBS loxP-FdHEA erhalten. Dieser Vektor codiert die mit der Maus IgG1-CH1 Domäne fusionierte vH-Domäne von HEA125. In Figur 18B ist die resultierende chimäre DNA Sequenz abgebildet, deren Sequenz anschliessend überprüft wurde.

### b. Spezifische Rekombination in den vH-Genlocus

Der in Beispiel 15a beschriebene Vektor pBS loxP-FdHEA wurde zusammen mit dem Cre-Expressionsvektor pMC-Cre wie in Beispiel 1d beschrieben in die in Beispiel 4d beschriebene Zelllinie HEA125-mhRek elektroporiert und anschliessend durch limitierte Verdünnung der eingesetzten ca. 10⁷ Zellen ca. 2.000 Klone getrennt propagiert. Eine ELISA-Platte wurde mit jeweils 100µl des jeweiligen Zellkulturüberstandes der beschriebenen 2.000 Klone gecoatet, geblockt (mit 1% Milchpulver in 200µl PBS) und anschliessend mit Peroxidase-konjugiertem 1-9E10 anti-myc Antikörper bzw. mit Peroxidase gekoppeltem Ziege-anti-Maus IgG Antikörper (Dianova) gefärbt (jeweils 1:2000 verdünnt in 100µl PBS). Nach zwischen geschalteten Waschschritten wurde residual gebundene Peroxidase mit dem Substrat OPD nachgewiesen. Im Ergebnis zeigten 3 der untersuchten Klone ein erhöhtes Signal bei der Färbung mit Peroxidase gekoppeltem Ziege-anti-Maus-IgG-Antikörper, während gleichzeitig kein detektierbares Signal bei der Färbung mit Peroxidase-konjugiertem 1-9E10-anti-myc Antikörper nachweisbar war. Die genomische Squenz der Klone wurde überprüft und im Ergebnis der Klon HEA125 Fab propagiert.

Die resultierende Zelllinie HEA125 Fab sekretiert ein monoklonales Fab-Antikörperfragment einer definierten Spezifität (im Beispiel die vH- und vKappa-Domänen von HEA125) mit humanisierter konstanter Kappa-Domäne. Zusätzlich dazu ist das vH-CH1-Exon innerhalb des vH-Genlocus von 2 unterschiedlichen loxP-Stellen flankiert. Das vKappa-Exon ist von 2 unterschiedlichen FRT-Stellen flankiert.

### Beispiel 16: Spezifische Rekombination in geeignete Genloci

### a. Zelllinie mit spezifischen Rekombinationssignalen in einem aktiven Genlocus

In die Zelllinie Jurkat wurde der linearisierte Vektor pBS MvHG418MdeltaPGK (Figur 13B) elektroporiert und wie in Beispiel 3b beschrieben G418-resistente Zellen selektioniert. Dabei wurde durch limitierte Verdünnung der Zellklon Jurkat G418 erhalten.

### b. Chimäre DNA

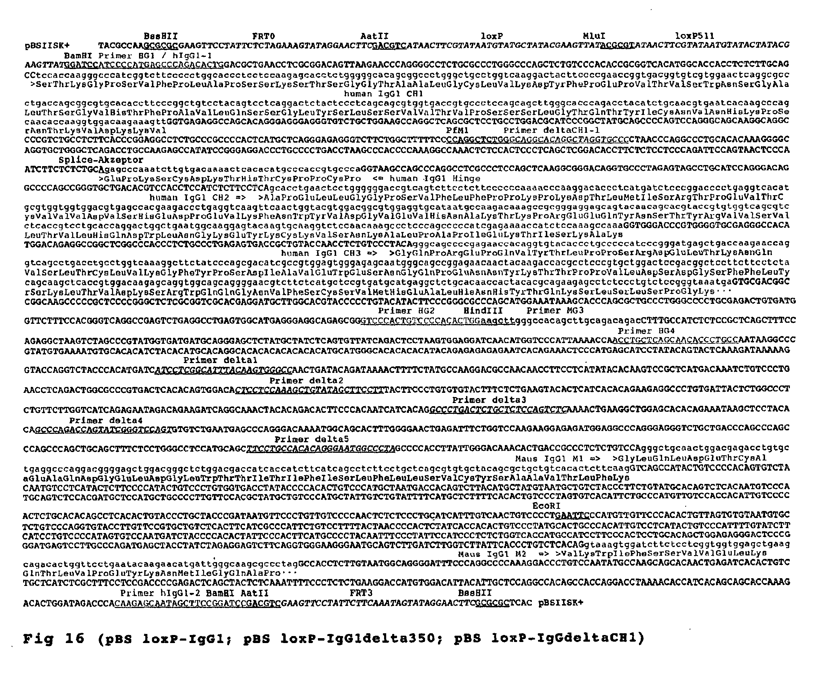

In die Zelllinie Jurkat G418 wurde der in Figur 16 beschriebene Vektor pBS loxP-IgG1 zusammen mit dem Flp-Expressionsvektor pOG44 elektroporiert, wie in Beispiel 1d beschrieben und anschliessend durch limitierte Verdünnung der eingesetzten ca. 10⁷ Zellen ca. 500 Klone getrennt propagiert. Von diesen 500 Klonen waren 4 Klone G418-sensitv. Die Sequenzierung genomischer DNA mit Hilfe FRTspezifischer PCR-Primer ergab die Zelllinie Jurkat loxP-IgG1.

### c. Spezifische Rekombination

In die Zellen der expandierten in Beispiel 18b beschriebenen Zelllinie Jurkat loxP-IgG1wurde anschliessend der Vektor pBS loxPvH (Figur 15B) zusammen mit dem Cre-Expressionsvektor pMC-Cre elektroporiert, die Zellen nach 3 Tagen mit FITC-markiertem Ziege-anti-Mensch-IgG1-Antikörper gefärbt und wie beschrieben mittels FACS-Sorter Zellen isoliert. Als Ergebnis wurden 425 aus ca. 10⁶ Zellen sortiert und davon 10 Klone getrennt propagiert, die im FACS eine deutliche grüne Fluoreszenz aufwiesen. Nach der Sequenzierung PCR-amplifizierter genomischer DNA war das Resultat dieser Vorgehensweise die Zelllinie Jurkat loxPvHEAIgG1. Diese Zelllinie codiert die vH-Domäne von HEA125 fusioniert an die konstanten humanen CH1, CH2 und CH3-Domänen. Ein Teil dieser schweren Antikörperkette wird auf der Oberfläche der Zelllinie Jurkat loxPvHEAIgG1-Zellen präsentiert.

Eine Variation dieser Vorgehensweise ist schematisch in Figur 6 dargestellt. Dabei wird in die vorselektionierte Zelllinie Jurkat G418 ein loxP-flankierte scFv-Antikörpergen, fusioniert an Hinge, CH2-, CH3-, M1- und M2-Domänen als Genkassette einrekombiniert. Dieses Rekombinationsereignis ist direkt auf Grund der Oberflächenexpression des einrekombinierten scFv-Antikörpers selektionierbar. Gleiches gilt für die ggfs. nachfolgende spezifische Rekombination einer Vielfalt unterschiedlicher scFv-Antikörper-Genkassetten.

Alternativ können als Ausgangsmaterial bereits vorhandene Zelllinien verwendet werden, die analog zu der in Beispiel 18a beschriebenen Vorgehensweise erhalten wurden. Beispiele sind die von Invitrogen vertriebenen Zelllinien Flp-In^{™}-293 (R750-07), Flp-In^{™}-CV-1 (R752-07) und Flp-In^{™}-CHO (R758-07).

In einer weiteren bevorzugten Versuchsdurchführung wird sowohl für die Integration des Resistenzgens in die Rekombinasekassette (siehe Beispiel 18a, G418-Selektion), wie auch für das ausschneiden des Resistenzgens durch Flp oder Cre (siehe Beispiel 18b) eine einfache Selektion der gewünschten Rekombinationsereignisse ermöglicht. Ein Beispiel dafür ist die Fusion des Neophosphoryltransferase II Gens an das Gen der Herpes Simplex Thymidinkinase. Zur Selektion von Zellen, die das integrierte chimäre Gen verloren haben wird dann Gancyclovir eingesetzt (Syntex #115561), das in Anwesenheit der Thymidinkinase (TK) in ein Zellgift umgewandelt wird (Masour et al., 1988, Nature 336, 348-352). Vorher können sehr einfach durch Selektion mit Gancyclovir Zelllinien hergestellt werden, deren endogene TK nicht mehr funktioniert.

### Beispiel 17: "Exon trap" und Oberflächen-Präsentation

### a. "Exon trap"-Zelllinie

In die Zellen der expandierten in Beispiel 18a beschriebenen Zelllinie Jurkat G418 wurde der Vektor pBS loxP-IgGdeltaCH1 (Figur 16) zusammen mit dem Flp-Expressionsvektor pOG44 elektroporiert, wie in Beispiel 1d beschrieben und anschliessend durch limitierte Verdünnung der eingesetzten ca. 10⁷ Zellen ca. 500 Klone getrennt propagiert. Von diesen 500 Klonen waren 5 Klone G418-sensitv. Die Sequenzierung genomischer DNA mit Hilfe FRTspezifischer PCR-Primer ergab die Zelllinie Jurkat loxP-IgG1deltaCH1. Diese Zelllinie codiert die konstanten Hinge, CH2, CH3, M1 und M2-Domänen eines IgG1-Antikörpers unter der Kontrolle eines endogenen Jurkat-Promotors. Im Bereich der variablen Domänen befindet sich eine loxP-Austauschkassette. Der Splice-Akzeptor am 5'-Ende des Hinge-Exons ist in Figur 16 zusammen mit dem offenen Leseraster dargestellt.

### b. Genomische DNA

Humane Lymphocyten wurden über einen Ficollgradienten nach Standardmethoden angereinigt und daraus die genomische DNA gewonnen (siehe z.B. Sambrook and Russell: Molecular Cloning, a laboratory manual, 3rd Edition, 2001, ISBN 0-87969-577-3). Diese DNA wurde mit AatII, NotI, MluI oder SalI geschnitten und jeweils in einem 0,8% TAE Agarosegel nach Grösse selektioniert (1-5 kb). Die Grössenselektionierte DNA wurde anschliessend in den in Figur 15D beschriebenen Vektor pBS loxPklon kloniert und anschliessend ein hochkomplexes Gemisch (>10⁶ verschiedene Transformanden) von Vektor-DNA isoliert (Qiagen Plasmid Purification Kit).

### c. Spezifische Rekombination

Das in Beispiel 19b beschriebene hochkomplexe Gemisch von Vektor-DNA wurde zusammen mit dem Cre-Expressionsvektor pMC-Cre in die Zellen des expandierten Klons Jurkat loxP-IgGldeltaCH1 elektroporiert, die Zellen nach 3 Tagen mit FITC-markiertem Ziege-anti-human-IgG-Antikörper gefärbt und wie beschrieben mittels FACS-Sorter Zellen isoliert. Als Ergebnis wurden 125 aus ca. 10⁸ Zellen sortiert und miteinander vereinigt, die im FACS eine deutliche grüne Fluoreszenz aufwiesen. Diese Exon-Trap-Bibliothek wurde in Zellkultur expandiert.

Das Resultat dieser Vorgehensweise ist eine Exon-Trap-Bibliothek, deren einzelne Mitglieder jeweils unterschiedliche humane Exon-codierte Domänen jeweils fusioniert an eine konstante CH2 und CH3-Domäne auf der Oberfläche der Zellen präsentieren.

### Beispiel 18: Suche nach Unterschieden in 2 komplexen Gemischen / Suche nach Tumor-assoziierten Antigenen

Es wurden ca. 10⁸ über einen Ficoll Gradienten angereinigte normale unmarkierte humane T-Lymphocyten mit ca. 10⁷ Zellen der in Beispiel 7 beschriebenen Antikörperbibliothek in 5ml DPBS-Puffer gemischt und 10 min auf Eis vorinkubiert.Die Zellen der in Beispiel 7 beschriebenen Antikörperbibliothek wurden dabei vorher wie beschrieben mit PE-markiertem Protein G angefärbt. Anschliessend wurden ca. 10⁶ Zellen der Lymphomzelllinie Jurkat mit FITC-markiertem anti-CD5-Antikörper angefärbt und dazugegeben. Die Jurkat Zellen wurden unmittelbar vorher zusätzlich mit 400 rad bestrahlt. Nach weiteren 20 min auf Eis wurden im FACS Sorter Doubletten sortiert, die eine grün-rote Doppelfluoreszenz aufwiesen. Im Ergebnis wurden 5 einzelne Zellen sortiert und expandiert. Der Zellkulturüberstand von einer dieser 5 Zelllinien ergab im FACS ein spezifisches Signal auf Jurkat Zellen verglichen mit der Färbung von aus dem Blut gewonnenen Lymphocyten. Der Nachweis der ins Medium sekretierten Antikörper erfolgte dabei durch FITC-markierten-Ziege-anti-Mensch-IgG-Antikörper (Dianova).

Die in den vorstehenden Beispielen beschriebenen Vorgehensweisen können vom Fachmann in vielfältiger Weise variiert bzw. miteinander kombiniert werden, z.B.:
■ Kann eine konstante Maus-Kappa-Domäne auch gegen eine konstante Mensch-Lambda-Domäne ausgetauscht werden (Beispiel 1e);
■ Ergibt jeweils einer aus insgesamt 9 verschiedenen Exon-Trap-Vektoren mit verschobenen Splice-Donor oder Splice-Akzeptor-Stellen ein Genprodukt im richtigen Leseraster, wobei ein Leader-Exon vorgegeben sein kann oder auch nicht (im letzteren Fall werden nur 3 verschiedene Exon-Trap-Vektoren benötigt; Beispiel 19);
■ Ist im Prinzip jeder aktive Genlocus geeignet, um ausgehend von einer Zelllinie durch spezifische Rekombination eine Vielfalt unterschiedlicher Zellen bzw. damit assoziierter Genprodukte zu erzeugen (Beispiele 3,4, 17, 18, 19);
■ Können auch gleiche spezifische Rekombinationssignale die variablen Sequenzen flankieren (Beispiele 3, 4, 12, 13, 14, 15, 16, 17, 18, 19);
■ Oder gleiche invertierte Rekombinationssignale die variablen Sequenzen flankieren;
■ Oder mehrere (viele) hintereinandergeschaltete variablen Sequenzen, die jeweils von Rekombinationssignalen flankiert sind einrekombiniert werden (Figur 6);
■ Kann an Stelle eines Rekombinasen-Expressionsvektors auch das Rekombinasenprotein selbst verwendet werden;
■ Kann dieses Rekombinasenprotein an eine Tet-Repressor-Domäne fusioniert werden, um dadurch die Rekombinationseffizienz von DAN-Sequenzen mit Tet-Operator-Stellen zu erhöhen;
■ Kann eine Zelllinie hergestellt werden, die die benötigten spezifischen Rekombinasen, insbesondere induzierbar produziert (Beispiele 3d, 4d), wobei z.B. die Cre-Aktivität mit dem Vektor pSVlacZT sehr einfach überprüft werden kann;
■ Kann die Vielfalt der Antikörpergene (Beispiele 5, 6, 7, 18) auch durch die chemische Synthese vieler unterschiedlicher CDRs innerhalb eines oder weniger vorgegebener Antikörpergen-Frameworks erfolgen;
■ Oder durch die Vervielfältigung von cDNA oder von scFv-Antikörperbibliotheken (Beispiel 18) durch PCR;
■ Kann die Vielfalt der einrekombinierten variablen Antikörpergene durch negative Selektion mit Gancyclovir vorselektioniert werden (Beispiel 18);
■ Können an Stelle einer G418-Resistenz oder eines myc-Epitops (Beispiele 3, 4, 18) auch andere selektionierbare Gensegmente eingebaut werden (z.B. EGFP);
■ Ist die Anwendung sowohl einer positiv (z.B. G418) wie negativ (z.B. Gancyclovir) selektionierbaren Rekombinationskassette möglich (Beispiel 18);
■ Können Bibliotheken (Beispiele 7, 16, 17, 18, 19) mit Arrays jeder Art kombiniert werden um ein massives paralleles Screening zu ermöglichen;
■ Können sehr einfach vielfältige Formen von bispezifischen, bifunktionellen oder allgemein veränderten Antikörpern hergestellt werden (Beispiele 12, 13, 14, 15, 18);
■ Kann die ins Kulturmedium sekretierte Spleissvariante eines Antikörpers (allgemein: eines entsprechenden Fusionsproteins; Beispiele 12, 13, 16, 18, 19) zur schnellen und einfachen Charakterisierung einer selektionierten Zelllinie eingesetzt werden(Beispiel 9), oder auch einer Sub-Bibliothek (Beispiel 8), oder auch als Charakterisierung einer erstellten Bibliothek dienen;
■ Kann die sekretierte Spleissvariante, insbesondere eines humanen Antikörpers u.U. direkt als pharmakologisch wirksame Substanz eingesetzt werden;
■ Können T-Zellbibliotheken (Beispiel 16, 17) mit ZellBibliotheken MHC-gebundener Antigene kombiniert werden, um T-Zell-spezifische Epitope zu suchen.

## Patentansprüche

1. Verfahren zur Humanisierung einer Hybridomzelle, **dadurch gekennzeichnet, dass**
(a)eine eine oder mehrere humane konstante IgG-, IgM-, IgA-, IgD- oder IgE-Domänen codierende DNA-Sequenz in die Hybridomzelllinie transfiziert wird;
(b) die DNA-Sequenz der humanen konstanten IgG-, IgM-, IgA-, IgD- oder IgE-Domänen von DNA-Sequenzen flankiert wird, die zu den chromosomalen Genbereichen homolog sind, die die konstanten Domänen des aktiven, die schwere Kette des Antikörpers codierenden Genlocus der genannten Hybridomzelle flankieren;
(c) eine oder wenige Zellen expandiert werden, die auf Grund einer homologen Rekombination eine IgG-, IgM-, IgA-, IgD- oder IgE-schwere Kette mit einem humanisierten konstanten Anteil exprimieren;
(d) eine humane konstante Kappa- oder Lambda-Domäne codierende DNA-Sequenz in die Hybridomzelllinie transfiziert wird, wobei diese DNA-Sequenz von DNA-Sequenzen flankiert wird, die zu den chromosomalen Genbereichen homolog sind, die die konstante Kappa- oder Lambda-Domäne des aktiven Kappa- oder Lambda-Genlocus der Hybridomzelle flankieren;
(e) anschliessend eine oder wenige Zellen expandiert werden, die auf Grund einer homologen Rekombination eine Kappa- oder Lambda-Kette mit einem humanisierten konstanten Anteil exprimieren,
wobei die exprimierten Antikörper auf Grund der kovalenten Kopplung der membranständigen Spleissvariante der schweren Antikörperkette an die Oberfläche der sie jeweils exprimierenden Zellen gebunden sind, was den Nachweis und die Selektion derjenigen Zellen erlaubt, die humanisierte konstante Antikörperdomänen präsentieren.

2. Verfahren nach Anspruch 1, wobei zunächst die konstanten Domänen der leichten Antikörperkette humanisiert werden und danach die konstanten Domänen der schweren Antikörperkette.

3. Verfahren nach Anspruch 1, wobei nur die konstanten Domänen der schweren Antikörperkette oder nur die konstanten Domänen der leichten Antikörperkette humanisiert werden.

4. Verfahren nach einem der Ansprüche 1 bis 3, wobei das Intron in Richtung 5'-Ende vor dem M1-Exon des aktiven IgG-, IgM-, IgA-, IgD- oder IgE-Gens um mehr als 50 Basenpaare verkürzt ist.

5. Verfahren nach einem der Ansprüche 1 bis 4, wobei an die humanisierten konstanten Domänen zusätzliche Proteincodierende DNA-Sequenzen fusioniert sind.

6. Verfahren nach Anspruch 5, wobei die zusätzlichen Proteincodierenden DNA-Sequenzen eine Linkersequenz und einen Einzelkettenantikörper codieren, der C-terminal an die konstante Domäne der leichten Antikörperkette fusioniert ist.

7. Verfahren nach einem der Ansprüche 1 bis 6, wobei die homologen Bereiche der transfizierten DNA-Sequenzen zu den jeweiligen Antikörper-Genloci der Hybridom-Zelle sich über mindestens 400 Basenpaare erstrecken.

8. Verfahren nach einem der Asnprüche 1 bis 7, wobei bei den homologen Rekombinationen keine Resistenzmarker in die Zellen eingeführt werden, die zur Selektion homologer Rekombinationsereignisse eingesetzt werden.

## Claims

1. A method of humanizing a hybridoma cell, **characterized by**
(a) transfecting a DNA sequence coding for one or more human constant IgG, IgM, IgA, IgD or IgE domains into the hybridoma cell line;
(b) flanking the DNA sequence of the human constant IgG, IgM, IgA, IgD or IgE domains by DNA sequences homologous to the chromosomal gene regions flanking the constant domains of the active hybridoma cell gene locus coding for the heavy chain of the antibody;
(c) expanding one or few cells which on account of a homologous recombination express an IgG, IgM, IgA, IgD or IgE heavy chain with a humanized constant portion;
(d) transfecting a DNA sequence coding or a human constant kappa or lambda domain into the hybridoma cell line, this DNA sequence being flanked by DNA sequences homologous to the chromosomal gene regions which flank the constant kappa or lambda domain of the active kappa or lambda gene locus of the hybridoma cell;
(e) subsequently expanding one or few cells which on account of a homologous recombination express a kappa or lambda chain with a humanized constant portion,
wherein on account of the covalent linkage of the membrane-bound splicing variant of the heavy antibody chain the expressed antibodies are bound to the surface of the particular cells expressing them so as to detect and select the cells presenting humanized constant antibody domains.

2. The method according to claim 1, wherein the constant domains of the light antibody chain are initially humanized and thereafter the constant domains of the heavy antibody chain.

3. The method according to claim 1, wherein only the constant domains of the heavy antibody chain or only the constant domains of the light antibody chain are humanized.

4. The method according to any of claims 1 to 3, wherein the intron is shortened by more than 50 base pairs in the 5' end direction upstream of the M1 exon of the active IgG, IgM, IgA, IgD or IgE gene.

5. The method according to any of claims 1 to 4, wherein additional protein-encoding DNA sequences are fused to the humanized constant domains.

6. The method according to claim 5, wherein the additional protein-encoding DNA sequences code for a linker sequence and a single-chain antibody, which is fused C-terminally to the constant domain of the light antibody chain.

7. The method according to any o claims 1 to 6, wherein the homologous regions of the transfected DNA sequences to the particular antibody gene loci of the hybridoma cell extend over at least 400 base pairs.

8. The method according to any of claims 1 to 7, wherein in the homologous recombinations no resistance markers are introduced into the cells which are used for the selection of homologous recombination events.

## Revendications

1. Procédé pour humaniser une cellule hybridome, **caractérisé par le fait que**
(a) une séquence d'ADN codant un ou plusieurs domaines IgG, IgM, IgA, IgD ou IgE constants humains est transfixée dans la lignée de la cellule hybridome;
(b) la séquence d'ADN des domaines IgG, IgM, IgA, IgD ou IgE constants humains est flanquée de séquences d'ADN qui sont homologues aux zones de gène chromosomal qui flanquent les domaines constants du locus actif, codant la chaîne lourde de l'anticorps, de ladite cellule hybridome;
(c) il est expansé une ou quelques cellules qui, par suite d'une recombinaison homologue, expriment une chaîne lourde d'IgG, d'IgM, d'IgA, d'IgD ou d'IgE avec une part constante humanisée;
(d) une séquence d'ADN codant des domaines Kappa ou Lambda constants humains est transfixée dans la lignée de la cellule hybridome, cette séquence d'ADN étant flanquée de séquences d'ADN qui sont homologues aux zones de gène chromosomal qui flanquent les domaines Kappa ou Lambda constants du locus de gène Kappa ou Lambda actif de la cellule hybridome;
(e) ensuite, il est expansé une ou quelques cellules qui, par suite d'une recombinaison homologue, expriment une chaîne Kappa ou Lambda avec une part constante humanisée,
les anticorps exprimés étant, du fait du couplage covalent de la variante d'épissage liée à la membrane de la chaîne lourde d'anticorps, liés, chacun, à la surface des cellules qui les expriment respectivement, ce qui permet la preuve et la sélection des cellules présentant des domaines d'anticorps constants humanisés.

2. Procédé selon la revendication 1, dans lequel il est tout d'abord humanisé les domaines constants de la chaîne légère d'anticorps et ensuite les domaines constants de la chaîne lourde d'anticorps.

3. Procédé selon la revendication 1, dans lequel seuls les domaines constants de la chaîne lourde d'anticorps ou seuls les domaines constants de la chaîne légère d'anticorps sont humanisés.

4. Procédé selon l'une des revendications 1 à 3, dans lequel l'intron est, en direction de l'extrémité 5' avant l'exon M1 du gène IgG, IgM, IgA, IgD ou IgE actif, raccourci de plus de 50 paires de bases.

5. Procédé selon l'une des revendications 1 à 4, dans lequel aux domaines constants humanisés sont fusionnées des séquences d'ADN codant des protéines additionnelles.

6. Procédé selon la revendication 5, dans lequel les séquences d'ADN codant des protéines additionnelles codent une séquence de liaison et un anticorps à chaîne unique, la terminaison C est fusionnée aux domaines constants de la chaîne légère d'anticorps.

7. Procédé selon l'une des revendications 1 à 6, dans lequel les zones homologues des séquences d'ADN transfixées aux locus d'anticorps respectifs de la cellule hybridome s'étendent sur au moins 400 paires de bases.

8. Procédé selon l'une des revendications 1 à 7, dans lequel, dans les recombinaisons homologues, il n'est pas introduit dans les cellules de marqueurs de résistance qui sont utilisés pour la sélection d'événement de recombinaison homologue.
